# EUROPEAN PATENT APPLICATION

(11) **EP 4 534 683 A1**
(43) Date of publication of application: **09.04.2025**
(21) Application number: 23815286.2
(22) Date of filing: 01.06.2023
(51) Int. Cl.: C12N 15/90, C12N 9/22, C12N 15/113

(54) **NOVEL CRISPR GENE EDITING SYSTEM**

(30) Priority: 01.06.2022 CN 202210620277
(71) Applicant: INSTITUTE OF GENETICS AND DEVELOPMENTAL BIOLOGY, CHINESE ACADEMY OF SCIENCES, Chaoyang District Beijing 100101 (CN); Beijing Qi Biodesign Biotechnology Company Limited, Beijing 102206 (CN)
(72) Inventor: GAO, Caixia, Beijing 100101 (CN); JIN, Shuai, Beijing 100101 (CN); ZHU, Zixu, Beijing 102206 (CN); LI, Yunjia, Beijing 100101 (CN); ZHAO, Kevin T·, Beijing 102206 (CN); LIU, Guanwen, Beijing 102206 (CN)
(74) Representative: Michalski Hüttermann & Partner Patentanwälte mbB
(86) International application number: PCT/CN2023/097783
(87) International publication number: WO 2023/232109

(57) **Abstract**

The present invention belongs to the field of genetic engineering. Specifically, the present invention relates to a novel CRISPR gene editing system and uses thereof. More specifically, the present invention provides a TraC (intermediates between Transposon and CRISPR-Cas12) effector protein or functional variant thereof, gene editing systems based thereon and uses thereof.

## Description

### Technical field

The present invention belongs to the field of genetic engineering. Specifically, the present invention relates to a novel CRISPR gene editing system and uses thereof. More specifically, the present invention provides a TraC (intermediates between Transposon and CRISPR-Cas12) effector protein or functional variant thereof, gene editing systems based thereon and uses thereof.

### Background

Type V effector proteins are Cas12 proteins with multiple functional domains, and their hallmark feature is that they contain a RuvC-like domain, which is generally responsible for the cleavage of target DNA. There are many subtypes of Type V proteins. The subtypes that have been discovered and classified include Cas12a-k, a total of 11 subtypes, of which Cas12a and Cas12b have been developed into efficient eukaryotic gene editing systems. Cas12a, also known as Cpf1 protein, contains a RuvC-like domain similar to Cas9 protein or TnpB protein, but compared to Cas9, Cas12a family proteins lack the HNH domain and use only the RuvC domain to cleave both strands of DNA. Cas12b was previously called C2c1 (Class 2 Candidate 1) when it was discovered. Its C-terminal sequence is very similar to TnpB protein of IS605 family, but it does not have significant sequence similarity with other Class II family proteins. Its Cas gene includes Cas1/Cas4 fusion gene, Cas2, and Cas12b gene. The maturation of its crRNA also requires the participation of trRNA. Cas12c was originally called C2c3 (Class 2 Candidate 3), and its Cas gene only includes Cas1 and Cas12c genes, and the Cas12c gene only has limited similarity with the TnpB homologous sequence of Cpf1.

Due to the improvement of bioinformatics analysis methods, the number of Type V subtypes has exploded in recent years, including Cas12a, Cas12b and Cas12c proteins, a total of 10 Type V subtypes have been found. The nucleic acid interference activity of these subtypes has also been gradually demonstrated experimentally. For example, scientists from Arbor Biotech have demonstrated the DNA double-strand cleavage activity of the effector proteins Cas12c, Cas12g, Cas12h and Cas12i from Type V-C, Type V-G, Type V-H, and Type V-I through in vitro experiments. In addition, the effector proteins of Type V-D and Type V-E subtypes are CasX and CasY, respectively, also known as Cas12d and Cas12e, which were originally found in the metagenomes of "unculturable" microorganisms, and were also shown to have genome editing activity in Escherichia coli and human cell lines in 2019. The effector protein of Type V-F subtype, which was previously considered to be one of the subtypes of Type V-U family, is Cas14 (also known as Cas12f), which can cut single-stranded DNA and RNA, and is only one-third the size of Cas9 protein. The Cas14 protein was first developed as a nucleic acid detection tool DETECTOR, and it has recently been shown to have DNA double-strand cleavage activity in prokaryotic and eukaryotic organisms. The Casϕ protein (also known as Cas12j) recently discovered in macrophages has also been proved to have the ability to cut DNA double strands in prokaryotes, animal cells and plant cells. In Type V-K type, its effector protein Cas12k is "hijacked" by the transposon Tn7, which can generate an R-loop at the target site, and utilize the targeting ability of crRNA to achieve site-directed transposition of the transposon. This hijacked protein provides a new strategy for site-directed DNA insertion.

Identifying novel CRISPR effector proteins that can achieve gene editing can enrich genome editing tools and is of great significance to the field of biomedicine.

### Brief description of the invention

The present invention provides at least the following embodiments:
Embodiment 1. An engineered clustered regularly interspaced short palindromic repeat (CRISPR) system comprising:
   a) a TraC (intermediates between **Tra**nsposon and CRISPR-Cas12) effector protein or one or more nucleotide sequences encoding such effector protein; and
   b) one or more guide RNAs, or a nucleotide sequence encoding the one or more guide RNAs,

   wherein the guide RNA is selected from i) a guide RNA derived from a transposon right-element (reRNA) and/or ii) a guide RNA comprising tracrRNA and/or crRNA, such as a single guide RNA (sgRNA) comprising tracrRNA and crRNA;
   the TraC effector protein can form a CRISPR complex with the guide RNA;
   the TraC effector protein can target and bind to a target DNA sequence under the guidance of the guide RNA derived from transposon right element (reRNA), and can also target and bind to a target DNA sequence under the guidance of the guide RNA comprising tracrRNA and/or crRNA.
Embodiment 2. The engineered CRISPR system of Embodiment 1, wherein the tracrRNA contains a non-targeting strand binding sequence (NTB) which is complementary to the non-targeting strand (NTS).
Embodiment 3. An engineered regularly interspaced short palindromic repeat sequence CRISPR vector system comprising one or more constructs, comprising:
   a) a first regulatory element operably linked to a nucleotide sequence encoding a TraC (intermediates between Transposon and CRISPR-Cas12) effector protein; and
   b) a second regulatory element operably linked to one or more nucleotide sequences encoding one or more guide RNAs selected from i) a guide RNA derived from a transposon right-element (reRNA) and/or ii) a guide RNA comprising tracrRNA and/or crRNA, such as a single guide RNA (sgRNA) comprising tracrRNA and crRNA;

   the TraC effector protein can form a CRISPR complex with the guide RNA;
   the TraC effector protein can target and bind to a target DNA sequence under the guidance of the guide RNA derived from a transposon right element, and can also target and bind to a target DNA sequence under the guidance of the guide RNA comprising tracrRNA and/or crRNA.
Embodiment 4. The engineered CRISPR vector system according to Embodiment 3, wherein the tracrRNA contains a non-targeting strand binding sequence (NTB) which is complementary to the non-targeting strand (NTS).
Embodiment 5. The system according to Embodiment 2 or 4, wherein the guide RNA is a guide RNA comprising tracrRNA and crRNA, wherein the tracrRNA contains a non-targeting strand binding sequence (NTB) complementary to the non-targeting strand (NTS), wherein the guide RNA hybridizes to the targeting strand (TS) of the target DNA sequence via the crRNA, and hybridizes to the non-targeting strand (NTS) via the NTB.
Embodiment 6. The system of Embodiment 4, wherein when transcribed, the one or more guide RNAs hybridize to the target DNA, and the guide RNA forms a complex with the TraC effector protein that causes the cleavage at distal end of the target DNA sequence.
Embodiment 7. The system according to any one of Embodiments 1-6, wherein the target DNA sequence is within a cell, preferably within a eukaryotic cell.
Embodiment 8. The system of any one of Embodiments 1-7, wherein the effector protein comprises one or more nuclear localization sequences (NLS), cytoplasmic localization sequences, chloroplast localization sequences or mitochondrial localization sequences.
Embodiment 9. The system according to any one of Embodiments 1-8, wherein the nucleic acid sequence encoding the effector protein is codon-optimized for expression in eukaryotic cells.
Embodiment 10. The system according to any one of Embodiments 1-9, wherein components a) and b) or their nucleotide sequences are constructed on the same or different vectors.
Embodiment 11. A method for modifying a target DNA sequence, the method comprising delivering the system according to any one of Embodiments 1-10 to the target DNA sequence or into a cell containing the target DNA sequence.
Embodiment 12. A method of modifying a target DNA sequence, the method comprising delivering a composition of a TraC effector protein and one or more nucleic acid components to the target DNA sequence, wherein the effector protein can target and bind to the target DNA sequence under the guidance of the guide RNA derived from a transposon right element, and can also target and bind to the target DNA sequence under the guidance of the guide RNA comprising tracrRNA and/or crRNA; the effector protein and the one or more nucleic acid components form a CRISPR complex, and upon targeted binding of said complex to the target DNA sequence that is 3' of a prospacer adjacent motif (PAM), the effector protein induces modification of the target DNA sequence.
Embodiment 13. The method of Embodiment 12, wherein the target gene is within a cell, preferably within a eukaryotic cell.
Embodiment 14. The method of Embodiment 13, wherein the cell is an animal cell or a human cell.
Embodiment 15. The method of Embodiment 13, wherein the cell is a plant cell.
Embodiment 16. The method of Embodiment 12, wherein the effector protein comprises one or more nuclear localization sequences (NLS), cytoplasmic localization sequences, chloroplast localization sequences or mitochondrial localization sequences.
Embodiment 17. The method of Embodiment 12, wherein the effector protein and nucleic acid component, or a construct expressing the effector protein and nucleic acid component, are contained in a delivery system.
Embodiment 18. The method of Embodiment 17, wherein the delivery system comprises virus, virus-like particle, virosome, liposome, vesicle, exosome, liposomal nanoparticle (LNP), N-acetylgalactosamine ( GalNAc) or engineered bacteria.
Embodiment 19. A TraC (intermediates between Transposon and CRISPR-Cas12) effector protein or functional variant thereof for genome editing in an organism or an organism cell, wherein the TraC effector protein or a functional variant thereof can form a CRISPR complex with a guide RNA;
   the guide RNA is selected from i) a guide RNA derived from a transposon right element (reRNA) and/or ii) a guide RNA comprising tracrRNA and/or crRNA, such as a single guide RNA (sgRNA) comprising tracrRNA and crRNA;
   the TraC effector protein or functional variant thereof can target and bind to the target DNA sequence under the guidance of the guide RNA derived from a transposon right element, and can also target and bind to the target DNA sequence under the guidance of the guide RNA comprising tracrRNA and/or crRNA.
Embodiment 20. A TraC (intermediates between Transposon and CRISPR-Cas12) effector protein or functional variant thereof for genome editing in an organism or a cell of an organism, the TraC effector protein or functional variant thereof
   (i) comprises at least one, at least two or all three amino acid sequence motifs selected from the group consisting of "TSxxCxxCx", "GIDRG" and "CxxCGxxxxADxxAA", wherein x represents any amino acid, such as any naturally encoded amino acid;
   (ii) comprises an amino acid sequence that are at least 30%, at least 35%, at least 40%, at
   least 45%, at least 50%, at least 55%, at least 60%, at least 65%, at least 70%, at least 75%, at least 80%, at least 85%, at least 90%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, at least 99.9%, even 100% identical to any one of SEQ ID NOs: 1-37, or comprises an amino acid sequence having one or more, for example, 1, 2, 3, 4, 5, 6, 7, 8, 9 or 10 amino acid substitutions, deletions or additions relative to any one of SEQ ID NOs: 1-37.
Embodiment 21. The TraC effector protein or functional variant thereof according to Embodiment 20, wherein said effector protein functional variant is derived from SEQ ID NO: 25, and comprises one or more amino acid substitutions selected from K78R, D86R, S137R, V145R, I147R, P148R, D150R, V228R, V254R, A510R, A278R, K315R, S334R, L343R, A369R, H392R, L394R, S408R, N456R, V500R, A510R, T573R, relative to the sequence of SEQ ID NO: 25.
Embodiment 22. The TraC effector protein or functional variant thereof according to Embodiment 20 or 21, wherein the effector protein functional variant is derived from SEQ ID NO: 25, and comprises any set of mutations shown in Table 3 or 4 relative to the sequence of SEQ ID NO: 25.
Embodiment 23. The TraC effector protein or functional variant thereof according to Embodiment 20, wherein the effector protein functional variant comprises an amino acid sequence selected from SEQ ID NOs: 80-87.
Embodiment 24. The TraC effector protein or functional variant thereof according to any one of Embodiments 20-23, which has at least guide RNA-mediated sequence-specific targeting ability.
Embodiment 25. The TraC effector protein or functional variant thereof according to any one of Embodiments 20-23, which has guide RNA-mediated sequence-specific targeting ability, and double-stranded nucleic acid cleavage activity.
Embodiment 26. The TraC effector protein or functional variant thereof according to any one of Embodiments 20-23, which has guide RNA-mediated sequence-specific targeting capability, and nickase activity.
Embodiment 27. The TraC effector protein or functional variant thereof according to any one of Embodiments 20-23, which has guide RNA-mediated sequence-specific targeting ability but does not have double-stranded nucleic acid cleavage activity and/or nickase activity.
Embodiment 28. The TraC effector protein or functional variant thereof according to any one of Embodiments 24-24, wherein the guide RNA is selected from i) a guide RNA (reRNA) derived from a transposon right element and/or ii) a guide RNA comprising tracrRNA and/or crRNA, such as a single guide RNA (sgRNA) comprising tracrRNA and crRNA.
Embodiment 29. The TraC effector protein or functional variant thereof according to Embodiment 28, the TraC effector protein or functional variant thereof can target and bind to the target DNA sequence under the guidance of the guide RNA derived from a transposon right element, and can also target and bind to the target DNA sequence under the guidance of the guide RNA comprising tracrRNA and/or crRNA.
Embodiment 30. The TraC effector protein or functional variant thereof according to Embodiment 28, wherein the guide RNA is reRNA derived from the TnpB system, for example, the reRNA comprises a scaffold sequence shown in SEQ ID NO:77 or 78.
Embodiment 31. The TraC effector protein or functional variant thereof according to Embodiment 28, wherein the guide RNA is a single guide RNA (sgRNA) of tracrRNA and crRNA, for example, the sgRNA comprises a scaffold sequence shown in SEQ ID NO:75 or 76.
Embodiment 32. The TraC effector protein or functional variant thereof according to any one of Embodiments 19-31, further comprising at least one nuclear localization sequence (NLS), cytoplasmic localization sequence, chloroplast localization sequence or mitochondrial localization sequence.
Embodiment 33. A fusion protein comprising the TraC effector protein or functional variant thereof according to any one of Embodiments 19-32, and at least one other functional protein.
Embodiment 34. The fusion protein of Embodiment 33, wherein the other functional protein is a deaminase.
Embodiment 35. The fusion protein of Embodiment 34, wherein said deaminase is a cytosine deaminase, for example, said cytosine deaminase is selected from the group consisting of APOBEC1 deaminase, activation-induced cytidine deaminase (AID), APOBEC3G, CDA1, human APOBEC3A deaminase, double-stranded DNA deaminase (Ddd), single-stranded DNA deaminase (Sdd), or functional variants thereof.
Embodiment 36. The fusion protein of Embodiment 35, further comprising a uracil DNA glycosylase inhibitor (UGI).
Embodiment 37. The fusion protein of Embodiment 34, wherein the deaminase is an adenine deaminase, e.g., a DNA-dependent adenine deaminase derived from Escherichia coli tRNA adenine deaminase TadA (ecTadA).
Embodiment 38. The fusion protein of any one of Embodiments 34-37, wherein the fusion protein comprises a cytosine deaminase and an adenine deaminase.
Embodiment 39. The fusion protein of Embodiment 33, wherein said other functional protein is selected from transcription activator protein, transcription repressor protein, DNA methylase, DNA demethylase, reverse transcriptase.
Embodiment 40. The fusion protein according to any one of Embodiments 33-39, wherein different parts of the fusion protein can be independently linked through linkers or linked directly.
Embodiment 41. The fusion protein of any one of Embodiments 33-40, further comprising at least one nuclear localization sequence (NLS), cytoplasmic localization sequence, chloroplast localization sequence or mitochondrial localization sequence.
Embodiment 42.Use of the TraC effector protein or functional variant thereof according to any one of Embodiments 19-32 or the fusion protein according to any one of Embodiments 33-41 for genome editing in cells, preferably eukaryotic cells, more preferably plant cells.
Embodiment 43. The use according to Embodiment 42, wherein the genome editing includes base editing (Base Editor), prime editing (Prime Editor), or PrimeRoot editing (PrimRoot Editor).
Embodiment 44. A genome editing system for performing site-directed modification of a target nucleic acid sequence in a cell genome, comprising:
   the TraC effector protein or functional variant thereof of any one of Embodiments 19-32 or the fusion protein of any one of Embodiments 33-41; and/or
   an expression construct comprising the nucleotide sequence encoding the TraC effector protein or functional variant thereof according to any one of Embodiments 19-32 or the fusion protein according to any one of Embodiments 33-41.
Embodiment 45. The genome editing system of Embodiment 44, further comprising at least one guide RNA (gRNA) and/or an expression construct comprising a nucleotide sequence encoding the at least one guide RNA.
Embodiment 46. The genome editing system of Embodiment 45, said genome editing system comprises any one selected from the group consisting of:
   i) the TraC effector protein or functional variant thereof of any one of Embodiments 19-32 or the fusion protein of any one of Embodiments 33-41, and the at least one guide RNA, optionally, the CRISPR nuclease or functional variant thereof or the fusion protein forms a complex with the at least one guide RNA;
   ii) an expression construct comprising a nucleotide sequence encoding the TraC effector protein or functional variant thereof of any one of Embodiments 19-32 or a fusion protein of any one of Embodiments 33-41, and the at least one guide RNA;
   iii) the TraC effector protein or functional variant thereof of any one of Embodiments 19-32 or the fusion protein of any one of Embodiments 33-41, and an expression construct comprising a nucleotide sequence encoding the at least one guide RNA;
   iv) an expression construct comprising a nucleotide sequence encoding the TraC effector protein or functional variant thereof of any one of Embodiments 19-32 or a fusion protein of any one of Embodiments 33-41, and an expression construct comprising a nucleotide sequence encoding the at least one guide RNA;
   v) an expression construct comprising a nucleotide sequence encoding the TraC effector protein or functional variant thereof of any one of Embodiments 19-32 or or the fusion protein of any one of Embodiments 33-41, and a nucleotide sequence encoding the at least one guide RNA.
Embodiment 47. The genome editing system of any one of Embodiments 45-46, wherein the guide RNA is selected from i) a guide RNA derived from a transposon right element (reRNA) and/or ii) a guide RNA comprising tracrRNA and/or crRNA, such as a single-guide RNA (sgRNA) comprising tracrRNA and crRNA.
Embodiment 48. The genome editing system of Embodiment 47, wherein the guide RNA is reRNA derived from the TnpB system, for example, the reRNA comprises a scaffold sequence shown in SEQ ID NO:77 or 78.
Embodiment 49. The genome editing system according to any one of Embodiments 45-46, wherein the guide RNA is a single guide RNA (sgRNA) comprising tracrRNA and crRNA, for example, the sgRNA comprises a scaffold sequence shown in SEQ ID NO:75 or 76.
Embodiment 50. The genome editing system of Embodiment 47 or 49, wherein the guide RNA comprises tracrRNA and crRNA, such as a single guide RNA (sgRNA) comprising tracrRNA and crRNA, wherein the crRNA comprises a sequence identical to the target sequence immediately adjacent to the PAM, and the tracrRNA comprises a sequence (non-targeting strand binding sequence, NTB) complementary to a sequence distal to the PAM in the target sequence direction.
Embodiment 51. The genome editing system according to any one of Embodiments 44-50, wherein said genome editing system further comprises a donor nucleic acid molecule, said donor nucleic acid molecule comprising a nucleotide sequence to be site-directed inserted into the genome, for example, the nucleotide sequence to be inserted into the genome comprises on its both sides sequences homologous to the sequences flacking the target sequence in the genome.
Embodiment 52. The genome editing system according to any one of Embodiments 44-51, wherein the nucleotide sequence encoding the TraC effector protein or functional variant thereof or the fusion protein and/or the nucleotide sequence encoding the at least one guide RNA is operably linked to an expression control element such as a promoter.
Embodiment 53. The genome editing system of any one of Embodiments 44-52, wherein the components of the genome editing system are contained in a delivery system selected from the group consisting of virus, virus-like particle, virosome, liposome , vesicle, exosome, liposomal nanoparticle (LNP), N-acetylgalactosamine (GalNAc) or engineered bacteria.
Embodiment 54. A method of producing a genetically modified cell comprising introducing the genome editing system of any one of Embodiments 44-53 into said cell.
Embodiment 55. The method of Embodiment 54, wherein said cell is from prokaryotes or eukaryotes, preferably from mammals such as humans, mice, rats, monkeys, dogs, pigs, sheep, cattle, cats; poultry such as chickens, ducks , goose; plants, including monocots and dicots, such as rice, maize, wheat, sorghum, barley, soybean, peanut, Arabidopsis.

The advantages of the present invention mainly lie in:
(1) The inventors obtained new CRISPR effector proteins and genome editing systems thereof, which enriches the options and application scenarios of genome editing tools;
(2) The TraC sub-clade CRISPR effector proteins obtained in the present invention has a dual guidance mechanism, and has the targeted cleavage pathway of the TnpB system and the CRISPR system at the same time, that is, the TraC effector proteins can target and bind to the target DNA under the guidance of reRNA, and can also target and bind to target DNA under the guidance of sgRNA, which can help to achieve multiple genome editing with the same gene editing tool;
(3) The TraC effector proteins obtained in the present invention are the smallest monomers compared to the currently known monomeric Cas12 proteins, which is suitable for delivery and editing in vivo.
(4) The TraC effector proteins obtained in the present invention interact with the non-targeted strand of the target dsDNA to form bubbles structure under the guidance of the sgRNA containing the non-targeted strand complementary sequence (NTB) complementary to the non-targeted strand (NTS), which facilitates the opening and editing of DNA distal to the PAM.

### Brief Description of Drawings

Figure 1. Shows three structural motifs conserved in 86 Cas12 proteins.
Figure 2. Shows the prokaryotic expression system of TraC proteins.
Figure 3. A flowchart showing the use of a fluorescent reporter system to screen a CRISPR system capable of binding DNA double strands.
Figure 4. Screening results of dLbCas12a protein using fluorescent reporter system.
Figure 5. Flowchart for screening CRISPR systems capable of DNA double-strand cutting.
Figure 6. Test results of DNA double-strand cutting ability. A: Test results of TraC-875, TraC-365, TraC-655, TraC-445; B: Test results of TraC-297, TraC-459, TraC-466, TraC-949. LbCpf1 served as a positive control.
Figure 7. Flowchart for testing the DNA double-strand cutting ability of the novel CRISPR system using the plasmid interference system.
Figure 8. The test results of TraC-459, TraC-875 and TraC-297 proteins using the plasmid interference system.
Figure 9. A: appendix RNA secondary structure prediction and structural folding model analysis of V-type CRISPR and TnpB systems; B: Model of co-evolution of effectors and appendix RNAs.
Figure 10. Shows optimization of TraC protein sgRNA. A: sgRNA predicted for TraC-459 protein; B: Effect of tracrRNA:crRNA complementary region truncated length, tracrRNA 5' region truncated length, spacer length on TraC-459 protein editing efficiency.
Figure 11. Shows that the optimized sgRNA-opt can significantly improve the editing efficiency of TraC-459.
Figure 12 shows the use of plasmid interference experiments to analyze the dsDNA cutting ability of TraC-459 on E. coli E. coli under different guide RNAs.
Figure 13 shows the prediction results of the three-dimensional structure of TraC-459 protein.
Figure 14. Shows TraC-459 variant screening.
Figure 15. Prediction of the secondary structure of the TraC effector sgRNA complex reveals a bubble-like region at the end of the tracrRNA.
Figure 16. TraC effector targets DNA guided by a bubble-like region of the reprogrammed sgRNA.
Figure 17. Reprogrammed sgRNA can improve editing efficiency.
Figure 18. Shows that TraC protein is affected by temperature in plant cells. The editing efficiency of TraC-5M-7 at 32°C was 1-29 times higher than that at 25°C.

### Detailed description of the invention

### 1. Definition

In the present invention, unless indicated otherwise, the scientific and technological terminologies used herein refer to meanings commonly understood by a person skilled in the art. Also, the terminologies and experimental procedures used herein relating to protein and nucleotide chemistry, molecular biology, cell and tissue cultivation, microbiology, immunology, all belong to terminologies and conventional methods generally used in the art. For example, the standard DNA recombination and molecular cloning technology used herein are well known to a person skilled in the art, and are described in details in the following references: Sambrook, J., Fritsch, E.F. and Maniatis, T., Molecular Cloning: A Laboratory Manual; Cold Spring Harbor Laboratory Press: Cold Spring Harbor, 1989. In the meantime, in order to better understand the present invention, definitions and explanations for the relevant terminologies are provided below.

As used herein, the term "and/or" encompasses all combinations of items connected by the term, and each combination should be regarded as individually listed herein. For example, "A and/or B" covers "A", "A and B", and "B". For example, "A, B, and/or C" covers "A", "B", "C", "A and B", "A and C", "B and C", and "A and B and C".

When the term "comprise" is used herein to describe the sequence of a protein or nucleic acid, the protein or nucleic acid may consist of the sequence, or may have additional amino acids or nucleotide at one or both ends of the protein or nucleic acid, but still have the activity described in this invention. In addition, those skilled in the art know that the methionine encoded by the start codon at the N-terminus of the polypeptide will be retained under certain practical conditions (for example, when expressed in a specific expression system), but does not substantially affect the function of the polypeptide. Therefore, when describing the amino acid sequence of specific polypeptide in the specification and claims of the present application, although it may not include the methionine encoded by the start codon at the N-terminus, the sequence containing the methionine is also encompassed, correspondingly, its coding nucleotide sequence may also contain a start codon; vice versa.

"Genome" as used herein encompasses not only chromosomal DNA present in the nucleus, but also organelle DNA present in the subcellular components (e.g., mitochondria, plastids) of the cell.

As used herein, an "organism" includes any organism suitable for genome editing, preferably, an eukaryote. An example of organism includes but is not limited to, a mammal such as human, mouse, rat, monkey, dog, pig, sheep, cattle, cat; poultry such as chicken , duck, goose; a plant, including a monocotyledonous plant and a dicotyledonous plant such as rice, corn, wheat, sorghum, barley, soybean, peanut, Arabidopsis and the like.

A "genetically modified organism" or a "genetically modified cell" means an organism or a cell which comprises an exogenous polynucleotide or comprises a modified gene or expression regulatory sequence within its genome. For example, the exogenous polynucleotide can be stably integrated into the genome of the organism or cell and inherited in successive generations. The exogenous polynucleotide may be integrated into the genome alone or as a part of a recombinant DNA construct. The modified gene or expression regulatory sequence is a gene or expression regulatory sequence comprising one or more nucleotide substitutions, deletions and additions in the genome of the organism or cell.

The term "exogenous" with respect to sequence means a sequence that originates from a foreign species, or, if from the same species, is substantially modified from its native form in composition and/or genomic locus by deliberate human intervention.

"Polynucleotide", "nucleic acid sequence", "nucleotide sequence", or "nucleic acid fragment" are used interchangeably to refer to a polymer of RNA or DNA that is single- or double-stranded, optionally containing synthetic, non-natural or altered nucleotide bases. Nucleotides (usually found in their 5'-monophosphate form) are referred to by their single letter designation as follows: "A" for adenylate or deoxyadenylate (for RNA or DNA, respectively), "C" for cytidylate or deoxycytidylate, "G" for guanylate or deoxyguanylate, "U" for uridylate, "T" for deoxythymidylate, "R" for purines (A or G), "Y" for pyrimidines (C or T), "K" for G or T, "H" for A or C or T, "I" for inosine, and "N" for any nucleotide. Although nucleotide sequences herein may be presented as DNA sequences (comprising T), when referring to RNA, one skilled in the art can readily determine the corresponding RNA sequence (i.e., by replacing T with U).

"Polypeptide", "peptide", "amino acid sequence" and "protein" are used interchangeably herein to refer to a polymer of amino acid residues. The terms apply to amino acid polymers in which one or more amino acid residue is an artificial chemical analogue of a corresponding naturally occurring amino acid, as well as to naturally occurring amino acid polymers. The terms "polypeptide", "peptide", "amino acid sequence", and "protein" are also inclusive of modifications including, but not limited to, glycosylation, lipid attachment, sulfation, gamma-carboxylation of glutamic acid residues, hydroxylation and ADP-ribosylation.

Sequence "identity" has recognized meaning in the art, and the percentage of sequence identity between two nucleic acids or polypeptide molecules or regions can be calculated using the disclosed techniques. Sequence identity can be measured along the entire length of a polynucleotide or polypeptide or along a region of the molecule. (See, for example, Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991)_{∘} Although there are many methods for measuring the identity between two polynucleotides or polypeptides, the term "identity" is well known to the skilled person (Carrillo, H. & Lipman, D., SIAM J Applied Math 48: 1073 (1988)).

Suitable conserved amino acid replacements in peptides or proteins are known to those skilled in the art and can generally be carried out without altering the biological activity of the resulting molecule. In general, one skilled in the art recognizes that a single amino acid replacement in a non-essential region of a polypeptide does not substantially alter biological activity (See, for example, Watson et al., Molecular Biology of the Gene, 4th Edition, 1987, The Benjamin/Cummings Pub. co., p.224).

As used herein, an "expression construct" or "construct" refers to a vector suitable for expression of a nucleotide sequence of interest in an organism, such as a recombinant vector. "Expression" refers to the production of a functional product. For example, the expression of a nucleotide sequence may refer to transcription of a nucleotide sequence (such as transcribe to produce an mRNA or a functional RNA) and/or translation of RNA into a protein precursor or a mature protein.

"Expression construct" of the invention may be a linear nucleic acid fragment, a circular plasmid, a viral vector, or, in some embodiments, an RNA that can be translated (such as an mRNA).

"Expression construct" of the invention may comprise regulatory sequences and nucleotide sequences of interest that are derived from different sources, or regulatory sequences and nucleotide sequences of interest derived from the same source, but arranged in a manner different than that normally found in nature.

"Regulatory sequence" or "regulatory element" are used interchangeably and refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences may include, but are not limited to, promoters, translation leader sequences, introns, and polyadenylation recognition sequences.

"Promoter" refers to a nucleic acid fragment capable of controlling the transcription of another nucleic acid fragment. In some embodiments of the present invention, the promoter is a promoter capable of controlling the transcription of a gene in a cell, whether or not it is derived from the cell. The promoter may be a constitutive promoter or a tissue-specific promoter or a developmentally-regulated promoter or an inducible promoter.

"Constitutive promoter" refers to a promoter that may cause expression of a gene in most circumstances in most cell types. "Tissue-specific promoter" and "tissue-preferred promoter" are used interchangeably, and refer to a promoter that is expressed predominantly but not necessarily exclusively in one tissue or organ, but that may also be expressed in one specific cell or cell type. "Developmentally regulated promoter" refers to a promoter whose activity is determined by developmental events. "Inducible promoter" selectively expresses a DNA sequence operably linked to it in response to an endogenous or exogenous stimulus (environment, hormones, or chemical signals, and so on).

As used herein, the term "operably linked" means that a regulatory element (for example but not limited to, a promoter sequence, a transcription termination sequence, and so on) is associated to a nucleic acid sequence (such as a coding sequence or an open reading frame), such that the transcription of the nucleotide sequence is controlled and regulated by the transcriptional regulatory element. Techniques for operably linking a regulatory element region to a nucleic acid molecule are known in the art.

"Introduction" of a nucleic acid molecule (e.g., plasmid, linear nucleic acid fragment, RNA, etc.) or protein into an organism means that the nucleic acid or protein is used to transform a cell of the organism such that the nucleic acid or protein functions in the cell. As used in the present invention, "transformation" includes both stable and transient transformations.

"Stable transformation" refers to the introduction of an exogenous nucleotide sequence into the genome, resulting in the stable inheritance of foreign genes. Once stably transformed, the exogenous nucleic acid sequence is stably integrated into the genome of the organism and any of its successive generations.

"Transient transformation" refers to the introduction of a nucleic acid molecule or protein into a cell, performing its function without the stable inheritance of an exogenous gene. In transient transformation, the exogenous nucleic acid sequence is not integrated into the genome.

"Trait" refers to the physiological, morphological, biochemical, or physical characteristics of a cell or an organism.

"Agronomic trait" is a measurable parameter including but not limited to, leaf greenness, yield, growth rate, biomass, fresh weight at maturation, dry weight at maturation, fruit yield, seed yield, total plant nitrogen content, fruit nitrogen content, seed nitrogen content, nitrogen content in a vegetative tissue, total plant free amino acid content, fruit free amino acid content, seed free amino acid content, free amino acid content in a vegetative tissue, total plant protein content, fruit protein content, seed protein content, protein content in a vegetative tissue, drought tolerance, nitrogen uptake, root lodging, harvest index, stalk lodging, plant height, ear height, ear length, disease resistance, cold resistance, salt tolerance, and tiller number and so on.

### 2. Genome editing system

The present invention provides a novel class of CRISPR effector proteins, which have the targeted cutting activity of both TnpB system and CRISPR system, that is, they can target and bind to target DNA under the guidance of reRNA, and can also binds to the target DNA guided by guide RNA comprising tracrRNA and/or crRNA, such as sgRNA. Such subtypes of CRISPR nucleases are also referred to herein as TraC (intermediates between transposon and CRISPR-Cas12) effector proteins.

Accordingly, in one aspect, the invention provides an n engineered clustered regularly interspaced short palindromic repeat (CRISPR) system comprising:
a) a TraC (intermediates between Transposon and CRISPR-Cas12) effector protein or one or more nucleotide sequences encoding such effector protein; and
b) one or more guide RNAs, or a nucleotide sequence encoding the one or more guide RNAs,

wherein the guide RNA is selected from i) a guide RNA derived from a transposon right-element (reRNA) and/or ii) a guide RNA comprising tracrRNA and/or crRNA, such as a single guide RNA (sgRNA) comprising tracrRNA and crRNA;
the TraC effector protein can form a CRISPR complex with the guide RNA;
the TraC effector protein can target and bind to a target DNA sequence under the guidance of the guide RNA derived from transposon right element (reRNA), and can also target and bind to a target DNA sequence under the guidance of the guide RNA comprising tracrRNA and/or crRNA.

In some embodiments, the engineered clustered regularly interspaced short palindromic repeat (CRISPR) system is a genome editing system for genome editing in an organism or a cell of an organism.

In some embodiments, the TraC effector protein is as defined below.

In some embodiments, the tracrRNA contains a non-targeting strand binding sequence (NTB) that is complementary to a non-targeting strand (NTS).

In one aspect, the present invention also provides an engineered regularly interspaced short palindromic repeat sequence CRISPR vector system comprising one or more constructs, comprising:
a) a first regulatory element operably linked to a nucleotide sequence encoding a TraC (intermediates between Transposon and CRISPR-Cas12) effector protein; and
b) a second regulatory element operably linked to one or more nucleotide sequences encoding one or more guide RNAs selected from i) a guide RNA derived from a transposon right-element (reRNA) and/or ii) a guide RNA comprising tracrRNA and/or crRNA, such as a single guide RNA (sgRNA) comprising tracrRNA and crRNA;

the TraC effector protein can form a CRISPR complex with the guide RNA;
the TraC effector protein can target and bind to a target DNA sequence under the guidance of the guide RNA derived from a transposon right element, and can also target and bind to a target DNA sequence under the guidance of the guide RNA comprising tracrRNA and/or crRNA.

In some embodiments, the TraC effector protein is as defined below.

In some embodiments, the tracrRNA contains a non-targeting strand binding sequence (NTB) that is complementary to a non-targeting strand (NTS).

In some embodiments, the guide RNAis a guide RNA comprising tracrRNA and/or crRNA, wherein the tracrRNA contains a non-targeting strand binding sequence (NTB) complementary to the non-targeting strand (NTS), wherein the guide RNA hybridizes to the targeting strand (TS) of the target DNA sequence via the crRNA, and hybridizes to the non-targeting strand (NTS) via the NTB.

In some embodiments, when transcribed, the one or more guide RNAs hybridize to the target DNA, and the guide RNA forms a complex with the TraC effector protein that causes the cleavage at distal end of the target DNA sequence.

In some embodiments, the target DNA sequence is within a cell, preferably within a eukaryotic cell.

In some embodiments, the effector protein comprises one or more nuclear localization sequences (NLS), cytoplasmic localization sequences, chloroplast localization sequences or mitochondrial localization sequences.

In some embodiments, the nucleic acid sequence encoding the effector protein is codon-optimized for expression in eukaryotic cells.

In some embodiments, components a) and b) or their nucleotide sequences are constructed on the same or different vectors.

In one aspect, the present invention provides a method for modifying a target DNA sequence, the method comprising delivering the system of the invention to the target DNA sequence or into a cell containing the target DNA sequence.

In one aspect, the present invention provides a method of modifying a target DNA sequence, the method comprising delivering a composition of a TraC effector protein and one or more nucleic acid components to the target DNA sequence, wherein the effector protein can target and bind to the target DNA sequence under the guidance of the guide RNA derived from a transposon right element, and can also target and bind to the target DNA sequence under the guidance of the guide RNA comprising tracrRNA and/or crRNA; the effector protein and the one or more nucleic acid components form a CRISPR complex, and upon targeted binding of said complex to the target DNA sequence that is 3' of a prospacer adjacent motif (PAM), the effector protein induces modification of the target DNA sequence.

In some embodiments, the target gene is within a cell, preferably within a eukaryotic cell.

In some embodiments, the cell is an animal cell or a human cell.

In some embodiments, the cell is a plant cell.

In some embodiments, the effector protein comprises one or more nuclear localization sequences (NLS), cytoplasmic localization sequences, chloroplast localization sequences or mitochondrial localization sequences.

In some embodiments, the effector protein and nucleic acid component, or a construct expressing the effector protein and nucleic acid component, are contained in a delivery system.

In some embodiments, the delivery system comprises virus, virus-like particle, virosome, liposome, vesicle, exosome, liposomal nanoparticle (LNP), N-acetylgalactosamine ( GalNAc) or engineered bacteria.

In one aspect, the present invention provides a TraC (intermediates between Transposon and CRISPR-Cas12) effector protein or functional variant thereof for genome editing in an organism or an organism cell, wherein the TraC effector protein or a functional variant thereof can form a CRISPR complex with a guide RNA;
the guide RNA is selected from i) a guide RNA derived from a transposon right element (reRNA) and/or ii) a guide RNA comprising tracrRNA and/or crRNA, such as a single guide RNA (sgRNA) comprising tracrRNA and crRNA;
the TraC effector protein or functional variant thereof can target and bind to the target DNA sequence under the guidance of the guide RNA derived from a transposon right element, and can also target and bind to the target DNA sequence under the guidance of the guide RNA comprising tracrRNA and/or crRNA.

In one aspect, the present invention provides a TraC (intermediates between Transposon and CRISPR-Cas12) effector protein or functional variant thereof for genome editing in an organism or a cell of an organism, the TraC effector protein or functional variant thereof
(i) comprises at least one, at least two or all three amino acid sequence motifs selected from the group consisting of "TSxxCxxCx", "GIDRG" and "CxxCGxxxxADxxAA", wherein x represents any amino acid, such as any naturally encoded amino acid;
(ii) comprises an amino acid sequence that are at least 30%, at least 35%, at least 40%, at least 45%, at least 50%, at least 55%, at least 60%, at least 65%, at least 70%, at least 75%, at least 80%, at least 85%, at least 90%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, at least 99.9%, even 100% identical to any one of SEQ ID NOs: 1-37, or comprises an amino acid sequence having one or more, for example, 1, 2, 3, 4, 5, 6, 7, 8, 9 or 10 amino acid substitutions, deletions or additions relative to any one of SEQ ID NOs: 1-37.

In some embodiments, said effector protein functional variant is derived from SEQ ID NO: 25, and comprises one or more amino acid substitutions selected from K78R, D86R, S137R, V145R, I147R, P148R, D150R, V228R, V254R, A510R, A278R, K315R, S334R, L343R, A369R, H392R, L394R, S408R, N456R, V500R, A510R, T573R, relative to the sequence of SEQ ID NO: 25.

In some embodiments, the effector protein functional variant is derived from SEQ ID NO: 25, and comprises any set of mutations shown in Table 3 or 4 relative to the sequence of SEQ ID NO: 25.

In some embodiments, the effector protein functional variant comprises an amino acid sequence selected from SEQ ID NOs: 80-87.

In some embodiments, the TraC effector protein or functional variant thereof has at least the guide RNA-mediated sequence-specific targeting ability. That is, the TraC effector protein or functional variant thereof is capable of forming a complex with a guide RNA and binding to a specific target sequence (e.g., a DNA target sequence).

In some embodiments, the TraC effector protein or functional variant thereof has the guide RNA-mediated sequence-specific targeting ability, and double-stranded nucleic acid (e.g., double-stranded DNA) cleavage activity. For example, after forming a complex with a guide RNA and binding to a specific target sequence (e.g., a DNA target sequence), the TraC effector protein or functional variant thereof is capable of cleaving the double-stranded nucleic acid (e.g., double-stranded DNA) within or near the target sequence, forming a double-strand break (DSB).

In some embodiments, the TraC effector protein or functional variant thereof has guide RNA-mediated sequence-specific targeting ability, and nickase activity. For example, after forming a complex with a guide RNA and binding to a specific target sequence (e.g., a DNA target sequence), the TraC effector protein or functional variant thereof is capable of creating a nick within or near the target sequence. A TraC effector protein or functional variant thereof with nickase activity is also referred to as a TraC nickase.

In some embodiments, the TraC effector protein or functional variant thereof has guide RNA-mediated sequence-specific targeting ability but does not have double-stranded nucleic acid cleavage activity and/or nickase activity. TraC effector protein or functional variant thereof that does not have double-stranded nucleic acid cleavage activity and/or nickase activity is also referred to as dead TraC effector protein.

"Guide RNA" and "gRNA" are used interchangeably herein to refer to a gene that is capable of forming a complex with a TraC effector protein or a functional variant thereof and that is capable of targeting said complex due to a certain identity to the target sequence. RNA molecules directed to a target sequence. In general, the gRNA of a CRISPR system targets the target sequence through base pairing between the crRNA and the complementary strand of the target sequence.

In the present invention, the guide RNA can be selected from i) a guide RNA (reRNA) derived from a transposon right element and/or ii) a guide RNA comprising tracrRNA and/or crRNA, such as a single guide RNA (sgRNA) comprising tracrRNA and crRNA.

In some embodiments, the TraC effector protein or functional variant thereof can target and bind to the target DNA sequence under the guidance of the guide RNA derived from a transposon right element, and can also target and bind to the target DNA sequence under the guidance of the guide RNA comprising tracrRNA and/or crRNA.

In some embodiments, the guide RNA is a guide RNA derived from a transposon right element (reRNA), for example, the reRNA comprises a scaffold sequence shown in SEQ ID NO:77 or 78. The form or sequence of the specific reRNA can vary depending on the specific TraC effector protein, and the design thereof can refer to Karvelis, T. et al. Transposon-associated TnpB is a programmable RNA-guided DNA endonuclease. Nature 599, 692-696 (2021).

In some embodiments, the guide RNA comprises tracrRNA and/or crRNA. In some embodiments, the guide RNA is a guide RNA formed by complementation of tracrRNA and crRNA. In some embodiments, the guide RNA is a single guide RNA (sgRNA) comprising tracrRNA and crRNA, wherein tracrRNA and crRNA are fused. In some embodiments, the guide RNA may only comprise crRNA, which may also be referred to as sgRNA. The form or sequence of the specific gRNA may vary depending on the specific TraC effector protein.

Herein, a guide RNA comprising tracrRNA and/or crRNA may also be referred to as a CRISPR system guide RNA. Guide RNAs comprising tracrRNA and/or crRNA are the conventional guide RNA formats for CRISPR systems. The sequence of tracrRNA and/or crRNA can be obtained by analyzing the sequence near the CRISPR effector locus. It is within the capabilities of those skilled in the art to analyze and obtain guide RNAs comprising tracrRNA and/or crRNA for CRISPR effector proteins.

In some embodiments, the guide RNA comprising tracrRNA and/or crRNA is derived or matured from the nucleotide sequence of one of SEQ ID NOs: 38-74.

In some embodiments, the guide RNA comprises tracrRNA and crRNA, such as a single guide RNA (sgRNA) comprising tracrRNA and crRNA. In some embodiments, the crRNA comprises a sequence identical to the target sequence immediately adjacent to the PAM(e.g., 3' to the PAM), thereby binding complementary to the opposite strand of the PAM (the targeting strand). In some embodiments, the tracrRNA comprises a sequence complementary to a sequence distal to the PAM (in the direction of the target sequence) (non-targeting strand binding sequence, NTB). In some embodiments, the non-targeting strand binding sequence is located at the 5' end of the tracrRNA.

The binding of NTB in tracrRNA and the sequence distal to PAM can help the effector protein-guide RNA complex to open the DNA region distal to PAM and improve editing efficiency.

In some embodiments, the complement of the non-targeting strand binding sequence is about 10 to about 50 nucleotides from the PAM, for example about 10, about 16, about 20, about 24, about 28, about 30, about 40 , or about 50 nucleotides, preferably about 20 nucleotides from the PAM. In some embodiments, the non-targeting strand binding sequence is about 5 to about 20 nucleotides, preferably about 8 to 12 nucleotides, more preferably about 10 nucleotides in length. In some embodiments, the complement of the non-targeting strand binding sequence at least partially overlaps with the target sequence. In some embodiments, the complement of the non-targeting strand binding sequence is comprised in the target sequence.

As used herein, "target sequence" refers to a sequence in the genome approximately 20 nucleotides in length characterized by flanking (e.g., 5' flanking) PAM (prospacer adjacent motif) sequences. In general, the PAM is required for the recognition of the target sequence by a complex of a CRISPR nuclease, such as a TraC effector protein of the invention or a functional variant thereof, with a guide RNA. Based on the presence of PAMs, one skilled in the art can readily determine target sequences in the genome that can be used for targeting. And depending on the position of the PAM, the target sequence can be located on any strand of the genomic DNA molecule, the crRNA-bound strand is called the targeting strand (TS), and the strand complementary to the targeting strand is called the non-targeting strand (NTS).

In some embodiments, the sgRNA comprises a scaffold sequence shown in SEQ ID NO:75 or 76.

In some embodiments, nucleotides 154-209 in the scaffold sequence shown in SEQ ID NO: 75 or nucleotides 92-147 in the scaffold sequence shown in SEQ ID NO: 76 are reprogrammable regions, which can be reprogrammed to a contain non-targeting strand-binding sequence (NTB).

In one aspect, the invention also provides a protein complex of the TraC effector protein or functional variant thereof and at least one other functional protein. In some embodiments, the TraC effector protein or functional variant thereof and the other functional protein form a protein complex via an affinity tag that mediates specific binding. In some embodiments, the other functional protein forms a protein complex with the TraC effector protein or functional variant thereof by specifically binding to a guide RNA.

In one aspect, the present invention also provides a fusion protein of the TraC effector protein or functional variant thereof o and at least one other functional protein.

In some embodiments, the other functional protein is a deaminase. Thus, the protein complex or fusion protein can be used for base editing in an organism or a cell of an organism. A protein complex or fusion protein comprising the TraC effector protein or functional variant thereof and a deaminase is also referred to as a base editor. In some embodiments, the protein complex or fusion protein may comprise one or more of the deaminase.

In some embodiments, the deaminase is a cytosine deaminase. "Cytosine deaminase" refers to a deaminase capable of accepting single-stranded DNA as substrate and catalyzing the deamination of cytidine or deoxycytidine to uracil or deoxyuracil, respectively. Examples of cytosine deaminase useful in the present invention include, but are not limited to, e.g., APOBEC1 deaminase, activation-induced cytidine deaminase (AID), APOBEC3G, CDA1, human APOBEC3A deaminase, double-stranded DNA deaminase (Ddd), single-stranded DNA deaminase (Sdd) (Ddd and Sdd refer to CN202310220057.1, PCT/CN2023/080052) or their functional variants. Each of said documents or patents is incorporated herein by reference in its entirety..

In some embodiments of the invention, the cytosine deaminase in the protein complex or fusion protein is capable of converting the cytidine in the single-stranded DNA produced in the formation of the protein complex- or fusion protein-guide RNA-DNA complex into U by deamination, and then realize the base replacement from C to T through base mismatch repair.

In some embodiments, the protein complex or fusion protein comprising cytosine deaminase further comprises a uracil DNA glycosylase inhibitor (UGI). Uracil DNA glycosylase catalyzes the removal of U from DNA and initiates base excision repair (BER), resulting in the repair of U:G to C:G. Therefore, without being bound by any theory, inclusion of a uracil DNA glycosylase inhibitor (UGI) in the protein complex or fusion protein of the present invention will be able to increase the efficiency of C to T base editing.

In some embodiments, the deaminase is an adenine deaminase. "Adenine deaminase" refers to a domain capable of accepting single-stranded DNA as substrate and catalyzing the formation of inosine (I) from adenosine or deoxyadenosine (A).

In the present invention, the adenine deaminase in the protein complex or fusion protein can convert the adenosine of single-stranded DNA produced in the formation of the protein complex- or fusion protein-guide RNA-DNA complex to inosine (I) by deamination, and since DNA polymerase treats inosine (I) as guanine (G), substitution of A to G can be achieved by base mismatch repair.

In some embodiments, the adenine deaminase is a DNA-dependent adenine deaminase derived from the E. coli tRNA adenine deaminase TadA (ecTadA).

In some embodiments, the protein complex or fusion protein includes a cytosine deaminase and an adenine deaminase.

In some embodiments, the other functional proteins can be a transcriptional activator protein, a transcriptional repressor protein, a DNA methylase, a DNA demethylase, etc., thereby enabling transcriptional regulation and/or epigenetic modification.

In some embodiments, the other functional protein can be a reverse transcriptase. The protein complex or fusion protein comprising the TraC effector protein or functional variant thereof and a reverse transcriptase can be used for the so-called prime editing. A protein complex or fusion protein comprising the TraC effector protein or a functional variant thereof and reverse transcriptase can be used for large fragment DNA insertion, such as a guide editor (prime editor) (Anzalone, A.V, Randolph, P.B., Davis, J.R. et al. Search-and-replace genome editing without double-strand breaks or donor DNA. Nature 576, 149-157 (2019).), PrimeRoot editor (PrimeRoot editor) (Sun, C., Lei, Y., Li, B. et al. Precise integration of large DNA sequences in plant genomes using PrimeRoot editors. Nat Biotechnol (2023). ), each of which is incorporated herein by reference in its entirety.

Different parts of the fusion protein of the present invention can be linked independently through a linker or directly. The linker described herein can be a non-functional amino acid sequence without secondary structure of 1-50 (e.g., 1, 2, 3, 4, 5, 6, 7, 8, 9, 10, 11, 12, 13, 14, 15, 16, 17, 18, 19, 20 or 20-25, 25-50), or more amino acids in length. For example, the linker may be a flexible liner.

In some embodiments of various aspects of the invention, the TraC effector protein or functional variant thereof or the other functional protein forming the protein complex or the fusion protein is recombinantly produced. In some embodiments of various aspects of the invention, the TraC effector protein or functional variant thereof or the other functional protein forming the protein complex or the fusion protein further contains a fusion tag, e.g., a tag for isolating and/or purifying the TraC effector protein or functional variant thereof or the other functional protein forming the protein complex or the fusion protein. Methods for recombinant production of proteins are known in the art. A variety of tags that can be used to isolate and/or purify proteins are known in the art, including but not limited to His tag, GST tag, and the like. Generally, these tags do not alter the activity of the protein of interest.

In some embodiments of aspects of the invention, the TraC effector protein or functional variant thereof or the other functional protein forming the protein complex or the fusion protein of the invention further comprises a nuclear localization sequence (NLS), for example, is linked to the nuclear localization sequence via a linker. In general, one or more NLSs in the TraC effector protein or functional variant thereof or the other functional protein forming the protein complex or the fusion protein should be of sufficient strength to drive the accumulation of TraC effector protein or functional variant thereof or the other functional protein forming the protein complex or the fusion protein of the invention in the nucleus in an amount that can achieve its genome editing function. In general, the strength of nuclear localization activity is determined by the number, location, or one or more specific NLSs as used in the TraC effector protein or functional variant thereof or the other functional protein forming the protein complex or the fusion protein of the invention, or a combination of these factors. Exemplary nuclear localization sequences include, but are not limited to, the SV40 nuclear localization signal sequence, the nucleoplasmin nuclear localization signal sequence. In addition, according to the DNA position to be edited, the TraC effector protein or functional variant thereof or the other functional protein forming the protein complex or the fusion protein of the invention may also include other localization sequences, such as cytoplasmic localization sequences, chloroplast localization sequences, mitochondrial localization sequences, etc..

In one aspect, the present invention provides use of the TraC effector protein or functional variant thereof or said other functional protein forming the protein complex or said fusion protein of the present invention for genome editing in a cell, preferably a eukaryotic cell, more preferably a plant cell.

In one aspect, the present invention provides a genome editing system for site-directed modification of a target nucleic acid sequence in the genome of a cell, comprising the TraC effector protein or functional variant thereof or said other functional protein forming the protein complex or said fusion protein of the present invention and/or an expression construct comprising a nucleotide sequence encoding the TraC effector protein or functional variant thereof or said other functional protein forming the protein complex or said fusion protein of the present invention.

As used herein, the terms "genome editing system" and "gene editing system" are used interchangeably and refer to a combination of components required for genome editing of the genome within a cell of an organism, wherein the individual components of the system, such as the TraC effector protein or functional variant thereof or the other functional protein forming the protein complex or the fusion protein, gRNA or the corresponding expression constructs, etc. can each exist independently, or can be included in a composition in any combination. In some embodiments, the components of the genome editing system are contained in a delivery system selected from the group consisting of viruses, virus-like particles, virosomes, liposomes, vesicles, exosomes, liposomal nano Granules (LNP), N-acetylgalactosamine (GalNAc) or engineered bacteria.

In some embodiments, the genome editing system further comprises at least one guide RNA (gRNA) and/or an expression construct comprising a nucleotide sequence encoding the at least one guide RNA.

In some embodiments, the guide RNA is selected from i) a guide RNA derived from a transposon right element (reRNA) and/or ii) a guide RNA comprising tracrRNA and/or crRNA, such as a single-guide RNA (sgRNA) comprising tracrRNA and crRNA.

In some embodiments, the guide RNA is a single guide RNA (sgRNA) comprising tracrRNA and crRNA, for example, the sgRNA comprises a scaffold sequence shown in SEQ ID NO:75 or 76.

In some embodiments, the guide RNA derived from the CRISPR system comprises tracrRNA and crRNA, such as a single guide RNA (sgRNA) comprising tracrRNA and crRNA. In some embodiments, the crRNA comprises a sequence identical to the target sequence immediately adjacent to the PAM, thereby binding complementary to the opposite strand of the PAM. In some embodiments, the tracrRNA comprises a sequence (non-targeting strand binding sequence, NTB) complementary to a sequence distal to the PAM in the target sequence direction. In some embodiments, the non-targeting strand binding sequence is located at the 5' end of the tracrRNA.

In some embodiments, the complement of the non-targeting strand binding sequence is about 10 to about 50 nucleotides from the PAM, for example about 10, about 16, about 20, about 24, about 28, about 30, about 40 , or about 50 nucleotides, preferably about 20 nucleotides from the PAM. In some embodiments, the non-targeting strand binding sequence is about 5 to about 20 nucleotides, preferably about 8 to 12 nucleotides, more preferably about 10 nucleotides in length. In some embodiments, the complement of the non-targeting strand binding sequence at least partially overlaps with the target sequence. In some embodiments, the complement of the non-targeting strand binding sequence is comprised in the target sequence.

In some embodiments, nucleotides 154-209 in the scaffold sequence shown in SEQ ID NO: 75 or nucleotides 92-147 in the scaffold sequence shown in SEQ ID NO: 76 are reprogrammable regions, which can be reprogrammed to contain a non-targeting strand-binding sequence (NTB).

In general, the 5' or 3' end of the target sequence targeted by the genome editing system of the present invention needs to contain a protospacer adjacent motif (PAM). The form or sequence of the specific PAM will vary depending on the specific TraC effector protein .

In some embodiments, when used for prime editing, the gRNA can be a so-called pegRNA. The pegRNA additionally contains a reverse transcription template (RT) sequence and a primer binding site (PBS) sequence on the basis of sgRNA.

In some embodiments, the PAMs recognized by the TraC effector protein or functional variant thereof of the invention are T-rich PAMs. In some embodiments, the PAMs recognized by the TraC effector protein or functional variant thereof of the invention are G-rich PAMs. The PAM may be, for example, 5'-TTTN-3', 5'-TGTNNN-3', PolyT, PolyG, 5'-TTTG-3', 5'-TTC-3', 5'-TGA-3', 5'-YTTC-3', 5'-CTCGTG-3', 5'-GTTG-3', 5'-CTTG-3', 5'-TCTG-3', 5'-TTTA-3', 5' - TTAG-3', where N represents A, G, C or T and Y represents C or G.

Based on the presence of PAMs, those skilled in the art can readily determine target sequences in the genome that can be used for targeting and optionally editing and design suitable guide RNAs accordingly. For example, if there is a PAM sequence 5'-TTC-3' in the genome, about 18 to about 35, preferably 20, 21, 22 or 23 consecutive nucleotides immediately adjacent to 5' or 3' of the PAM can be used as the target sequence.

In some embodiments, the at least one guide RNA is encoded by different expression constructs. In some embodiments, the at least one guide RNA is encoded by a same expression construct. In some embodiments, the at least one guide RNA and the TraC effector protein or functional variant thereof or the fusion protein of the invention are encoded by a same expression construct.

For example, in some embodiments, the genome editing system can comprise any one selected from the group consisting of:
i) the TraC effector protein or functional variant thereof of the invention or the fusion protein of the invention, and the at least one guide RNA, optionally, the TraC effector protein or functional variant thereof or the fusion protein forms a complex with the at least one guide RNA;
ii) an expression construct comprising a nucleotide sequence encoding the TraC effector protein or functional variant thereof of the invention or a fusion protein of the invention, and the at least one guide RNA;
iii) the TraC effector protein or functional variant thereof of the invention or the fusion protein of the invention, and an expression construct comprising a nucleotide sequence encoding the at least one guide RNA;
iv) an expression construct comprising a nucleotide sequence encoding the TraC effector protein or functional variant thereof of the invention or a fusion protein of the invention, and an expression construct comprising a nucleotide sequence encoding the at least one guide RNA;
v) an expression construct comprising a nucleotide sequence encoding the TraC effector protein or functional variant thereof of the invention or the fusion protein of the invention, and a nucleotide sequence encoding the at least one guide RNA.

In some embodiments, the genome editing system further comprises a donor nucleic acid molecule comprising a nucleotide sequence to be site-directed inserted into the genome. In some embodiments, the nucleotide sequence to be inserted into the genome contains flanking sequences homologous to the sequences flanking the target sequence in the genome. After editing, the nucleotide sequence to be site-directed inserted into the genome can be integrated into the genome by homologous recombination.

In order to obtain efficient expression in the cell, in some embodiments of the present invention, the nucleotide sequence encoding the TraC effector protein or functional variant thereof or the other functional protein forming the protein complex or the fusion protein is codon-optimized for the organism from which the cell to be edited is derived.

Codon optimization refers to a process of modifying a nucleic acid sequence for enhanced expression in the host cells of interest by replacing at least one codon (e.g. about or more than about 1, 2, 3, 4, 5, 10, 15, 20, 25, 50, or more codons) of the native sequence with codons that are more frequently or most frequently used in the genes of that host cell while maintaining the native amino acid sequence. Various species exhibit particular bias for certain codons of a particular amino acid. Codon bias (differences in codon usage between organisms) often correlates with the efficiency of translation of messenger RNA (mRNA), which is in turn believed to be dependent on, among other things, the properties of the codons being translated and the availability of particular transfer RNA (tRNA) molecules. The predominance of selected tRNAs in a cell is generally a reflection of the codons used most frequently in peptide synthesis. Accordingly, genes can be tailored for optimal gene expression in a given organism based on codon optimization. Codon usage tables are readily available, for example, at the"Codon Usage Database" available at www.kazusa.orjp/codon/ and these tables can be adapted in a number of ways. See Nakamura, Y., et al."Codon usage tabulated from the international DNA sequence databases: status for the year 2000" Nucl. Acids Res. 28:292 (2000).

The cell that can be genome edited by the TraC effector protein or functional variant thereof or the fusion protein or the genome editing system of the present invention may be from a prokaryote or an eukaryote, preferably from an eukaryote, includes but is not limited to a mammal such as human, mouse, rat, monkey, dog, pig, sheep, cattle, cat; poultry such as chicken , duck, goose; a plant, including a monocotyledonous plant and a dicotyledonous plant such as rice, corn, wheat, sorghum, barley, soybean, peanut, Arabidopsis and the like.

In some embodiments of the invention, the nucleotide sequence encoding the TraC effector protein or functional variant thereof or the fusion protein and/or the nucleotide sequence encoding the at least one guide RNA is operably linked to an expression regulatory element such as a promoter.

Examples of promoters that can be used in the present invention include but are not limited to polymerase (pol) I, pol II or pol III promoters. Examples of pol I promoters include chicken RNA pol I promoter. Examples of pol II promoters include but are not limited to cytomegalovirus immediate early (CMV) promoter, rous sarcoma virus long terminal repeat(RSV-LTR) promoter and simian virus 40(SV40) immediate early promoter. Examples of pol III promoters include U6 and H1 promoter. Inducible promoter such as metalothionein promoter can be used. Other examples of promoters include T7 bacteriophage promoter, T3 bacteriophage promoter, β-galactosidase promoter and Sp6 bacteriophage promoter etc. When used for plants, promoters that can be used include but are not limited to cauliflower mosaic virus 35S promoter, maize Ubi-1 promoter, wheat U6 promoter, rice U3 promoter, maize U3 promoter and rice actin promoter etc.

In some embodiments, for precise production of guide RNA within the cell, in the expression construct comprising a nucleotide sequence encoding the at least one guide RNA, the 5' end of the guide RNA coding sequence is linked to 3' end of a first ribozyme coding sequence, wherein the first ribozyme is designed to cleave the first ribozyme-guide RNA fusion RNA transcribed in the cell at the 5' end of the guide RNA, thereby forming a guide RNA that does not carry additional nucleotide at the 5' end. In one embodiment, the 3' end of the guide RNA coding sequence is linked to the 5' end of a second ribozyme coding sequence, wherein the second ribozyme is designed to cleave the guide RNA-second ribozyme fusion RNA transcribed in the cell at the 3' end of the guide RNA, thereby forming a guide RNA that does not carry additional nucleotide at the 3' end. In some embodiments, the 5' end of the guide RNA coding sequence is linked to the 3' end of a first ribozyme coding sequence, and the 3' end of the guide RNA coding sequence is linked to the 5' end of a second ribozyme coding sequence, wherein the first ribozyme is designed to cleave the first ribozyme-guide RNA-second ribozyme fusion RNA transcribed in the cell at the 5' end of the guide RNA, and the second ribozyme is designed to cleave the first ribozyme-guide RNA-second ribozyme fusion RNA transcribed in the cell at the 3' end of the guide RNA, thereby forming a guide RNA that does not carry additional nucleotides at the 5' and 3' ends.

The design of the first or second ribozyme is within the skill of those skilled in the art. See, for example, Gao et al., JIPB, Apr, 2014; Vol 56, Issue 4, 343-349.

In some embodiments, for precise production of guide RNA within the cell, in the expression construct comprising a nucleotide sequence encoding the at least one guide RNA, the 5' end of the guide RNA coding sequence is linked to 3' end of a first tRNA coding sequence, wherein the first tRNA is designed to cleave the first tRNA-guide RNA fusion RNA transcribed in the cell at the 5' end of the guide RNA (i.e., cleaved by the precise tRNA-processing machinery present within the cell that precisely excises the 5' and 3' extra sequences of the precursor tRNA to form the mature tRNA), thereby forming a guide RNA that does not carry additional nucleotide at the 5' end. In one embodiment, the 3' end of the guide RNA coding sequence is linked to the 5' end of a second tRNA coding sequence, wherein the second tRNA is designed to cleave the guide RNA-second tRNA fusion RNA transcribed in the cell at the 3' end of the guide RNA, thereby forming a guide RNA that does not carry additional nucleotide at the 3' end. In some embodiments, the 5' end of the guide RNA coding sequence is linked to the 3' end of a first tRNA coding sequence, and the 3' end of the guide RNA coding sequence is linked to the 5' end of a second tRNA coding sequence, wherein the first tRNA is designed to cleave the first tRNA-guide RNA-second tRNA fusion RNA transcribed in the cell at the 5' end of the guide RNA, and the second tRNA is designed to cleave the first tRNA-guide RNA-second tRNAfusion RNA transcribed in the cell at the 3' end of the guide RNA, thereby forming a guide RNA that does not carry additional nucleotides at the 5' and 3' ends.

The design of such tRNA-guide RNA fusions is within the skill of those skilled in the art. For example, see Xie et al., PNAS, Mar 17, 2015; vol. 112, no. 11, 3570-3575.

### 3. Method for site-directed modification of a target nucleic acid sequence in the genome of a cell

In another aspect, the present invention provides a method for site-directed modification of a target nucleic acid sequence in the genome of a cell, comprising introducing the genome editing system of the present invention into the cell.

In another aspect, the present invention also provides a method of producing a genetically modified cell, comprising introducing the genome editing system of the present invention into the cell.

In another aspect, the present invention also provides a genetically modified organism comprising the genetically modified cell produced by the method of the present invention or progeny cell thereof.

In the present invention, the target sequence to be modified may be located at any location in the genome, for example, in a functional gene such as a protein-encoding gene, or may be, for example, located in a gene expression regulatory region such as a promoter region or an enhancer region, thereby the gene functional modification or gene expression modification can be achieved. Modifications in the target sequence of the cell can be detected by T7EI, PCR/RE or sequencing methods.

In the methods of the present invention, the gene editing system can be introduced into cells by a variety of methods well known to those skilled in the art.

Methods that can be used to introduce a gene editing system of the present invention into a cell include, but are not limited to, calcium phosphate transfection, protoplast fusion, electroporation, lipofection, microinjection, viral infection (e.g., baculovirus, vaccinia virus, adenovirus, adeno-associated virus, lentivirus and other viruses), gene gun method, PEG-mediated protoplast transformation, Agrobacterium-mediated transformation.

In some embodiments, the method of the invention is performed in vitro. For example, the cell is an isolated cell, or a cell in an isolated tissue or organ.

In some other embodiments, the method of the present invention can also be performed in vivo. For example, the cell is a cell within an organism, and the system of the invention can be introduced in vivo by, for example, a virus or Agrobacterium-mediated method.

The cell that can be genome-edited by the method of the present invention is derived from a prokaryote or an eukaryote, including but not limited to, a mammal such as human, mouse, rat, monkey, dog, pig, sheep , cattle, cat; poultry such as chicken, duck, goose; a plant including monocotyledonous and dicotyledonous plant, such as rice, corn, wheat, sorghum, barley, soybean, peanut, Arabidopsis, etc.

Accordingly, the present invention provides a method of producing a genetically modified plant comprising introducing the genome editing system of the present invention into at least one plant, thereby resulting in a modification in the genome of said at least one plant.

In the method of the present invention, the genome editing system may be introduced into the plant with a variety of methods well known to those skilled in the art. The methods used for introducing the genome editing system of the present invention into a plant include, but are not limited to gene gun method, PEG-mediated protoplast transformation, Agrobacterium-mediated transformation, plant virus-mediated transformation, pollen tube pathway and ovary injection method.

In the method of the present invention, the modification of the target sequence can be achieved by only introducing or producing the TraC effector protein or functional variant thereof or the fusion protein and the guide RNA in the plant cell, and the modification can be stably inherited, without any need to stably transform the genome editing system into the plant. This avoids the potential off-target effect of the stable genome editing system and avoids the integration of the exogenous nucleotide sequence in the plant genome, thereby providing greater biosafety.

**In** some preferred embodiments, the introduction is carried out in the absence of selection pressure to avoid integration of the exogenous nucleotide sequence into the plant genome.

**In** some embodiments, the introduction comprises transforming the genome editing system of the present invention into an isolated plant cell or tissue and then regenerating the transformed plant cell or tissue into an intact plant. Preferably, the regeneration is carried out in the absence of selection pressure, i.e., no selection agent for the selection gene on the expression vector is used during tissue culture. Avoiding the use of a selection agent can increase the regeneration efficiency of the plant, obtaining a modified plant free of exogenous nucleotide sequences.

**In** some other embodiments, the genome editing system of the present invention can be transformed into specific parts of an intact plant, such as leaves, shoot tips, pollen tubes, young ears or hypocotyls. This is particularly suitable for the transformation of plants that are difficult to regenerate in tissue culture.

**In** some embodiments of the invention, the in vitro expressed protein and/or the in vitro transcribed RNA molecule are directly transformed into the plant. The protein and/or RNA molecule is capable of performing genome editing in plant cells and is subsequently degraded by the cell, avoiding integration of the exogenous nucleotide sequence in the plant genome.

Thus, in some embodiments, genetic modification of a plant using the method of the present invention may result in a plant whose genome is free of integration of exogenous polynucleotide, i.e., a transgene-free modified plant.

**In** some embodiments, the method further comprises treating (such as culturing) the plant cell, tissue or intact plant into which the genome editing system of the invention has been introduced at an elevated temperature (relative to the temperature of conventional cultivation, such as room temperature), the elevated temperature is for example 32°C. **In** some preferred embodiments, the plant is rice.

**In** some embodiments of the present invention, the modification is associated with plant traits, such as agronomic traits. For example, the modification results in a plant having altered (preferably improved) traits, such as agronomic traits, relative to a wild type plant.

**In** some embodiments, the method further comprises the step of screening a plant having the desired modification and/or desired traits, such as agronomic traits.

In some embodiments of the present invention, the method further comprises the step of obtaining progenies of the genetically modified plant. Preferably, the genetically modified plant or the progenies thereof have the desired modification and/or the desired traits, such as agronomic traits.

In another aspect, the invention also provides a genetically modified plant or progenies or a part thereof, wherein the plant is obtained by the method according to the present invention as described above. In some embodiments, the genetically modified plant or the progenies or a part thereof is transgene-free. Preferably, the genetically modified plant or progenies thereof have the desired genetic modification and/or the desired traits, such as agronomic traits.

In another aspect, the present invention provides a method of plant breeding comprising crossing a first genetically modified plant obtained by the above method of the present invention with a second plant not containing the modification, thereby the genetic modification is introduced into the second plant. Preferably, the first genetically modified plant has the desired traits, such as the agronomic traits.

### 4. Therapeutic application

The present invention also covers the application of the genome editing system of the present invention in the treatment of diseases.

By modifying disease-related genes through the genome editing system of the present invention, the up-regulation, down-regulation, inactivation, activation or mutation correction of disease-related genes can be realized, thereby realizing the prevention and/or treatment of diseases. For example, the genome modification described in the present invention can be located in the protein coding region of the disease-related gene, or can be located in the gene expression regulatory region such as the promoter region or enhancer region, so that the functional modification of the disease-related gene or the modification of disease-associated gene expression can be ahieved. Therefore, the modification of the disease-related genes described herein includes the modification of the disease-related genes themselves (such as protein coding regions), and also includes the modification of their expression regulation regions (such as promoters, enhancers, introns, etc.).

A "disease-related" gene refers to any gene whose transcription or translation product is produced at an abnormal level or in an abnormal form in cells derived from a disease-affected tissue as compared to non-disease control tissues or cells. Where altered expression is associated with the onset and/or progression of a disease, it may be a gene that is expressed at abnormally high levels; it may be a gene that is expressed at abnormally low levels. A disease-related gene also refers to a gene that has one or more mutations or genetic variations that are directly responsible or in linkage disequilibrium with one or more genes responsible for the etiology of the disease. Said mutation or genetic variation is for example a single nucleotide variation (SNV). Transcribed or translated products may be known or unknown, and may be at normal or abnormal levels.

Accordingly, the present invention also provides a method of treating a disease in a subject in need thereof, comprising delivering to the subject an effective amount of the genome editing system of the present invention to modify a gene related to the disease. The present invention also provides a use of a genome editing system for preparing a pharmaceutical composition for treating a disease in a subject in need thereof, wherein the genome editing system is used to modify a gene related to the disease. The present invention also provides a pharmaceutical composition for treating a disease in a subject in need thereof, which comprises the genome editing system of the present invention, and optionally a pharmaceutically acceptable carrier, wherein the genome editing system is used to modify the disease- related genes.

Preferably, the "subject" of the present invention is a mammal, such as a human.

In some embodiments, the genome editing system described herein is used to introduce point mutations into a nucleic acid.

In some embodiments, the genome editing system described herein is used to cause correction of a genetic defect, for example correcting a point mutation that results in a loss of function in a gene product. In some embodiments, the genetic defect is associated with a disease or disorder (eg, a lysosomal storage disease or a metabolic disease, such as, eg, type 1 diabetes). In some embodiments, the methods provided herein can be used to introduce inactivating point mutations into genes or alleles encoding gene products associated with a disease or disorder.

In some embodiments, the protocols described herein are intended for use in the treatment of patients with diseases associated with or caused by point mutations that can be corrected by the genome editing systems provided herein. In some embodiments, the disease is a proliferative disease. In some embodiments, the disease is a genetic disease. In some embodiments, the disease is a neoplastic disease. In some embodiments, the disease is a metabolic disease. In some embodiments, the disease is a lysosomal storage disease.

In some embodiments, the embodiments described herein are intended to be useful in the treatment of mitochondrial diseases or disorders. As used herein, "mitochondrial disease" relates to diseases caused by abnormal mitochondria, such as mitochondrial gene mutations, enzyme pathways, and the like. Examples of disorders include, but are not limited to: neurological disorders, loss of motor control, muscle weakness and pain, gastrointestinal disorders and difficulty swallowing, poor growth, heart disease, liver disease, diabetes, respiratory complications, epilepsy, vision/hearing problems, lactic acid toxic, stunted and susceptible to infection.

Examples of diseases described in the present invention include, but are not limited to, genetic diseases, circulatory system diseases, muscle diseases, brain, central nervous and immune system diseases, Alzheimer's disease, secretase disorders, amyotrophic lateral sclerosis (ALS ), autism, trinucleotide repeat expansion disorders, hearing disorders, gene-targeted therapy of non-dividing cells (neurons, muscles), liver and kidney diseases, epithelial and lung diseases, cancer, Usher syndrome or retinitis pigmentosa-39, cystic fibrosis, HIV and AIDS, beta thalassemia, sickle cell disease, herpes simplex virus, autism, drug addiction, age-related macular degeneration, schizophrenia . Other diseases to be treated by correcting point mutations or introducing inactivating mutations into disease-associated genes are known to those skilled in the art, and thus the present disclosure is not limited in this respect. In addition to the diseases exemplarily described in the present invention, the strategy and genome editing system provided by the present invention can also be used to treat other related diseases, and the application will be obvious to those skilled in the art. The applicable diseases or targets of the present invention refer to WO2015089465A1 (PCT/US2014/070135), WO2016205711A1 (PCT/US2016/038181), WO2018141835A1 (PCT/EP2018/052491), WO2020191234A1 (PCT/US2 020/023713), WO2020191233A1 (PCT/ Related diseases for which the genome editing system listed in US2020/023712), WO2019079347A1 (PCT/US2018/056146), WO2021155065A1 (PCT/US2021/015580) is applicable.

Administration of the genome editing system or pharmaceutical composition of the invention can be tailored to the weight and species of the patient or subject. The frequency of administration is within the range permitted by medical or veterinary medicine. It depends on conventional factors including the patient's or subject's age, sex, general health, other conditions, and the particular condition or symptom being addressed.

### 5. Kit

The present invention also includes a kit for use in the method of the present invention, the kit comprising the genome editing system of the present invention, and instructions for use. The kit generally includes a label indicating the intended use and/or method for use of the contents of the kit. The term label includes any written or recorded material provided on or with the kit or otherwise provided with the kit.

### Examples

### Example 1. Discovery of novel CRISPR system by bioinformatics

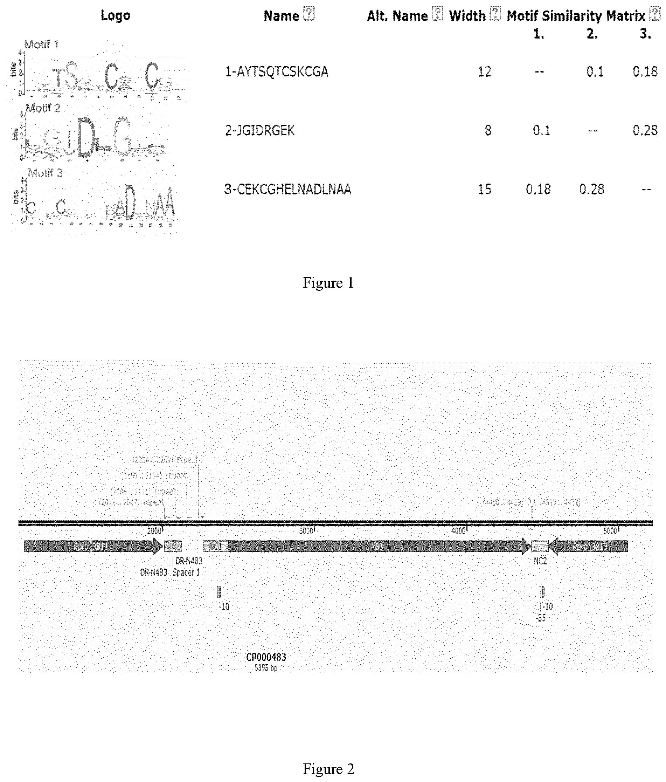

First, we developed a more specific strategy for identifying genes encoding CRISPR effector proteins to identify genes with shared highly conserved motifs. We used the MEME motif software to predict the conserved domains of the 86 known Cas12b-Cas12i family Cas12 proteins, and found three structural motifs conserved in the 86 Cas12 proteins (Figure 1), respectively "TSxxCxxCx" , "GIDRG", and "CxxCGxxxxADxxAA".

Then we searched the microbial genome/metagenomic data in the published NCBI public database, preliminarily searched all proteins within 10kb upstream and downstream of 32,562 CRISPR arrays in the GTDB database, and selected 166 candidate proteins with at least two of the three conservative motifs shown. In the next step, candidate proteins with the same type as the annotated proteins or similar to the annotated protein sequences were filtered out through CRISPR type analysis and protein similarity analysis, and 37 new proteins (SEQ ID NO:1 - 37) were obtained. These proteins are defined as intermediates between Transposon and CRISPR-Cas12 (TraC). Correspondingly, the CRISPR system with TraC as the effector protein is defined as the CRISPR-TraC system.

The prokaryotic expression system of TraC proteins is shown in Figure 2 as an example. The prokaryotic expression of the TraC-N483 protein is exemplarily shown in Fig. 2. 483 in the figure indicates the name of the new protein, and repeat is the CRISPR locus region. NC1 and NC2 are non-coding RNA regions where tracrRNA may exist. When synthesizing the gene, the pTac promoter was used to drive the expression of the protein gene, and J22119 was used to promote the expression of the Repeat-spacer-repeat-noncoding sequence (SEQ ID NO: 38-74).

**Table 1: Sequence information table**

| SEQ ID NO: | Description |
|---|---|
| 1 | TraC-365 amino acid sequence |
| 2 | TraC-445 amino acid sequence |
| 3 | TraC-515 amino acid sequence |
| 4 | TraC-655 amino acid sequence |
| 5 | TraC-685 amino acid sequence |
| 6 | TraC-685-2 amino acid sequence |
| 7 | TraC-875 amino acid sequence |
| 8 | TraC-975 amino acid sequence |
| 9 | TraC-N287 amino acid sequence |
| 10 | TraC-N483 amino acid sequence |
| 11 | TraC-N510 amino acid sequence |
| 12 | TraC-N607 amino acid sequence |
| 13 | TraC-N611 amino acid sequence |
| 14 | TraC-N701 amino acid sequence |
| 15 | TraC-N929 amino acid sequence |
| 16 | TraC-079 amino acid sequence |
| 17 | TraC-081 amino acid sequence |
| 18 | TraC-102 amino acid sequence |
| 19 | TraC-153 amino acid sequence |
| 20 | TraC-205 amino acid sequence |
| 21 | TraC-252 amino acid sequence |
| 22 | TraC-297 amino acid sequence |
| 23 | TraC-393 amino acid sequence |
| 24 | TraC-447 amino acid sequence |
| 25 | TraC-459 amino acid sequence |
| 26 | TraC-466 amino acid sequence |
| 27 | TraC-479 amino acid sequence |
| 28 | TraC-572 amino acid sequence |
| 29 | TraC-703 amino acid sequence |
| 30 | TraC-750 amino acid sequence |
| 31 | TraC-762 amino acid sequence |
| 32 | TraC-763 amino acid sequence |
| 33 | TraC-765 amino acid sequence |
| 34 | TraC-949 amino acid sequence |
| 35 | TraC-963 amino acid sequence |
| 36 | TraC-999 amino acid sequence |
| 37 | TraC-1105 amino acid sequence |
| 38 | TraC-365 crRNA and non-coding DNA sequence |
| 39 | TraC-445 crRNA and non-coding DNA sequence |
| 40 | TraC-515 crRNA and non-coding DNA sequence |
| 41 | TraC-655 crRNA and non-coding DNA sequence |
| 42 | TraC-685 crRNA and non-coding DNA sequence |
| 43 | TraC-685-2 crRNA and non-coding DNA sequence |
| 44 | TraC-875 crRNA and non-coding DNA sequence |
| 45 | TraC-975 crRNA and non-coding DNA sequence |
| 46 | TraC-N287 crRNA and non-coding DNA sequence |
| 47 | TraC-N483 crRNA and non-coding DNA sequence |
| 48 | TraC-N510 crRNA and non-coding DNA sequence |
| 49 | TraC-N607 crRNA and non-coding DNA sequence |
| 50 | TraC-N611 crRNA and non-coding DNA sequence |
| 51 | TraC-N701 crRNA and non-coding DNA sequence |
| 52 | TraC-N929 crRNA and non-coding DNA sequence |
| 53 | TraC-079 crRNA and non-coding DNA sequence |
| 54 | TraC-081 crRNA and non-coding DNA sequence |
| 55 | TraC-102 crRNA and non-coding DNA sequence |
| 56 | TraC-153 crRNA and non-coding DNA sequence |
| 57 | TraC-205 crRNA and non-coding DNA sequence |
| 58 | TraC-252 crRNA and non-coding DNA sequence |
| 59 | TraC-297 crRNA and non-coding DNA sequence |
| 60 | TraC-393 crRNA and non-coding DNA sequence |
| 61 | TraC-447 crRNA and non-coding DNA sequence |
| 62 | TraC-459 crRNA and non-coding DNA sequence |
| 63 | TraC-466 crRNA and non-coding DNA sequence |
| 64 | TraC-479 crRNA and non-coding DNA sequence |
| 65 | TraC-572 crRNA and non-coding DNA sequence |
| 66 | TraC-703 crRNA and non-coding DNA sequence |
| 67 | TraC-750 crRNA and non-coding DNA sequence |
| 68 | TraC-762 crRNA and non-coding DNA sequence |
| 69 | TraC-763 crRNA and non-coding DNA sequence |
| 70 | TraC-765 crRNA and non-coding DNA sequence |
| 71 | TraC-949 crRNA and non-coding DNA sequence |
| 72 | TraC-963 crRNA and non-coding DNA sequence |
| 73 | TraC-999 crRNA and non-coding DNA sequence |
| 74 | TraC-1105 crRNA and non-coding DNA sequence |

### Example 2: Screening of novel CRISPR systems with DNA binding ability in prokaryotic

### cells using a fluorescent reporter system

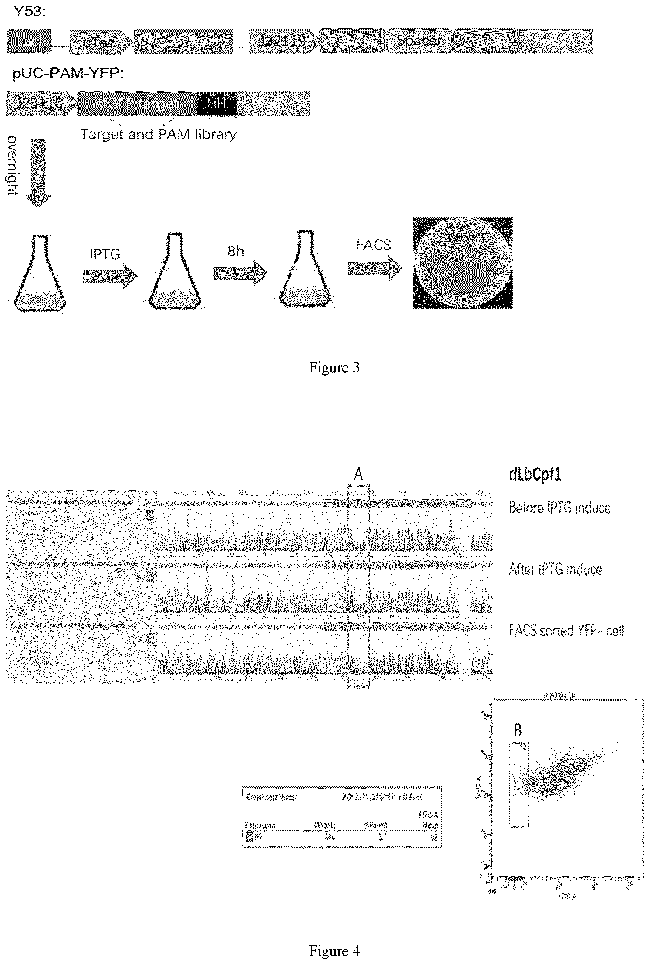

The inventors used a fluorescent reporter system to screen the function of the novel CRISPR systems, which can screen the CRISPR system with DNA double-strand binding ability. The specific experimental design is shown in Figure 3 and Figure 4:
Taking dead LbCas12a without cleavage activity as an example (dCas was dLbCas12a, and the corresponding Y53 vector was called Y53-dLbCas12a), a plasmid with p15a as the backbone was used to express Cas12 protein, miniCRISPR (repeat-spacer-repeat) and non-coding RNA Sequence (ncRNA). Another plasmid with pBR322 was used as the backbone to express yellow fluorescent protein (YFP) (pUC-PAM-YFP), in which there is a target site complementary to the spacer sequence in the 5'untranslated region of the YFP protein and the upstream random PAM library. The sequence was: nnnnnnGTGATCGACAGCAACAAGTGAGCG or nnnnGTGATCGACAGCAACAACAAGTGAGCG, where nnnnnn and nnnn were PAM libraries of different lengths, covering 4096 and 256 PAM sequences respectively, as shown in Figure 3.

If the tested protein can successfully mature crRNA and target the target site in the 5' untranslated region of YFP under the guidance of crRNA, the protein will continue to bind to the 5' untranslated region of YFP and repress the transcription of YFP, resulting in decreased expression of YFP. However, the protein will only work when there is a suitable PAM. Therefore, bacteria with low YFP expression can be sorted by flow cytometry, and then the sorted bacteria can be sequenced to quickly obtain the PAM sequence of the tested protein. Fig. 4 is the screening result of dLbCas12a protein, the bacterium with extremely low expression of YFP in the P2 area (box B) of flow cytometry sorting, the PAM of the YFP negative cell of flow cytometry sorting was found to be TTTN ( The results are the same as the previously reported LbCas12a PAM study), while the PAM of bacteria in flow cytometry fluorescent cell sorting (FACS) before or after IPTG-induced protein expression was in the form of a random library (NNNN) (box A).

The above-mentioned system can be used to screen the DNA double-strand binding characteristics of the candidate proteins of the new CRISPR systems. And the inventors screened some representative candidate proteins. Among them, TraC-N287, TraC-445, TraC-483, and TraC-655 all have T-enriched PAMs by screen, and TraC-N701 has a G-enriched PAM, which implies that most of these proteins have T-enriched or a small amount of G-enriched PAM. The enriched PAMs are consistent with the previously reported finding that most Cas12 family proteins recognize T-enriched PAMs.

### Example 3: Detailed detection of PAM with double-strand cleavage function protein by

### next-generation sequencing

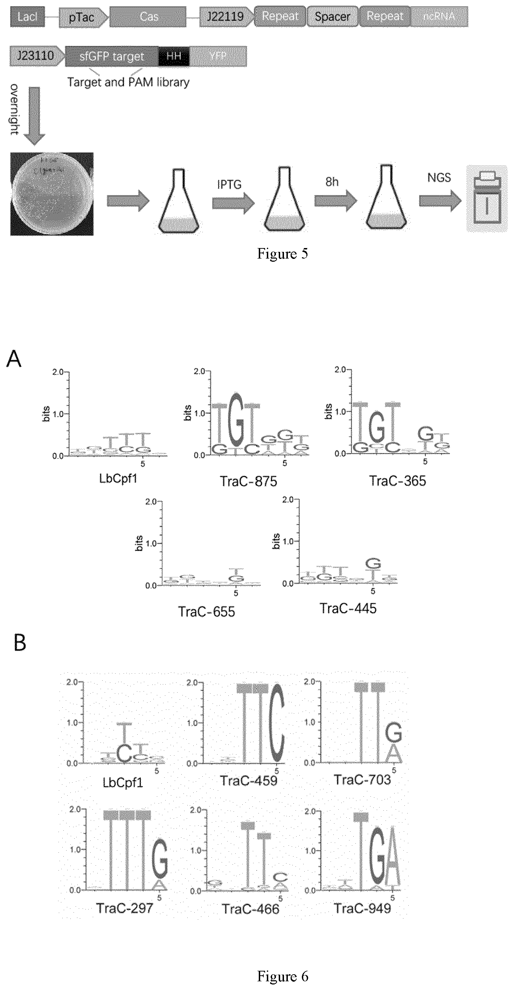

In addition, this system can be used to screen for CRISPR systems capable of cutting DNA double strands. The specific experimental design is as follows:
As shown in Figure 5, the plasmid with the PAM library and the plasmid expressing the protein are co-transformed (this is the treatment group), and the protein expression vector with the deletion of the crRNA expression frame is co-transformed with the plasmid of the PAM library to form a control group. Theoretically the PAM that can be recognized and cleaved by the test protein will be lost, resulting in a decrease in the proportion of targeted PAM compared with the control group, so that the PAM sequence of the test protein can be obtained by comparing the reduction of the two PAM libraries by next-generation sequencing.

The inventors tested the PAM sequences of TraC-875, TraC-365, TraC-655, TraC-445 (Figure 6A), TraC-297, TraC-459, TraC-466, TraC-949 (Figure 6B), and the two group tests were performed with LbCpf1 as a positive control. The results of the LbCpf1 positive control in the two groups of experiments were consistent with the expected results, and the enrichment of TTTN PAM appeared in both groups, indicating that the results of the two groups of experiments can be trusted. In the first set of experiments, TraC-875 and TraC-365 proteins showed enrichment of TGTNNN PAM; TraC-655 and TraC-445 showed enrichment of PolyT or PolyG PAM with weak signal (Fig. 6A). This result is similar to the PAM result detected by flow cytometry in Example 2 above. This type of Cas protein has 5' Poly T or Poly G PAM. The experimental results of the PAM types of TraC-297, TraC-459, TraC-466, and TraC-949 proteins (Figure 6B) found that TraC-297 recognized TTTG type PAMs, TraC-459 proteins recognized TTC type PAMs, TraC-466 proteins recognized TTC-type PAMs, TraC-949 protein recognized TGA-type PAMs. These result further explores the working requirements of this class of proteins in eukaryotic systems.

### Example 4: Screening of novel CRISPR systems with DNA cutting ability in prokaryotic cells using the plasmid interference system

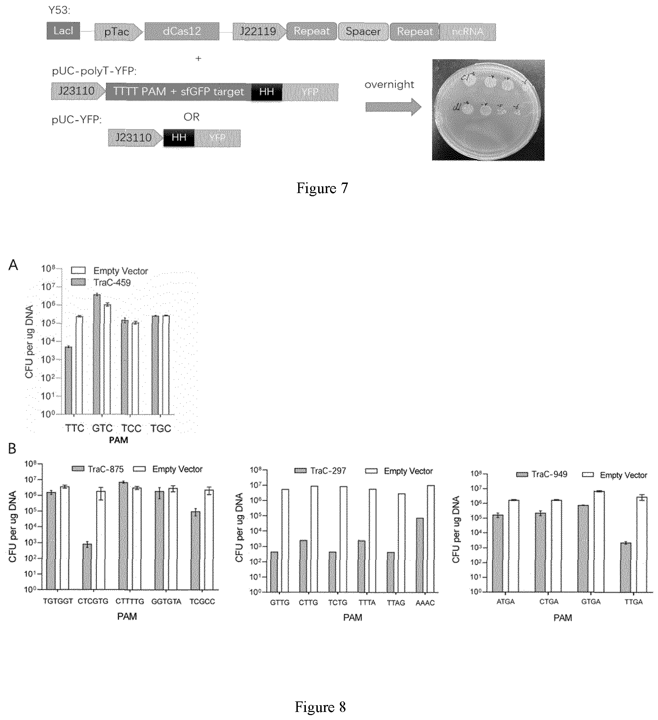

In order to further test the DNA double-strand cutting ability of the new CRISPR systems, the inventors used a plasmid interference system as the detection model, and the specific experimental design is shown in Figure 7. A plasmid interference experiment system was used to verify the specific PAM information of the candidate proteins with obvious PAM obtained in Example 3, and the specific implementation process is as follows: taking the candidate protein TraC-459 as an example (this protein obtained in Example 3 can recognize a typical 5 '-TTC-3' PAM motif, the 3' of the motif is next to the GFP-T1 target (SEQ ID NO: 79)), the pUC-polyT-YFP vector was used as a template to construct a series of target vectors with PAM sequences recognized bt Tra-C459 (pUC-TTC-YFP, pUC-GTC-YFP, pUC-TCC-YFP, pUC-TTG-YFP, pUC-TGC-YFP, pUC-CTTC-YFP, pUC-GTTC-YFP and pUC-TTTC -YFP); the Y53-459 vector and the above-mentioned target vectors were co-transformed into competent E. coli, and the Y53 empty vector was co-transformed with each target vector as a control; after culturing overnight on the double-resistance Lb solid medium, the number of positive clones grown was calculated to test the targeting ability of TraC-459 on different PAMs. From the results, it can be concluded that the candidate protein TraC-459 can recognize the PAM of TTC, while the targeting ability on other PAMs is low (FIG. 8A). This conclusion is the same as the result of the second-generation test in Example 3.

Similarly, the PAM of TraC-875 and TraC-297 proteins were verified, and it was found that TraC-875 protein has a strong cleavage activity for the 5'-CTCGTG-3' PAM motif, and its detailed PAM sequence needs to be further explored; TraC-297 protein can cleave target sequence under the PAM motif of 5'-GTTG-3', 5'-CTTG-3', 5'-TCTG-3', 5'-TTTA-3', 5'-TTAG-3' extensively and efficiently; TraC-949 protein can cut the target sequence under the 5'-NTGA-3'PAM motif, and has the highest cutting efficiency of the target sequence under the 5'-TTGA-3'PAM motif, while the cleavage efficiency for 5'-TTGA-3', 5'-ATGA-3', 5'-GTGA-3', 5'-CTGA-3'PAM targets is relatively low. The results are shown in Figure 8B.

### Example 5: Evolutionary Models of Newly Predicted CRISPR Systems

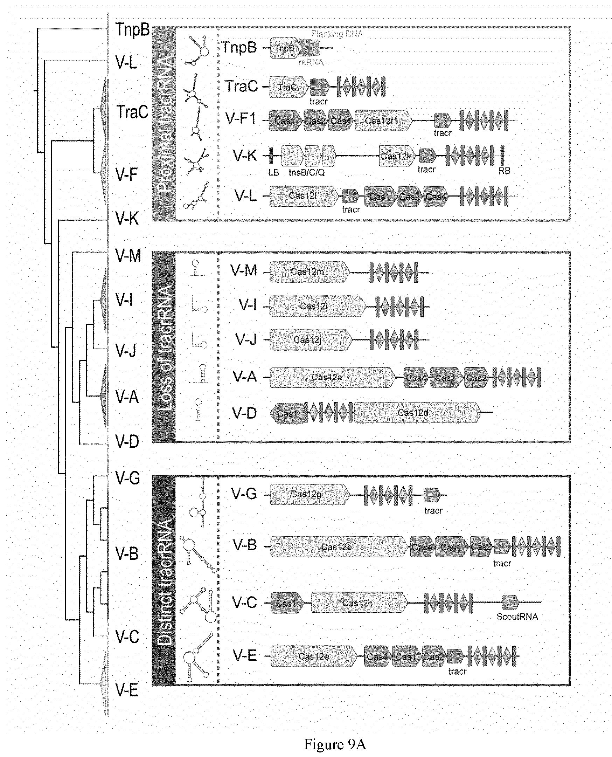

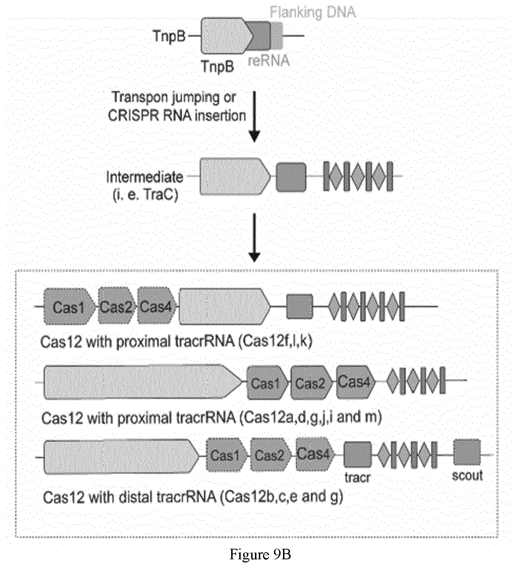

To further analyze the structural and functional characteristics of the TraC proteins of the newly obtained CRISPR systems, the inventors predicted the appendix RNA secondary structures of the V-type CRISPR and TnpB systems and analyzed their structural folding models (Fig. 9A). The study found that different protein subtypes can be divided into three types according to the folding model, and the folding model reflects the characteristics of the three types of CRISPR loci, which are the distance between the CRISPR protein and tracrRNA or the absence of tracrRNA. The classification results indicated that the TnpB protein may have undergone transposon jumping to the CRISPR site, or reRNA splitted into tracrRNA and CRISPR RNA. The diversity of appendix RNA combinations also supports a model of co-evolution of effectors and appendix RNAs (see Figure 9B for evolutionary models).

### Example 6: Editing activity of TraC protein using sgRNA as guide RNA

In this example, the TraC-459 protein in the TraC systems was selected to verify its DNA editing activity.

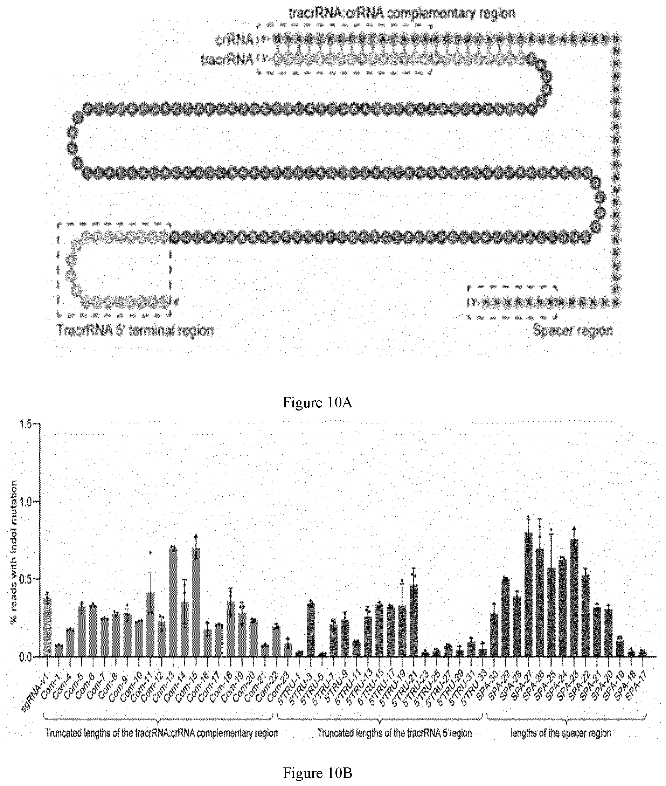

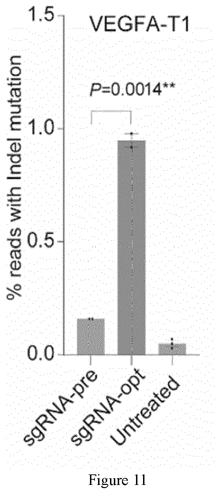

On the one hand, the structure and length design of the sgRNA will affect the editing efficiency of the CRISPR system, so the inventors screened the most suitable sgRNA structure for the TraC systems. First, the inventors designed a predicted sgRNA (sgRNA-predicted, sgRNA-pre for short) by recombining tracrRNA and crRNA targeting the VEGFA-T1 site of HEK293T cells (see Figure 10A). Subsequently, based on sgRNA-pre, the effects of tracrRNA:crRNA complementary region truncated length, tracrRNA 5' region truncated length, and spacer length on TraC-459 protein editing efficiency were tested respectively (see Figure 10B). The results showed that when tracrRNA:crRNA complementary region was truncated to a length of 11-15 bp, or the tracrRNA 5' region was truncated to a length of 19-21 bp, or the spacer was 22-27 bp in length, a better editing effect was obtained. Based on this, an optimized sgRNA (sgRNA-optimal, sgRNA-opt for short) was obtained. This optimization strategy is called the second generation sgRNA optimization method of the TraC system (sgRNA-v2 for short). Figure 11 shows that sgRNA-opt, as a guide RNA, can significantly improve the editing efficiency of TraC-459.

### Example 7: Editing activity of TraC protein using reRNA as guide RNA

The co-evolutionary model in Example 5 predicted that the TraC protein is an evolutionary descendant of TnpB. Since the TnpB system uses the 3' flanking sequence as a guide RNA for DNA cleavage (Karvelis, T. et al. Transposon-associated TnpB is a programmable RNA-guided DNA endonuclease. Nature 599, 692-696 (2021). ). This example examined whether the guide RNA of TnpB can be used in the CRISPR system of TraC protein.

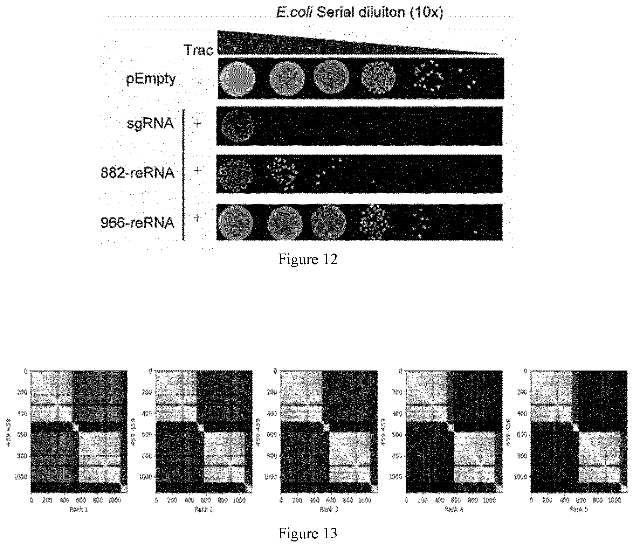

In the in vivo experimental verification, based on the three-dimensional structure clustering analysis, the inventors selected reRNAs (882-TnpB-reRNA, 966-TnpB-reRNA) of TnpB mutant proteins with similar structures as the guide RNAs to be verified. For the GFP-T1 target, the inventors fused the scaffold sequences of 882-TnpB-reRNA and 966-TnpB-reRNA with the targeting sequence of GFP-T1. Subsequently, the plasmid interference experiment in Example 4 was used to analyze the dsDNA cutting ability of TraC-459 in Escherichia coli under different guide RNAs, and the experimental results are shown in FIG. 12. The experimental results showed that TraC-459 showed different degrees of DNA interference activity relative to the blank vector control (shown as pEmpty in Figure 12) under different types of guide RNA.

The results of Examples 6 and 7 showed that TraC4-59 has a dual-guided mechanism and both targeted cleavage pathways of the TnpB system and the CRISPR system, that is, the TraC effector protein can target and bind to the target DNA under the guidance of reRNA, and can also target and bind to target DNA under the guidance of sgRNA.

### Example 8: Prediction of protein working form of TraC protein

In order to further explain the working form of the TraC system proteins, the inventors constructed a dimer sequence of the TraC-459 protein, and used the multimer v3 model of AlphaFold2 to predict the three-dimensional structure of the TraC-459 protein. In the best 5 protein structures of TraC-459 (Rank 1-5), there was no dimer interaction (Figure 13). The upper figure is a predicted alignment error (PAE) heatmap (provided a distance error for each pair of residues. It gave an estimate of the position error at residue x by AlphaFold2 when the predicted and true structures were aligned at residue y. Values ranged from 0-35 Å (white-black). Typically it is displayed as a heatmap image with residue numbers running along the vertical and horizontal axes, with the color of each pixel representing the PAE value for the corresponding residue pair. If relative positions of two domains are reliably predicted, the PAE value of residue pairs with one residue in each domain will be very low (less than 5A, white in the figure is 0). The horizontal and vertical coordinates in the figure are two TraC-459 monomer protein length. The first 575 amino acids is a TraC-459 monomer protein, and the last 575 is another TraC-459 amino acid. The heat map only shows as white in the previous TraC-459 monomer and the latter TraC-459 monomer. The region between two TraC-459 monomers is black. Combined with the compact protein structure of 575aa, this implies that TraC-459 is the smallest Cas12 monomer.

### Example 9: Optimization of TraC protein

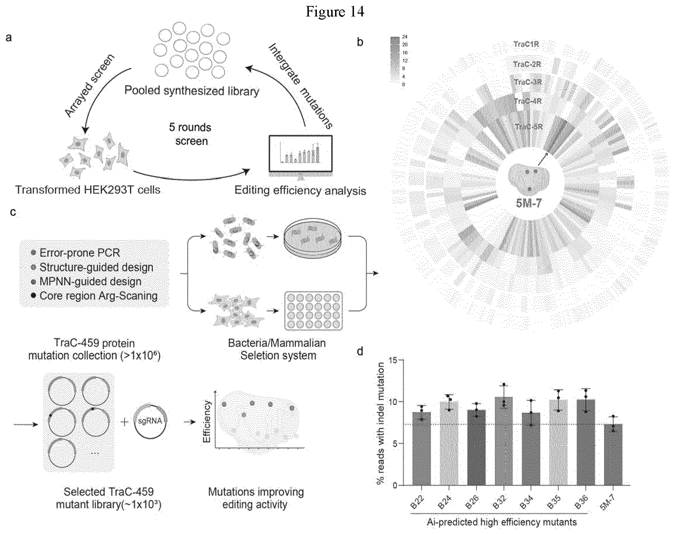

In order to further verify the effectiveness of the TraC protein and expand its application scenarios, this example obtained a series of optimized TraC-459 variants through arginine scanning mutation, directed evolution and artificial intelligence-assisted evolution. The screening process is shown in Figure 14a-c. After the intracellular editing efficiency test of the TraC-459 mutant library, some of the TraC-459 mutants screened out had higher editing efficiency. Taking five-mutation mutants as an example, the editing efficiency of the five-mutation mutants in the mutant library was tested experimentally, and a total of three sets of parallel experiments were carried out (Table 2). Among them, the ratio of the editing efficiency of the obtained mutant to the wild-type TraC-459>1 indicates that the mutant has higher editing efficiency. The results of mutants with improved editing efficiency screened according to this method are shown in Table 3. Representative 5-arginine mutant obtained through arginine scanning mutation screening is TraC-5M-7 (S137R, P148R, D150R, K315R and A369R), namely the TraC-5M-7 mutant in Figure 14b. The study shown that the editing efficiency of TraC-5M-7 at the VEGFA-T1 site was 24.02 times higher than that of the original TraC-459. The TraC mutants with improved editing efficiency designed according to this method are shown in Table 3.

In order to further design TraC-459 with increased editing activity, the inventors developed a deep learning model through the data of a series of TraC variants, and obtained 7 representative mutants TraC-B22, -B24, -B26, - B32, -B34, -B35, and B36 which had enhanced editing activity in human cells (editing activity is shown in Figure 14d, and the mutation sites are shown in Table 4).

**Table 2 The results of scanning mutagenesis experiment of five amino acid mutant arginine**

| **Mutant** | **mutation sites** | **editing efficiency** | | | **Mutant to Wt Efficiency ratio** |
|---|---|---|---|---|---|
| | | **Expeime nt Group 1** | **Expeime nt Group 2** | **Expeime nt Group 3** | |
| Wt TraC-459 | - | 0.44 | 0.4 | 0.63 | - |
| TraC-5M-1 | K78R/S137R/P148R/S408R/V500R | 1.07 | 1. 01 | 1. 11 | 2. 17 |
| TraC-5M-2 | S137R/P148R/D150R/K315R/H392 R | 0.91 | 0.84 | 0.93 | 1.82 |
| TraC-5M-3 | S137R/P148R/D150R/K315R/K78R | 4.67 | 4. 58 | 5.41 | 9.97 |
| TraC-5M-4 | S137R/P148R/D150R/K315R/L332 R | 7.65 | 7. 33 | 8. 29 | 15. 83 |
| TraC-5M-5 | S137R/P148R/D150R/K315R/S408 R | 6.74 | 4. 51 | 8. 1 | 13. 16 |
| TraC-5M-6 | S137R/P148R/D150R/K315R/K386 R | 6. 2 | 4. 26 | 6. 98 | 11. 86 |
| TraC-5M-7 | S137R/P148R/D150R/K315R/A369 R | 10.71 | 11. 46 | 13. 14 | 24.02 |
| TraC-5M-8 | S137R/P148R/D150R/K315R/V500 R | 7.84 | 6.4 | 9.94 | 16. 45 |
| TraC-5M-9 | S137R/K386R/S408R/V500R/K78R | 0.93 | 0.69 | 0.74 | 1.61 |
| TraC-5M-10 | S137R/K386R/S408R/V500R/P148 R | 3. 82 | 2. 28 | 4.24 | 7.03 |
| TraC-5M-11 | S137R/K386R/S408R/V500R/D150 R | 4.09 | 3 | 4. 7 | 8.02 |
| TraC-5M-12 | S137R/K386R/S408R/V500R/K315 R | 0. 39 | 0.92 | 0. 33 | 1. 12 |
| TraC-5M-13 | S137R/K386R/S408R/V500R/A369 R | 0. 76 | 1.63 | 0.91 | 2. 24 |
| TraC-5M-14 | P148R/K386R/H392R/V500R/K78R | 1. 31 | 0.69 | 1.4 | 2. 31 |
| TraC-5M-15 | P148R/K386R/H392R/V500R/S137 R | 1. 84 | 2. 3 | 1.86 | 4. 08 |
| TraC-5M-16 | P148R/K386R/H392R/V500R/D150 R | 3.01 | 3. 45 | 3. 55 | 6. 81 |
| TraC-5M-17 | P148R/K386R/H392R/V500R/K315 R | 1. 39 | 0.93 | 1. 22 | 2. 41 |
| TraC-5M-18 | P148R/K386R/H392R/V500R/L332 R | 1. 51 | 1. 52 | 1. 57 | 3. 13 |
| TraC-5M-19 | P148R/K386R/H392R/V500R/A369 R | 2. 77 | 2.92 | 2.67 | 5.69 |
| TraC-5M-20 | P148R/K386R/H392R/V500R/S408 R | 0.47 | 1. 05 | 0. 51 | 1. 38 |
| TraC-5M-21 | K78R/S137R/P148R/K315R/A369R | 3.05 | 2. 37 | 3. 02 | 2. 10 |
| TraC-5M-22 | S137R/P148R/K315R/A369R/H392 R | 1. 99 | 3. 52 | 1. 9 | 1.84 |
| TraC-5M-23 | S137R/P148R/K315R/A369R/S408 R | 0.94 | 2. 96 | 0.93 | 1. 20 |
| TraC-5M-24 | S137R/P148R/K315R/A369R/V500 R | 3.01 | 3. 78 | 2.99 | 2.43 |
| TraC-5M-25 | S137R/P148R/K315R/L332R/A369 R | 3.61 | 4.8 | 3.6 | 2.99 |
| TraC-5M-26 | D150R/A369R/K386R/H392R/V500 R | 0.93 | 2. 3 | 1. 03 | 1.06 |
| TraC-5M-27 | K78R/D150R/A369R/K386R/V500R | 3. 73 | 3. 54 | 3. 8 | 2. 75 |
| TraC-5M-28 | K78R/S137R/P148R/A369R/V500R | 1. 25 | 0.6 | 1. 36 | 0.80 |
| TraC-5M-29 | K78R/D150R/A369R/S408R/V500R | 2 | 2. 75 | 2. 24 | 1. 74 |
| TraC-5M-30 | K78R/D150R/L332R/A369R/V500R | 5. 34 | 6.69 | 5. 14 | 4. 27 |
| TraC-5M-31 | K78R/D150R/K315R/A369R/V500R | 3.41 | 5. 76 | 3.68 | 3. 20 |
| TraC-5M-32 | K78R/P148R/D150R/A369R/V500R | 14. 15 | 7. 73 | 13. 85 | 8. 89 |
| TraC-5M-33 | K78R/S137R/D150R/A369R/V500R | 3.13 | 3.66 | 2.97 | 2.43 |
| TraC-5M-34 | K78R/S137R/D150R/K315R/A369R | 3. 46 | 1.9 | 3. 52 | 2. 21 |
| TraC-5M-35 | S137R/D150R/K315R/A369R/H392 R | 4. 32 | 3. 39 | 3. 85 | 2. 88 |
| TraC-5M-36 | S137R/D150R/K315R/A369R/K386 R | 3. 21 | 4. 09 | 3. 35 | 2. 65 |
| TraC-5M-37 | S137R/D150R/K315R/A369R/S408 R | 0. 73 | 0.4 | 0. 71 | 0.46 |
| TraC-5M-38 | S137R/D150R/K315R/A369R/V500 R | 2. 89 | 2.91 | 2.67 | 2. 11 |
| TraC-5M-39 | S137R/D150R/K315R/L332R/A369 R | 2. 27 | 6. 04 | 2.65 | 2. 73 |

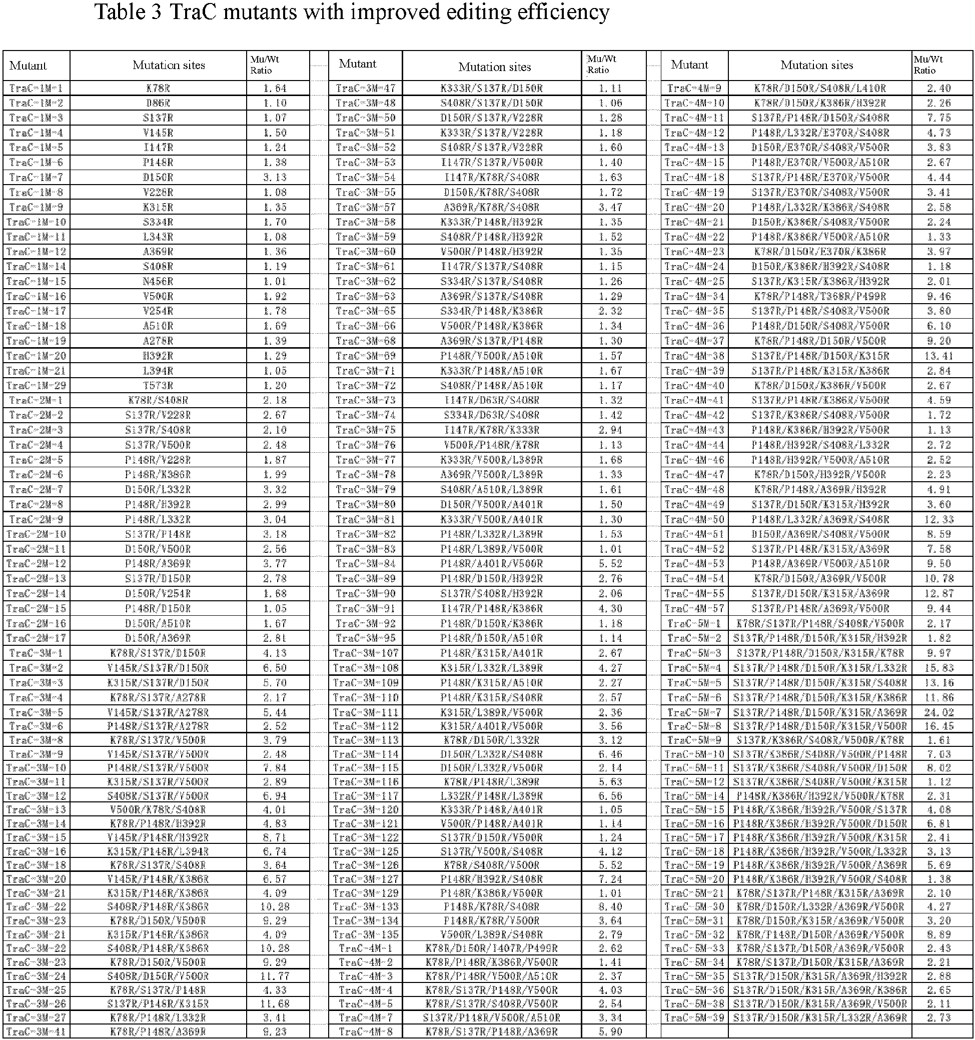

**Table 4 Mutation sites and amino acid sequences of representative TraC-459 mutants**

| **Mutant** | **mutation sites** | **Sequence** |
|---|---|---|
| TraC-5M-7 | S137R, P148R, D150R, K315R, A369R | SEQ ID NO: 80 |
| TraC-B22 | S137R, P148R, D150R, M284I, K315R, A369R, A423S | SEQ ID NO: 81 |
| TraC-B24 | S137R, P148R, D150R, K315R, A369R, I417F, A510R | SEQ ID NO: 82 |
| TraC-B26 | S137R, P148R, D150R, P245G, K315R, A369R, T426R | SEQ ID NO: 83 |
| TraC-B32 | S137R, P148R, D150R, K315R, A369R, I417F, I471V | SEQ ID NO: 84 |
| TraC-B34 | S137R, P148R, D150R, K189Q, K315R, A369R, A489G | SEQ ID NO: 85 |
| TraC-B35 | S137R, P148R, D150R, K315R, A369R, A423S, A489G | SEQ ID NO: 86 |
| TraC-B36 | S137R, P148R, D150R, K315R, A369R, P496G, A510R | SEQ ID NO: 87 |

### Example 10: Dual pairing function of the TraC system

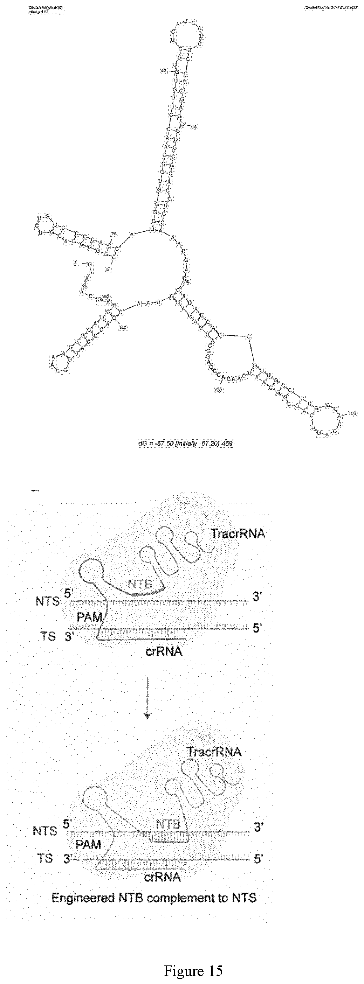

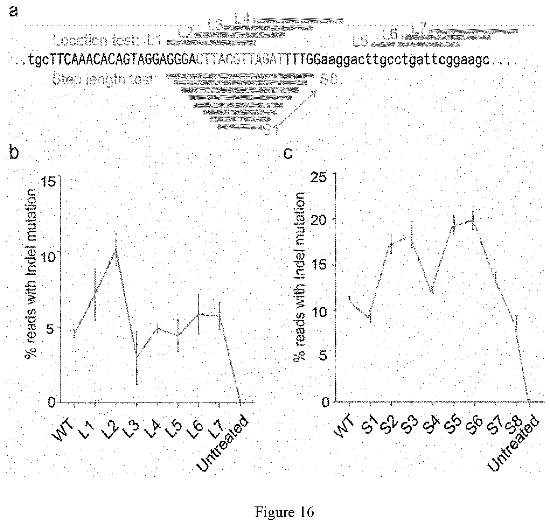

Subsequently, through the secondary structure prediction of sgRNA-opt, we found a bubble-like region at the end of tracrRNA (Figure 15). Combined with evolutionary analysis, this prominent region may be evolved from the flanking DNA during the reRNA evolution of TnpB. So this region may be a region that can be reprogrammed to target DNA. Combined with the reported structural information of TnpB, TnpB protein cannot autonomously open the terminal region of the target site distal to the PAM, resulting in low editing efficiency for regions with high GC content. Therefore, modification of this region may bind to the region distal to the PAM to help open the DNA duplex, thereby improving editing efficiency at high GC-content targets (Fig. 16a).

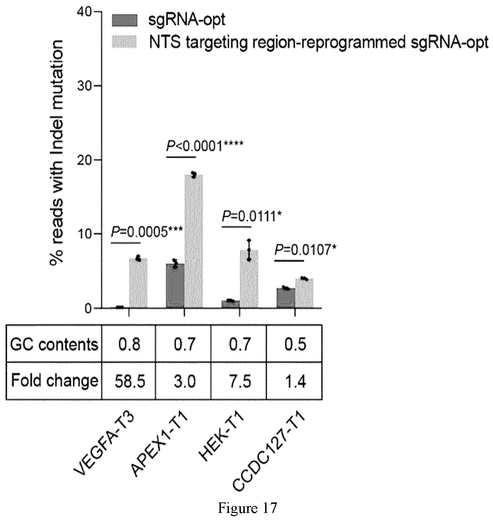

We reprogrammed the bubble region for the VEGFA-T1 target in HEK293 human cells and tested the most suitable paired region and paired length. The complementary region from 13 bp to 47 bp downstream of the PAM sequence in VEGFA-T1 (L1~L7 in Figure 16a) was evaluated and it was found that the 21-32 nt complementary region downstream of the PAM sequence (L2 construct) had the highest editing activity (Fig. 16b). Second, we tested the editing activity of the complementary length from 20 bp to 6 bp (S1 to S8) in the complementary region of the L2 construct, and found that the 10 bp complementary region length (S6) had the highest editing activity (Fig. 16c). Finally, we targeted the bubble structure region to the PAM distal region and found that the reprogramming form of sgRNA could improve editing efficiency on endogenous targets with high GC content in the PAM distal region of four human cell lines (Fig. 17 ). Such an additional reprogrammable region has not been found in other CRISPR systems.

Taken together, this example demonstrates that TraC-459 is a highly compact monomeric Cas12-like protein. By comparing with known proteins in the prior art, the inventors found that it is the smallest monomeric CRISPR effector protein currently known. And it has a unique sgRNA and reRNA dual-guided mechanism, and a double pairing function, which do not exist in other Cas12 subtypes.

### Example 11: TraC protein is affected by temperature in plant cells

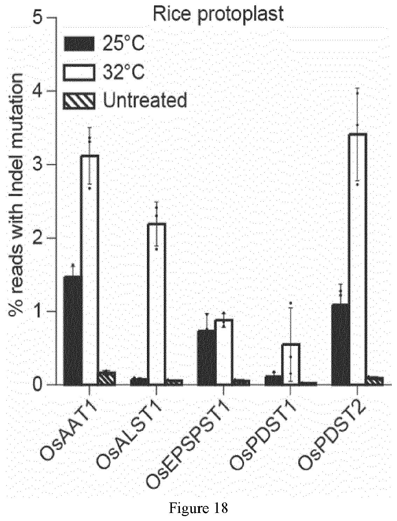

Among plant cells, some plant cells can tolerate high temperature culture. In order to verify the effect of temperature conditions on TraC protein in plant cells, the inventors selected five endogenous targets (OsAAT1, OsALST1, OsEPSPST1, OsPDST1, OsPDST2) to test. In vitro test showed that the editing efficiency of TraC-5M-7 at 32°C was 1-29 times higher than that at 25°C, and the highest efficiency reached 3.41% (Figure 18).

### Sequences

>SEQ ID NO:1 
MITVRKLKLSIMADEELRIQQLKWIKDEQYNQYRALNNGMAFLIADHMLNTAESTKIIYKNNEINKKKKKI
YYMEDKIKKENNKLEEEKILKFESDINKLKHEIKILENEKVELELETKNLSEQFKNHYVEDMYTRLDEIPF
QYKDNKSLVQNRLKKDFDFYLNNGGKRGERKPTAYKRDYPLLIRGRLLNFYYNKDNVFIKWIAGITFKVEL
GNKIKNNIELRHTLHQCMNNEKYKVCDSSLQFDNKNNIILNLTIDIPINTSENNFIEGRVMGVDLGMKIPA
YASFNDVEYCRAFGDIEDFLRVRTQLQSRMRKLQMALTLIKGGHGRGKKLQALNRLKDKEKDFVNTYNHMI
SKRIIEYSIKNCCGVINLEYLSLAAREKDLFLTLQPQKSNRIKRNWSYYDLQTKIENKAKKYGIIVKKIDP
YLTSQTCHICGNYDEGQRISQEQFECKACNRKFNADYNASKNIALSTKYINNNINESEFFKRYKNN
>SEQ ID NO:2 
MTDVPVSRIRNFSIVAHIDHGKSTLADRLLQDTGTVAARDMKEQFLDNMELERERGITIKLQAARMDYRAK
DGNDYVINLIDTPGHVDFSYEVSRSLAACEGALLVVDASQGVEAQTLANVYLALENNLEIVPVLNKIDLPG
AEPDRVAGEIEDTIGLDCSEAIHASAKEGIGIREILEAIVQKVPPPADTVDKPLRALIFDSYYDSYRGVIV
YFRVMDGRVKKGDKVRLMASGKEYEIDELGVLAPNQCPIDELHAGEVGYLAASIKAVGDARVGDTITLVSK
PATEPWPGYTEAKPMVFCGLFPTDSDQFGELREALDRLRLNDAALSYEPETSSAMGFGFRCGFLGLLHMEI
VQERLEREYNLDLIATAPSVVYRVTKIDGEVVMIDNPSELPEPQYRETIEEPYVKVEMITPESFVGTLMEL
SQTRRGVFVDMRYLTQGRTTLIYEIPLAEVVTDFFDQMKSRSRGYASMEYSLIGYRENPLVRLDVLINGDR
VDSLSAIVHRDKAYYVGRALVEKLKELIPRHQFKVPIQATIGSRVVASEAIPALRKDVLAKCYGGDISRKK
KLLKKQAEGKKRMKAIGTVDVPQSAFMAVLKIDRE
>SEQ ID NO:3 
MSALSLSRPLAARLARRNIHYAWVVAAVTFLTMLVTAGAVGIPGVLIAPLEAEFGWTNGQISAAFAIRLVL
FGLMGPFAAALMNRFGLRPVILASQLLIATGIFGAMAMTQLWQLTLLLGVVVGIGTGLTAMVLGATVVSRW
FAARRGLVIGLLTASTATGQLVFLPVLAALTEAVGWRSALALSLVTLMLTAALVMALMRNRPSDLGLPPFG
SATPVPEPPRQSLGAMLISPLTTLRDAVRVPMFWLLFGTFFVCGASTNGLVQTHFIALCGDYGMAAVTAAG
MLAVIGVFDFFGTVGSGWLSDRYDSRWLLFWYYSLRGLSLVWLPFSDFSFYALSIFAVFYGLDWVATVPPT
VKLTAEHFGERSNIVFGWVFTGHQLGAASAAWSAGATRTAYETYLPAFLTAGLLCLAAALVIALFIGSSGL
RRRATPGGARAAA
>SEQ ID NO:4 
MLKAYRYRIYPNKEQEEFFAKTFGACRFVWNKMLEGKLNALNNKEKLPKITPAKYKKQYQFLREVDSLALA
NVQLNQEKAFRDYFKNKKHFGLPKFKKKKDKQSYTTNNQGNTIRIDFEKQLLYLPKVKTGIKIKLHRIFEG
KIKSATITKTKSGNYYVSILVETDKVQNKIKQPKSKICGIDLGLKDFAIITNDNGSCKIENPKYLVRAEKR
LKRLQRQLSRKQKGSNNYKKTREKIAKLHEYISNARNDFLHKTSKAIIDENQVIVVEGLSVKALQQSMLSK
LVSDVSWGTFLRYLEYKANWYGRELIIVDRFYPSSKTCSVCGYINTQLRLSNRYWECPECNTFHDRDINAS
KNLYKVGLSRVGTTRTQACGECSDGGTMIYHRSTSQHSMKQEATTSVSGSSSLSSL
>SEQ ID NO:5 
LSEQLDDTPEQPNEVEETKKRKQRNKGKHPARIWSVFSRYLVSGREHFDKQVLLAHRFRNKLVELELQRRA
AANVVIAQASSELQPLIDALAAAEQVLEVSLQELKAVRAKHRRRAESAAQRDAVTNARTARNQASKALSKA
RKDAFASEAAQVGLWLAEEHHFQAVLAARHAFINDGLYWPTATDVQDRARAMRKGAPPVFRRFGGAEQAGR
IAVQIQKSTDKSQSEGGITFEEAFSCSHGFFRLEKKPGRDPLPEIADQPDYKSKRQQLLTYARAWLRVGSE
 
GKGARAKPCWVVADVLLTRQAPKTARIVQVYLDHSVIGDRERWRLSLVLTNQEGWPKPNRASGCMVGIDLG
WRLLDTGELRVAYACGADGQHHELRLPASLVKVWRRPDRIQQERDNLFNDVKARLLEWLKGREDLPDWLKE
QAEHLHLWKSSTRLSRLVDHWAGRDINWSSQRRIAGDEEILASLRGWVKRNLHLRDYQYHEREQLAAHRLD
VYRKWADGLARLYQTAVLEDADWRDLARLPSPEDDAVNETARYNQRMASPGLLASVITNMFAITSRVECAN
TTRECWRCGHTEAFDAEAQLIRVCPGCGDACDQDESAARVLLARGQALNQSQVAEAAPSS
>SEQ ID NO:6 
MDDAPRRTIPIKLDVSEERRGDLHQTKSQFLHCANRTSEWAWRYDDYCVTSKSKAENSLYDELREETDLTA
NLVQKGIRRAIEAVDAGVEKLQQGEQTSQPEFDSWSVVYDKRSASFNDDHATLSTPNGRVTTEYVLPPEDE
REDTPFGRYYESDDWDASSATLQYDEQDDTFYLHVTLKNPDYTVDRTERQEASHDDDGAENGVVLGVDLNV
TGAFAVTSTGKFIGSADYLTHKRDQYEKRRARLQQTGTRSAHLTIQSIGSRFSDWSLDWLHNRANDLIAEA
QATDVDGIIFENLDHIRENIANESKFQQWTYAKFVELVEYKVESTALFVDTVNPAYTSQRCSHCGFTHEDN
RDDKEFACQDCGYEVNADYNAAKNIAKRYCGYIHRGQKSRGGWATSQLALESGTLNVNGEYTPSALRG
>SEQ ID NO:7 
MPIRLSGADYRRAHEACHNAASLWNFFLASIQEYWKENKTDPTEKEMRHFLYEQRPDLRDGLHAHTVQLIL
SDLMDAISTYRSNRDSGDNNARAPHREKNYRPLEFSAGFGWRVTPDRQHIALSFGKGHKRITLPLPEIADP
KTGEILPPERWGSMQLCWNHNARKWSLHVSVPTPKPPQGNPNIIAAIDEGIINPMTVAIETENSYDVLVIN
GRHARAIKHYRNTRIATLQEKLSRCVKGSKRWHKLNAKRRKRIEAMTDSALHNANHQTTRKVADFIQAHDAG
RIVAGDVRDIEKNTRKDEAKRIKNTKNQRRRLSQWSRGKQGTLLEHKTGSTVGHINEAWSSKTCPACQTRN
HPNGRGYHCHNCGFTCNRDAVGAINILMRAQHGAYTPIDMDKPIRVKYLRATPIFQPKTVPSME
>SEQ ID NO:8 
MLGFTIPPKRKFQPRLMICKVSEFKANFTSDQIAIIREYQEGLRRLWNFALGQLINADENWHYDKVSKTYV
SKCTLVRHQVKDDLSKTWVAHSPVLDRRSRWIVAACQAYPTATTKAGHQRMADKDHVFTRAEVKASGWVGG
YGYSCPIQRDHNPGLISNSAMKDSKRGLGILARARDLQDPTIITPDLLKDLDRQLILNVQQKYRMGLLEQL
GGAWDKYVKDRYSKESLRRGKPKFKGTNTPVNTIRHPNPNAGGKKPQSADAVRAIGSDVLKIPGFGDVHLP
GLDKRWGKSRVKIMQIVEKASGFFVQLTREETPRRVRKVEPRGYAGIDWGWKEENYCSVSWVNEGGETRSQ
QISKPRFYRESQEQLGKAQQKLDEKLYRRLILWIHHPDTNLLDYVSEGDADILKTCRTVEDLCAHIGVDLN
ASTIQRLRWKQCGDSQGVKECKQKIKRIHEKTKRRRRSHQYRIASYLTTYSSGIAVEDGLQKKVGKAKAKA
KEDGSGFDKNQASAVTGQNKSNRDAAIGQQIDLIDKKTQEFGKSTQRIKFAADLPLSRICCKCLAYDPEMD
ISKPVYHCTQCGYEGDRDVNSSALIAKIGREPSLQKTIKQKIKKTRKKS
>SEQ ID NO:9 
MFAIKSMSELVQHHITIQLKAYLSTTQTALFENWTDSLRPLYNLALGLLYEEQQRRWRTNQKFLKNYLDKS
SLQTYLNEIENKPDIYPVEWHITKALPECDWLTKEENEVRKKDNTKSLACRTINRDGNFFTPIRPYWHLEE
PQKLAKFKCFTNQWLISCNLLTNYHLQKLLNVNMKVRQSFISMNLMEAWKRYQKGDFRKLKFKSKRNPVIS
LCNKQTNRIKFDPEANNCQLLGKEFGLIEFRGLHNRHQGQIQPRNGSLTKKADGYYLNLVFQVEHKPIPDS
DLQVGIDPGLVTLLTLSDGKCISNQRFLKENERHLTVLQKKLSRQTPGSKNWEKTKKALAKIHKQTADHRK
YYNHKVSTHLVNKYGAIAIEDTKLTNMNKRPKAEKREDGKGYEHNGAKAKAGLNQSFHDAGLGQLRAFLES
KANSYENRHIERVRANYTSQKCSRCGHTDSENRLTQASFHCLKCGLEMPADLNAAINIEQTAFGLDKS
>SEQ ID NO: 10 
MKRVTITIDGEQTKGIVIGTIAANHTAAEWLLTASVSAKSAKVRFDPEEAVAETSSLVMIAPTRTEKYLYL
VPDEQVQPVTTIVRKYGLLSPLDWDCPDYPAGDAFEHLFLQNKLWNDLVTIEREHRAKYRELIGSDEETAQ
MDTEIASIKDRLSVLDEGRKKLRVEHRKKKCPEIDCLDENIKKLKSELKAVASKAKETRAAAKDRIRAAGN
DIENLEKDRQAAVIKAYNNSGLWWGNYNAVLESYKKARIKALKDGAELKYHRFDGSGRFTNQIQGGMSVQD
LLEGNRNVASLRLVSSGELGDISGKKPPSLDLQSVGSRRDSREYGILAITLYTGTDEQSKKFRRTLSFPVI
LHRPLPEGATLKSLSVHRKRVGTDFVWSVVFTFTTDCPTYDQRSSTGNRCGLNLGWKKQAGGGLRVATIYD
GSDARHITLPQAIIDGLDYVNGDLQGRIDSAANENHAWLLEQWGGDELPESLQELRSMLRRSKRPHPAKFA
KAVIAWRNYPEYLGDARDEAEQRRKATKRLTIEMAHKREKLLRRRMDFYRNTAKQLTSVYDVICLDKMDLR
RLALLEKGDGTPNELTKIARKQRQQAISELRECLSKAAAKNGTQIEQVSTASSATCSACKGKMEQVDGIM
WRCRECRALVDQDINAAANLFREVL
>SEQ ID NO: 11 
MTSSIEPTSDASKPATTTRVFKYGLLLPRPEDRSAVWEQLRAAHRYYNKLIEIECHRRQAYRELRSELSRQ
VAVLEGRLAELNAQIDAALAGLQGERKAKRSAKTSGPEKEALKQLKAERKKLSAALKLERAAALNNPVLKT
AIEKLDERSHKELLEARANCGVYWGTYLLVEAAVQQARKSKIDPKFKRWNGTGRLGVQVQKGISVSGFFFK
AKEQKGGRQLVQVAELDPEAWSSPIRGVRRRLSRTTLRMRINSDAKGKPVWAEWPMVMHRPLPDGAQIMGV
TISAHKKADRTFFTVDVACRLPAVVVAPGPRMVGIDLGWRKIGDEIRVASWVDSDGQKGELRLPASIQARI
 
RKARDIRAIRDETRDKMLPVLATLLKPLKTSDEFKERIENLIMWRSFDRLHKLFWFWKNARFAGDEVPWYV
FETWHHRDRHLHQYEAGCRSGALGHRREIYRIFAAHVAKHYGKVVLERWDLRKMAERKPPEEDTADSREPN
RQRFDAGVSELRLAIVNAVEREGGLIWRTPAALTTMRCHACGHVHDESIVALQHQCVGCSLVWDQDENAGW
NLLASGQAMDNDPDLLAAANPPKKPRKPRFTKKKKASPTSSTMEHDEDLSPTGS
>SEQ ID NO: 12 
MIRTYKYSLKAPENFAEDCEDELRRMNDLWNRLIEIDRQRERSFKDLCRSTSAEYAAAQDEIEALREPIDN
LYDAIRAERIATRSKEPSDELRARRDELLGRRKALWEICKAIQKAIPKESQAPINEVYKTNVKLARQQSGC
FWGNYNAVIESFETAKSKAIKDGGRLHFKSFDGSGRFVNQIQGGMTVTELLAGSHSQAQLTNLVTTNKTKG
RFAFTAFTGKDDAGKRFRRQLFSEINYHRPIPADGVIKAVEVVKVPHDGKQKYKWHACFTVALPEVDIKHP
KRNIAGVNLGWRQFGGRLRVAVVVDDAGKKTEYFVPAELVSKFEAAETIQKAADDARNEMLSWLRTFYQDN
RDEAPQEWRESIQGLLRNRPSVDAANHLMTIWRECVFAQEESRRYAAWLKSDAALRRSYTGCRQNAVKWRE
EIYRHIAKELAERYAVLAVTDTPLSTMSRTKAKDDLAVDNALPESARRNRVIAAIYSLKEWIGKQAAKTGS
TVETITGKMTATCHKCGYVAEKRLRGSQYATCKSCGSELELDENAAINCRNHASGAVLISDKPEKTGRFQR
AKMAENDFARKIGDNASPLVT
>SEQ ID NO: 13 
MKTKTIEFKIYPTLAQSQTIDKWLQDIKWVWNKGLSLKFADRQKHYRTQIGDRNIPDGLPLRWKWRKVEAT
DKKGKITEKWEKIRLVGGGVVRPKSGYPYCPIREHRNIEDPGKFKYFRNDNSPQFVIDIPSEFKEGMADTL
KKAWQAYSDPKRPTQKPKFKGKQDKVRSLTSLHAGGKSKLLKPERIPGSDNGFVQFPKLGKLKVKGLYSRH
DWVEWGSAKIVKEPSGYYLHVCVDVPNDPLPKSDKSVGIDPGLLSVITTDQGREVAPPKLFRKQQTKLRRL
QRKASRQQKGGSNQKKTYQKVALQHEKIRRSRNAFNHKLSTKVVREYSGIVMEDLKIQNLSRKPKAKKRED
GNGYEQNGAKRKAGLNKSFADSALGDLISKIETKCKDTDREFVKVAAHHTTVDCSNCGAKIKKALSQRTHR
CTECGYEDGRDSNAAKNILIKGQKEFKTVYRAWAWEHGET
>SEQ ID NO: 14 
MFAIKSMSELVQHHITIQLKAYLSTTQTALFENWTDSLRPLYNLALGLLYEEQQRRWRTNQKFLKNYLDKS
SLQTYLNEIENKPDIYPVEWHITKALPECDWLTKEENEVRKKDNTKSLACRTINRDGNFFTPIRPYWHLEE
PAKLAKFKCFTNQWLISCNLLTNYHLQKLLNVTMKVRQSFISMNLMEAWKRYQKGDFRKLKFKSKRNPVIS
LCNKQTAIIKFDPEANNCQLLGKEFGLIEFRGLHNRHQGQIQPRNGSLTKKADGYYLNLVFQVEHKPIPDS
DLQVGIDPGLVTLLTLSDGKCISNPRFLKENERHLTVLQKKLSRQTPGSKNWEKTKKALAKIHKQTADHRK
YYNHKVSTHLVNKYGAIAIEDTKLTNMNKRPKAEKREDGKGYEHNGAKAKAGLNQSLHDAGLGQLRAFLES
KANSYENRHIERVKANYTSQKCSRCGHTDKENRLTQASFYCLKCGLEMPADLNAAINIEQAAFGLDKS
>SEQ ID NO: 15 
MVKKPPKEKEDKPKKVIEFKIHPNKEQVEEINRSFASCKLLWNLSIALKEESKQRYYRKNYKFDEFSPEIW
ELSYSGHYDEEKFKSLKDKEKELLIKNPCCKIAYFKKTSDGKEYTPLNAIPIRRFMNAEDIDKDAVNYLNR
QKLAFYFREDTAKFIGEIETEFKKGFFKSVIKTAYDAAKKGIRGIPKFKGRRDKVETLVNGQPDTIKIKSN
GVIVSSKIGLLKVRGLDRLQGKAPRMAKITRKATGYYLQLTVETDDTIYKESDKCVGLDMGAVAIFTDDLG
RQSEAKRYAKIQKKRLNRLQRQASRQKDNSNNQRKTYAKLARVHEKIARQRKGRNAQLAHKITSEYQSVIL
EDLKLKNMTAAAKPKEREDGDGYKQNGKKRKSGLNKALLDNAIGQLRTFIENKANERGRKIIRVNPKHTSQ
TCPNCGNIDKANRVSQSKFKCVSCGYEAHADQNAAANILIRGLQDEFLRAIGSLIKFPVSLIGKYPGLEGK
FTPDLDANQESIGDAPIENAEHSISKQMKQEGNRTPTQSENGSQSLIFSSAPPQPCGDSHGKNKPKALPDK
ASKRSSKKPRGAIPENPDQLTIWDLLD
>SEQ ID NO:16 
MSKITRKIEIIPDVEGLTHEESNEKCYKAFYNYDRKLYKVANLLVSQLYGLDNLLSLMRLQNEEYVDSQRK
LSFKSTTDTAKEEIKKRMEEIDAELMAIKKKIAPMHPQSYSYRAVNSSEYAKDMPSDILDSLKQDVYKHFN
DSKKEQIRGERSLTTYKRGMPIPFNLKKKHSIVCDGGNYYLPWFEDTRFRLNFGRDRSNNRAIIDNCIKTK
KYKLCAAAKIQLKERKLFLLITVDIPKAESVPVKGKVMGVDLGVVNPAYVAVNDGPERSRIGNGEAFQKQR
DVFRRRFRELQRSQLTQGGHGRKHKTKATEILRGKERNWVQTENHRISREIVNLASRWKVETIQMESLKGF
GKNQEGEVENNHKRLLGRWSYFELQKDIEYKAAMAGIKVQYVNPAYTSQTCHVCGQRGNRIERDTFICTNP
ECTCYNQVQDADMNAAINIAKSIKVVK
>SEQ ID NO:17 
MLQHVGTTWVSWTRTCRAGGGRWVSVASTKTVVLRAFKFTLAPTATQDQQLLRWCGNARLAFNYALASKRA
AHTEWRAQVDALVTSGVKEPVARKRVTGPKTPTKPAVYKAFIAERGDTREGLDGVCPWAHEINTHVFQSAF
IDADRAWKNWLDSFKGTRKGRRVGYPRFKKRGRARDAFRLHHTVTKPTIRFSTHRRLRLPTFGEVRLHDSA
RTLVRQIDRGTAVVQSVTVSRAGHRWYASVLCKVEMDLPSGPTRSQQAAGTVGIDFGVKALAALSKPLVPD
RPESTLLPNPRHLAKAAHRLKRAQQTLSRRQKGSARREKARRRVARLHHEVAVRRQSALHQITKRLTTRFA
 
TIAVEDLHVSGMTRSARGTMDKPGRKVRQKAGLNRAILDASMAEARRQITYKTSWYGSRLAVLDRWWPSSK
TCSACGWQNPSLTLADRVFECAQCGLTLDRDLNAARNIEQHAVQVASGTGETQNARGEPVRLPRPRAEKQG
STKREDTGPPGPVPPRRSDPPTPPNPRQGQAKLF
>SEQ ID NO:18 
MPIITRKIELKIVRLTDEEYDQQWKYLYQINNTIYQAANRISTHCLFNDEYEMRLRLRYMSQCNKIDKKLE
KLNPDKKTSDKEEQYRLLNERKELDEDFKNKKQDFQRIKRNSTYQLVSKEFLNYIPAEILTDLNQYVQNNH
NNNKNKVKSGERALSTYKKGMGIPFSIKPESGLRLFVKEEGIYLKWFKGILFRLEFGKDASNNRCIVERLI
ESDKQQKKKGEDYVANNSSIKLVKNGKNTRIFLLLSIDIPAKKQVLDKEVVLGVDLGIKCPLYLAINKDDN
FKMQIGDIEHFHNQRTMFQKRFKSLQKLMCTQGGHGRKKKLEPLEKLKEKERNWVHTQNHVYSREVIKQAL
KQNAGTIHMESLKDFGKGKEGYVKDEYKYLLRYWSYYELQSMIEYKAKLEGIEVKYIDPAYTSQTCSYCGE
RGERKKQEEFVCTNPQCKRRGEKINADFNAARNIAMSKKIVKDN
>SEQ ID NO:19 
MINKAYKFRIYPNQAQAILINKTIGCSRFVFNHFLSLWDHAYKETGKGLTYGICSAKLPAMKKELVWLKEV
DSIAIQSSVRNLADAYTRFFKNQNSAPRFKSKKNNVQSYTTKQTNENIAVVGNKIKLPKLGLVRFAKSREV
EGRIVNATVRRNPSGRYFVSLLVETEVQELPKTHSYIGIDVGLKDFAILSDGTHYENPKFFRSLEEKLAKA
QHVLSRRMKGSSRWNKQRVKVARIHEYISNARKDYLDKISTEIIKNHDVIGIEDLQVSNMLKNHKLAKAIS
EVSWLQFRSMLEYKAKWYGKQVIVVSKTFASSQLCSCCGYQNKDVKNLNLREWFCPSCQTHHDRDINASIN
LKNEAIRLLTARTAGLA
>SEQ ID NO:20 
MSSERAPKLRNVVTQQAYKYALEPTPRQQCAFSSHAGAARFAYNWGIARVADSLDAYAEQKAAGIDEPDVK
FPGHFDLCKMWTAWKNTAEWTDRHTGQTTTGVPWVASNFVGTYQAALRDAAGAWQRFFRARKTGARAGRPR
FKKRGRARDSFQLHGDGLRIVDAKHVNLPKIGTVKTFEATRKLARRLAKGSVPCPTCRATGKITDSASGKV
KKCSDCKAAGSRPAARIVRGTVARDSAGRWYLALTVELVREVRTAPTPRQLAGGPVGVDFGVRQVATLSTG
QLVDNPRHLESHLRRVKTAQQALSRCPPGSRRRAKAQQRLGRLHARVRHLRENSLQQATSALIHQHSVIAV
EGWDVQQTAQHASPKNLPKQIRRNRNRALLDTGIGAARWQLQSKGAWYGTTVVVTDRHAPTGRQCSACGTV
KATPIPPTQDEYRCPACGTSLDRRTNTARVLAAVAAQHHDAPSGGESKNARGENTRPTAPRRNGQFSAKRE
PRSRPPGRGQTGTPGT
>SEQ ID NO:21 
MDAKEFTGQYPLSKTLRFELRPIGRTWDNLEASGYLAEDRHRAECYPRAKELLDDNHRAFLNRVLPQIDMD
WHPIAEAFCKVHKNPGNKELAQDYNLQLSKRRKEISAYLQDADGYKGLFAKPALDEAMKIAKENGNESDIE
VLEAFNGFSVYFTGYHESRENIYSDEDMVSVAYRITEDNFPRFVSNALIFDKLNESHPDIISEVSGNLGVD
DIGKYFDVSNYNNFLSQAGIDDYNHIIGGHTTEDGLIQAFNVVLNLRHQKDPGFEKIQFKQLYKQILSVRT
SKSYIPKQFDNSKEMVDCICDYVSKIEKSETVERALKLVRNISSFDLRGIFVNKKNLRILSNKLIGDWDAI
ETALMHSSSSENDKKSVYDSAEAFTLDDIFSSVKKFSDASAEDIGNRAEDICRVISETAPFINDLRAVDLD
SLNDDGYEAAVSKIRESLEPYMDLFHELEIFSVGDEFPKCAAFYSELEEVSEQLIEIIPLFNKARSFCTRK
RYSTDKIKVNLKFPTLADGWDLNKERDNKAAILRKDGKYYLAILDMKKDLSSIRTSDEDESSFEKMEYKLL
PSPVKMLPKIFVKSKAAKEKYGLTDRMLECYDKGMHKSGSAFDLGFCHELIDYYKRCIAEYPGWDVFDFKF
RETSDYGSMKEFNEDVAGAGYYMSLRKIPCSEVYRLLDEKSIYLFQIYNKDYSENAHGNKNMHTMYWEGLF
SPQNLESPVFKLSGGAELFFRKSSIPNDAKTVHPKGSVLVPRNDVNGRRIPDSIYRELTRYFNRGDCRISD
EAKSYLDKVKTKKADHDIVKDRRFTVDKMMFHVPIAMNFKAISKPNLNKKVIDGIIDDQDLKIIGIDRGER
NLIYVTMVDRKGNILYQDSLNILNGYDYRKALDVREYDNKEARRNWTKVEGIRKMKEGYLSLAVSKLADMI
IENNAIIVMEDLNHGFKAGRSKIEKQVYQKFESMLINKLGYMVLKDKSIDQSGGALHGYQLANHVTTLASV
GKQCGVIFYIPAAFTSKIDPTTGFADLFALSNVKNVASMREFFSKMKSVIYDKAEGKFAFTFDYLDYNVKS
ECGRTLWTVYTVGERFTYSRVNREYVRKVPTDIIYDALQKAGISVEGDLRDRIAESDGDTLKSIFYAFKYA
LDMRVENREEDYIQSPVKNASGEFFCSKNAGKSLPQDSDANGAYNIALKGILQLRMLSEQYDPNAESIRLP
LITNKAWLTFMQSGMKTWKN
>SEQ ID NO:22 
MTGGNSMGGKVLCQTIRLELEYLYDEDGKRLPYYTELKRIQEQVYLADNRTIQILWEWDNYSVFYKKEHGV
APKAIDFSNGQGSIRGYVYDILKTEFPDWYSGNLTTSIQQTLASYKEVRKDVQAGKRSITSYKKDAPMDIH
NACISFEFEGGNAAVSLKVFSEPFRKACGYGSTTVTFKVKKLGKAVREIIELCARGTNGYAFGASKLIYNA
KKDCWFLHLAYKFRARENENLDPNHIMGVDLGIKYAAYMGFNHSPERYYIKGGEVETFRHKVEARKRSLQE
QGKYCGDGRIGHGYQTRMKPVQKLSDSIARFRDTANHKYSRYIVDMAVKHGCGKIQVEDLSNIKDRQDKFL
QEWTYFDLQQKIKYKAEAEGIEVVKVAPHHTSQRCSQCGYIDENNRPSQSEFKCGRCGFTANADYNASLNL
ATAGIEELIAEELRAKRKNASKH
>SEQ ID NO:23 
MSQTITVKIKLLSTKEQASILKNMSKEYISTINSLVSEMVAEKKSTKKTTKDVPANLPSAVKNQVIKDAKS
VFQKAKKSKYTAVSVLKKPVCIWNNQNYSFDFTHISMPIMIDGKTTKTPIRALLVDKDNRNFDLLKHKLGT
LRITQKSGKWIAQISVTIPTVERTGLKVMGVDLGLKVPAVAVTGDDKVRFFGNGRQNKYVRRMFKSKRKKL
GKLKKLNAIRNLDDKEQRWMKDQDHKISRAIVDFAKENKISVIRLEQLANIRQTARTSRKNEKNLHTWSFY
RLSQFIEYKANLVGIKVEYVNPAYTSQTCPKCSARNKAQDRKYKCECGFEKHRDLVGAMNIRYAPVIDGNS
QSA
>SEQ ID NO:24 
MPIITRKIELKIVKDGLTDEEYDQQWKYLYQINNTIYQAANRISTHCLFNDEYEMRLKLHMPRYKEIDEGL
ENIKTELEKLNTKKKASDKEKRDRLLNEQRKLVDERKELDKDVKNKKKDFLQCSKQNSTYQLVSKEFLKYI
PAEILTDLNQYVQNNYNNNKRKVKSGERALSTYKKGMGIPFSIKPESGLRLFIKENGIYLKWFKLNNDRKE
KEILFRLEFGKDTSNNRCIVERLIESDRQQKKKGEDYIANNSSIKLVKNGKNTRIFLLLSIDIPAKKQMLD
KDVVLGVDLGIKCPLYLAINKNDNFNMQIGDIEHFHNQRTMFQKRFKSLQKLMCTQGGHGRKKKLEPLEKL
KEKERNWVHTQNHVYSREVIKQALKQNAGTIHMESLKDFGKGKDGYVKDEYKYLLRYWSYYELQSMIEYKA
KLEGIEVKYIDPAYTSQTCSYCGERGERKKQEEFVCTNPQCKRCGEKINADFNAARNIAMSKKIVKDN
>SEQ ID NO:25 
MSVSFSLNAKKIRLENYAMKMRLYPSPTQAEQMDKMFLALRLAYNMTFHEVFQQNPAVCGDPDEDGNVWPS
YKKMANKTWRKALIDQNPAIAEAPAAAITTNNGLFLSNGQKAWKTGMHNLPANKADRKDFRFYSLSKPRRS
FAVQIPPDCIIPSDTNQKVARIKLPKIDGAIKARGFNRKIWFGPDGKHTYEEALAAHELSNNLTVRVSKDT
CGDYFICITFSQGKVKGDKPTWEFYQEVRVSPIPEPIGLDVGIKDIAILNTGTKYENKQFKRDRAATLKKM
SRQLSRRWGPANSAFRDYNKNIRAENRALEKAQQDPGSSGVGPEAPVLKSVAQPSRRYLTIQKNRAKLERK
IARRRDTYYHQVTAEVAGKSSLLAVETLRVKNMLQNHRLAFALSDAAMSDFISKLKYKARRIQVPLVAIGT
FQPSSQTCSVCGSINPAVKNLSIRVWTCPNCGTRHNRDINAAKNILAIAQNMLEKKVPFADEALPDEKPPA
APVKKAARKPRDAVFPDHPDLVIRFSKELTQLNDPRYVIVNKATNQIVDNAQGAGYRSAAKAKNCYKAKLA
WSSKTNK
>SEQ ID NO:26 
MPEIVKERITHHAMKMRLYPSPEQAEKIGRIFRALQLAYNITFHAVFCMDPAVCEEPKADGAVWPSYKKMA
KAPWRHALIEKNAAIAEAPAGALMTNEGLFLRDARRAWETGMHNLPINKADRKDFRFYHAGKPRRSFLVQM
ACGNLVPSPDNPKVAWITVQGVPGKMKARGFDRKIWFGAGGAHTYEEAVAAGEVASNLTVCVSQDSCGDYY
ISVRFSIGKKGARELYRETRACASPVPIGLDVGIKDIAITSEGTKYPNRRPKQRKSPVLERMSRQLSRRWG
PANPSFRDYNKAVRQANRTLDQGPPKPLAQPSRGYLAVQRSRARLERRIARQRESYYHQVTAELVRRASML
AVETLHVKNMMQNHRLAYALGDAAMSDFLEKLRYKAERSHVPLVAVGMFEPTSQRCSVCGAQNEAVKSLAV
RRWICPSCGAEHDRDINAARNILTIAQTTGPGEDKAKKDAGPRPKRGPRRPRAEVVFADRPQIVVRFSPEL
TKLNDPRYLIVDKETGAILDDAQGAGYRSITKAKNCYKAKAAWTKKRQETP
>SEQ ID NO:27 
MEQMTITAKVQIVATDTDKVLLDETMSAYRDACNYVSDYVFQTHDLKQFSLNKVLYSTLREKFGLKSQMAQ
SVFKTVIARYKTILENQKEWIKPSFKKPQYDLVWNRDYSLTQNCFSVNTLNGRVKLPYFADGMSKYFDHTI
YKFGTAKLINKHGKYYLHIPVTYDVEESNISDICNVVGIDRGINFVVATYDSNHKSGFVSGKAIKQKRANY
SKLRKELQMRQTPSSRQRIKAIGGRENRWMQDINHQVSKALVENNPKHTLFVLEDLSGIRNTIERVKTKDR
YVSVSWSFYDLEQKLIYKAKQNQSSIIKVDPRYTSQCCPCCGHVEKSNRNKKIHLFTCKNCGYKSNDDRIG
AMNLYRMGINYLVDSQVPNTVVTE
>SEQ ID NO:28 
MAETAVLRAFRFALDATAAQEEGFLRHAGASRWAFNHALGMKVAAHRQWQREVKALVEGGMLEAQARKTVK
VPVPTRPTIQKHLNRIKGDSRSPDHPEGAQGPQRPCPWFHEVSTYAFQCAFEDADRAWDNWQASLSGRRAG
RKVGYPRFKKKGRTKDSFRICHDAKKPTIRPDGYRRLRIPVLGSVRLHDTAKPLARLVDRGASVKSVTVSR
SGARWYASVLVSVLQDLPERPTRRQRQAGTVGVDFGVKTLAALSAPVTLPNLGTLTMVPNPRHLASDTRRL
TKAQQTLSRTTKGSARRRKAAQRVGIVHHRIASRRATYLHTLTKQLATGYAVVAVEHLNVAGMTSSARGTV
EEPGSKVRQKSGLNRSILDASPAEMRRQLDYKTRWNGSQLAVCDRWFPSSRTCSACGWQKPRLTLAERVFN
CGQCGLVIDRDLNAARNIAAHAVLVPHGTAAPGSGEASNARGAATRPATPRGGRQAALKREDTGPPRPVPP
QRSDPLTLFTLDVPDQQTAKRP
>SEQ ID NO:29 
MATEYTCITRKIEVHLHKHGDGEEAEQRRAEEFRIWNEINDNLYKAANRIVSHCFFNDAYEYRLKIQSPRY
NEIQKSLRYTKRNKLTDDEIKSLKAERKSLFDEFKKQRQTFLRGGVSEGANPEQNSTYKVVSNEFLDVIPS
HILTCLNQNVSSTYKCYSKEVEFGNRTIPNFKKGIPVPFPIKHGNILLLKKREDGSIFVDFPKGLEWDLSF
GRDRSNNREIVERILSGQYDAGTSSLQEGKNGKIFLLLVVKIPKQSNALDPNRVVGIDLGINIPLYAALND
NEYGGMSIGSREQFLKMRMRMTAQKRELQRNLHYSTNGGRGRSHKLQALERLEGKERNWVHLQNHIFSKNI
 
IEFAVKNNAGVIQMERLTGFGHDRNDEVDDGFKFILRYWSFFELQSMIEYKAEAAGIEVRYIDPYHTSQTC
SFCGHYEKGQRIDQATFICKNPECEKGKGKKRSDGTYIGINADWNAARNIALSDKFVDKKKK
>SEQ ID NO:30 
MKTTEKNVLMTKCIKVTLNRCVNYNMKEIMNIIREMQYLSSKAYNLATNYLYIWDTNSMNFKNLYEEKIVD
KDLLGKSKSAWIENRMNEIMKGFLTNNVAQARQDVINKYNKSKKDGLFIGKVTLPSYKMNGKVVIHNKAYR
FSKNEGYFVEIGLFNKEKKEELNCDWIKFKLDKIDSNKKATIYKILNGDYKQGSAQLHINKKGKIEFIISY
SFERENSIKLDKNRTLGIDIGIVNIAAMAIWDNNKQEWELTRYSHNLISGNEAIALRQKYYKLGLRNKELE
KNINRELHELEEKEYRGLSTNIISGHNLTYKRIMLNSKRIRLSQSCKWCGNSKVGHGRRVRCKQVDKIGNK
IERFKDTFNHKYSRYIVDFAVKNNCGIIQMENLKNFNPSEKFLKDWPYFDLQTKIEYKAKEYGIEVIKVNP
KYTSKRCSRCGCINELNRDCKKNQSKFKCVNDECNNYENADINAAKNIALPYIDKIIEQCLETNKVV
>SEQ ID NO:31 
MLRAYRLELEIASERKRRFLHQHFGAARFIYNWALRLVREKGFEFFKQGKGGIGKRILYYWREERDKVAPW
HHEINAHTFNGAVLDLGRDLKHHSPSQLRGRSRNRHRSAHFYGIKLQHIGRRSIKLPGSRAGEFRGGVYLK
VKKGTGKLYEDIQQGRIRTIKRITVSERAGRYFASVLCEVQEPEPLPCTGRVCGLDVGISSIVTVALSDGR
IIKQGNPQWLAKKLRRLRRIQRSLARCKTHFPRDPGFKSLHILRRDGEVLAVFPLMWRRQNGELVPEVGDD
VRILAEGVASYALLSRAPDRLEKPYRLTVGGQGSGANIELSTMRIRQGHPVRVVHLSVDRSHTMERRRKTL
ARAHYRVSVAREDFWHRLSTWLVRNCDVIVVESLSIKGMLRSGRFSRHIADAAWGTFFQMLQYKCERYGRT
LLRVERSYPSSKKCSRCGKIRKALSLSERTYRCDQCGLKIDRDENAALNLMKLGLAHLTSPTARLAGSDAG
GDTIAGGAAACT
>SEQ ID NO:32 
MITVRKVKLIVNSEEAEEINRTYKFIRDSIYAQYQGLNRCMGYLLSGYYANGMDIKSDGFKNHMKTIKNSL
NIFDDINFGIGIDSKSAITQKVKKDFSTSLKNGLAKGERGATNYKRNFPLMTRGRDVKISYLEDINTFVIR
WVNKIEFKVILGQKDNIELSHTLHKIINKEYNLGQCTFEFDKNNKLLLALNINIPDNLISKNKEIIPGRVL
GVDLGVKVPAMICLNDNTFIKKSIGSYNEFFKVRSQFKARRERLYKQLESSNGGKGRKHKLKATMQFRDKE
KNFARTYNHFLSKNIIEFAKKYKCETINLEELNKKGFDNNLLGKWGYYQLQSMIEYKAERVGIKVKYVDPA
FTSQTCSKCGYVDEENRITQDKFECQKCGFTLNADHNAAINIARK
>SEQ ID NO:33 
MTMFERYVKLPVYTINLRLYPTSSQKETIDTIIHELHKACNIAVYDMFTNLTNTTEREDKESKQKIHFPNI
NTMIKKDYLEKLREERPSISIIPASALSGNNGVFKRDLSKRLDAQVSEGNQKKKTNGKGVKRPIESSKAPY
YSKKHPRRSYTYQETLSKIIFNETNDNVVHLNLNKVGRIKARGMKNYLRILRFDETCNMTFKEYCELHKRT
AHLFTIKKDNCGDYFIQICLKNVYKLIKDSDNKKEIGIDVGVTDLMILSDGTKYSNPRFKNGEHGEVQKHR
EQLHRQLSRREGFANIKFREKYKVDHDTLPSKRYQETKLQAAKLERKVARQRKHHMENMVLDVIRQSSFIG
IENLSVKDMMKNVKKNKSET
>SEQ ID NO:34 
MPIITRKIELKIVKDGLTDEEYDQQWKYLYQINNTIYQAANRISTHCLFNDEYEMRLRLRYMSQCNKIDKK
LEKLNPDKKTSDKEEQYRLLNERKELDEDFKNKKQDFQRIKRNSTYQLVSKEFLNYIPAEILTDLNQYVQN
NHNNNKKKVKSGERALSTYKKGMGIPFSIKPESGLRLFVKEEGIYLKWFKGILFRLEFGKDASNNRCIVER
LIESDKQQKKKGEDYVANNSSIKLVKNGKNTRIFLLLSIDIPAKKQVLDKEVVLGVDLGIKCPLYLAINKD
DNFKMQIGDIEHFHNQRTMFQKRFKSLQKLMCTQGGHGRKKKLEPLEKLKEKERNWVHTQNHVYSREVIKQ
ALKQNAGTIHMESLKDFGKGKEGYVKDEYKYLLRYWSYYELQSMIEYKAKLEGIEVKYIDPAYTSQTCSYC
GERGERKKQEEFVCTNPQCKRRGEKINADFNAARNIAMSKKIVKDN
>SEQ ID NO:35 
MTKVVKLPLICEQSDKDGNPIDYKKIYEILFELQRQTREIKNKSIQYCWEFSNFSSDYYKQNHEYPKEKDI
LSYTLVGFVNDKFKTGNDLYSGNCSTTVRGACGEFKNSKTDFLKGTKSIINYKGNQPLDLHNKTIRFECIG
KDYYAYLKLLNRPAFQRNNFSSSEIKFKVLVYDNSSKTIVERCIDNIYKISASKLIYNEKKKCWVLNLSYS
FTNNNVCELDENKILGVDLGIHYPICASVNGERKFFKIDGGEIDHTRRKIEVRKKSLLKQGSSCGEGRIGH
GIKTRNKPVYNIEDKIACFRDTANHKYSRALINYAVNNNCGIIQMEKLTGITADSDRFLKNWSYFDLQTKI
EYKAKEAGITVVYIDPQYTSQRCSKCGYISKENRKVQAKFCCQKCGYEANADYNASQNIGIKDIDKIIKNT
K
>SEQ ID NO:36 
MITVRKLKLTIINDDETKRNEQYKFIRDSQYAQYQGLNLAMSVLTNAYLSSNRDIKSDLFKETQKNLKNSS
HIFDDITFGKGTDNKSLINQKVKKDFNSAIKNGLARGERNITNYKRTFPLMTRGTALKFSYKDDCSDEIII
KWVNKIVFKVVIGRKDKNYLELMHTLNKVINGEYKVGQSSIYFDKSNKLILNLTLYIPEKKDDDAINGRTL
GVDLGIKYPAYVCLNDDTFIRQHIGESLELSKQREQFRNRRKRLQQQLKNVKGGKGREKKLAALDKVAVCE
 
RNFVKTYNHTISKRIIDFAKKNKCEFINLEQLTKDGFDNIILSNWSYYELQNMIKYKADREGIKVRYVNPA
YTSQKCSKCGYIDKENRPTQEKFKCIKCGFELNADHNAAINISRLEE
>SEQ ID NO:37 
MRKLRKSFKTEINPTEEQKIKIRKTIGTCRYIYNFYLTHNKELYDNGKKFMSGKSFSVWLNNEYLPNHPEY
SWVKEVSSKSVKHSIECGCTAFTRFFKHQSAFPNYKKKGKSDVKMYFVKNNPKDCICERHRINIPTLGWVR
IKEKGYIPTTKDGYIIKSGSVSIKVDKFYVSVLVEIPDGQIANNSNDGIGIDLGLKDFAIVSNGKTYKNIN
KSARLKKLEKQLIREQRCLSRKYDENLKKGEVTQRANIQKQKRKVQKLHHKIDNVRTDYINKTIAEIVKTK
PSYITIENLNVKGMMKNRHLSKAVASQKFYEFRAKLQSKCNENGIELRVVARWYPSSKMCHRCGCIKRDLK
LSDRIYRCECGYVEDRDFNAALNLRDAITYEVA
>SEQ ID NO: 38 
GTAATAACTTAACTATATAGAATATGAATGTGATCGACAGCAACAAGTGAGCGGACGCAACTAATGTAATA
ACTTAACTATATAGAATATGAATTATTTAACAAGTTAAGGCGATTCAACGTCTTTGGTTAGAGGATTATAT
CACTCGGCAAAAGGGTTAATGTATGTCAATATGTTACCGACATACTATTCTAAAAAATAATTTTGCAGTAA
ATCAAATTTTCATTAAATATGCCTTAAAGTTAGTTGCATCAATATATTACATGTATTTTTAATTTTGTTAA
TAAAATATTACTATAGAATTACTGCAAAAACTTTCGTTTTAAAGATGAATAATAGCACTATAACTATATTT
ACATTAGGCATTCAAAGATTTTTATATACGAACTGATTTATAAATTAGAAATTTAGCTATATAAAAGGAGT
ACTTAATTTAATAAGGAACAATTAAATTTTTACTAATATTGAGTAGTTAAATACTACTCTTTTTTTATGCT
CAATTATAGAAAGGACGTGATTATTTGGATACGGAATTATGGAAG
>SEQ ID NO: 39 
GTGCTCAACGCCTTACGGCATCAGAGGTTTGATCACGTGATCGACAGCAACAAGTGAGCGGACGCAACTAA
TGTGCTCAACGCCTTACGGCATCAGAGGTTTGATCACAGATTATCTTTCGGCTGATTTTCGCTCTCTAATG
AACGCGAAAGATGGGCGTTTTCTGAATCGATTGGTGGCATGGTGACAGATCACAGGCGATCGGGGGGTGAC
GAAGCGGAGGCGAAGAGCGGCGGAGAGTAAGCCAGGTATGAATCGGCGGGTTAAAACTCCGATTTTGGGGA
GTACGGGTTTCTACTGATGCGAAAGGATTTGAAGGAGGTGGAGGCGATCAAAAAGGTGAGGTGGCGAATCT
GACGACAGACTCACCACCTCACGGGATTGGAAAGTTTGGAATAGGGCTGAAACGAGTGCTGTGCGGTGCTG
TGCGGTGCTTTGCATTTTCGTCTACTCCTCAATTCTGAAGCCTATTCTGTAACCTTCGTCTGGAAAGTCTT
GTTCCAATCGATCGACTGCGGTTGGCTGTCTCTTAGGTTTAATGAAACTAATCAGGAAAGCTTGCGACGAT
CATAGGTTGCTGAAATATTTGATCGCTTTGCATTCATGCTGTTTTTATGTGCTTAACGATACCTAAAGGCA
GCAAAATCGAGATTTTTCAAGTATAAAAACATTCATCAAACGCCAAGATCGATCAAAGGTGCAAAGACTGA
AAAATTGTATATTTGAATACGGTTTAAGATAAAATTAAAGAATTTCAAAAACTTGTAAAAAAATAATTCAC
GGTGACTTTTCCAAAAAAATTGGAGTACACTACCTGGGGAGTGCAGGCGTGCAAATACAGAGCGTCGCCGA
CCGTTAGCAACGTAAACAGTGACAGTCATGGGAAATAGCACAAAGTCCTCGAAATTTCCGATTGTTATTGA
AATGGAGCCACAGGCAAACTCTGACATCGAAGAATTGGTGAACTCCGGCAAAAGTCTCATTTGTGACAACG
TATCCGTTGCCCTAGAGCAACTGAAAGCCCAAGGAATTTTGCCCGAAAGCGTCATCCCCGTATTTGTTGTT
GTTGCATCCTCCGACGACTAGTGAAGAACTGATGTGCAATCATCCCCAAAGGCTGCTGAGCTGAAGCTTAA
GCTGAAGTTTTGGGGACAGATTTCGCAAGTGTCCGATCGTCCCGTGTAAGCATCAGCGCAAATTTACCCGC
CGCTTGAAACCCTTACGGGACAAGCGCCGAAATAGAACCTTACAAGCATCTTAGCCGATCGCCCCGCATCT
GCCAAAACATCACCCCCGATCGCCCTTTTGCCGATCGCCCAACCCTTGCCCCATCTCCCTTTCAGCCATTT
ACAAGCATCTCCAAAGATCCCAAAAATCGCCAAACCCAACCCCCATCAACATTCTGCGCCCTTCCAGCCCC
TCGCCCCGCCGCCACCGCCCCAGGTGCTTGTTTTTGGGCAACATTCCCACCCGATCGCCCCCGCCCGATCG
CGATCCATGCCGAAAGCCCGATTGCCCCATTGGCCCCAAACAATCCAGAGCGCCAAGGCGAATTAGACGCT
ATGGAATTCGTTACAATGCGGTTGGCTTCCTTTCCCCGTTTGCCCTACCGTTCCGCTCCTGGCGATCGACC
TGGGGCCCATGGGAGCCAACCCCTTGCAAGATGGTTCAAAGCTTGAGAATTTAGGGTGCATTCCGGCGTAA
CTTTCGGATCAGTGGGGGTTAAGGGCTGTTTTCAGGGACTTTTGCAGGGCGATCGGGACTCACCATCCCCA
TGCACCCGGTGAACCTCCCACAGTCGCATCCGGAGTTCGTTCCGCAAAGGGCGTAAGGAACATTGAGTCCT
TACTGGGTCACGGAACAAAGCCGATCGAGGCCGAGTGGGAAGGAAGCCAGTCAGCGGCGCTCCGAACTACA
ATAGGTCATCAAAATTCGCCAAGAGCCTGATCGGTGGTCTGAGTCCTACTCCATCCGGCATCAGTCCCTGG
GTAACCTGTTCTGAAAACTCGCAGTCGCCAATGCCCTAAACCCTTACCATTCGTTGGCTGAAGCGATCGAC
CAGTTGTTCGTTGCCCTTTGGCATTTCAACTCCCCATCATCCCGCGCTAGGGCGCTATGGCCGGCTCTTTG
CTCCCACAATGGATGCTTCTTCACTCCCAACGGCAACTTGGCCCGCAGTTTCTCCAGCACCACAGCCTGGG
TTGCAACCCGATCGGGGCTGGGGGGCAAGCCCC
>SEQ ID NO: 40 
GCTTCAATGAGGCCCGGGCTGTTGGCCCGGGAGTCGTGTGATCGACAGCAACAAGTGAGCGGACGCAACTA
ATGGGCTTCAATGAGGCCCGGGCTGTTGGCCCGGGAGTCGTCTCGAGCGCCGGATCGCCGCGCGGTTCGAT
CGCGCCGGAGGGGGCCCCACGGGGTGCCCGTCCGGCGCGGGAATCCGGACCGCGAACGCTTAGAGCCCTCG
GCGGCGCGCGATAAGATGTATATACATCGTGGCCGACATGATGCATATGCATCTCTGACGTTCCTCTCTCC
 
TCCGGCAACAGGTCGCTCCACCGGAGCGTGACGGGGCTAACGGCACGGTGTCGCAATGAGGCCCGGGCTGT
TGGGCTGGGAGTCGTTCCGGCTCTATCTCGCGCTCGCCGAGAACATGCGATTCAAGCCTCGGGACCACCTC
AAGCGCATCGAGCACCTGAAGCTTCAATGAGGCCCGGGAGTCGTTTGACTGCAAAAGTCAAGTCTCATTCC
GAATATCAGGGCCCCCCGGATTTCCCCTGGCCGGCGAGTGCCTTGCAGGGGTGTTTGCGAGGGGTCCGCGG
ATGCATGCGGGTGCGCATGGATTTGCCCACGTTCCGGTTCATCCACGATAACAAAGAACCCGCCGGAATCA
AGGCGTTGGCCGGTCTGCGAGCGCTGGGCGGAAGATCTGTCGCGCGGCAGCGCTCGCGCCGAGGCGGCGAA
GCTT
>SEQ ID NO: 41 
CTTTCAATTCCACACGGTACATTAGAAACAGTGATCGACAGCAACAAGTGAGCGGACGCAACTAATCTTTC
AATTCCACACGGTACATTAGAAACATTTCAAAAGCCATACCAGAGTTTAGCAGGTTTGAGCGTTTCTAATG
TACCGTGTGGAATTGAAAGTACACTCAAACACATAAAACTTTCTTTTTCTCTTTGGTTTCTAATGTACCGT
GTGGAATTGAAAGTGGTCTTCCAGCTCCAGCTCCTTTTCAAACCTTACCTGTGTTTCTAATGTACCGTATG
GAATTAAAAGGCAAATAGGGTTATAATAAAAGTTTTAAAAAGGAGGTAGGTGAGATGAAAGAAGTGCTTTT
TGCTTTCAATTCCACACGGTACATTAGAAACGAGGTTTTTGGAGCTAAATTTAAAGCATGAGTATATTCTT
TCAATTCCACACGGTACATTAGAAACCCCAAAAGAGATATTCAAGAAAAACATTATACATCAACACTTTGC
TAAAAGGGGTACCCTTTTCAAATGAAGTTAATTTTCGGAAATCTTCAGTTCCCAAAAAGGGGCTGAAAAAT
TGCCATTTCTAACTATTGAAACTCAACAGTTCCAAAAGTACCTCATCAAAACATCAAAACACCAAAATGTT
TTGATGAAGACATCAAAGGTATACTACCCCTATAATCTTACAAACACAGTCCTTATTCAACTGTCAAGATG
ACAAGGGGCTTATAAAACCCCTCAATCCTTTTTTTGATTTTAATCCTTAGAGAGAGAAATTGCTTATGATA
TATATCATAAAGTGTGTTGTTGGTTTTAGCTTTAACACTTCCAGCAAGAAGTCTGGAATCTTCTAAAGTCG
GAGATGAATTGCTTACTGATAAAAATAGGTTTCGGTATATAATATTTATGGGAATAGAAAATAAAATCAAG
GTAAAGAAAA
>SEQ ID NO: 42 
GTGACAAAGCCCTGTGCAGCGGGCTCAAAGCTGCGACGTGATCGACAGCAACAAGTGAGCGGACGCAACTA
ATGGGTGACAAAGCCCTGTGCAGCGGGCTCAAAGCTGCGACCCGAGGAGTAATAGTTCTGGCCAACAAGCA
GTTTGAGCACCTTTTGGAGGACCAGTACGATGGCGAACAGTCGCAGCATTCAGCCCGCTGCGGAGGGCTTT
GGTCCAGCGGTCATCGTAGCACGTGCTAACTCTGTCGCCCGTCGAAATGAGCGCTCGCGAGTGGCTGATAC
AGGTCTGCGAGGCCCACAGGGCCAGGCAGCCATGGCGTCTTGTTGATTCACAGGCAGGTCCTTAACCGCGC
CAAGGCTCTCTGCAGCGGACTAAATGCTGCAAAACCAATCGGTCCCACCTTTCAGGCCCCCTCTGTTGCTA
CCGTACATCCGTTCACAGAGTCAGGGCCTGCGCGGCGGCGCGGTAGCCGCGGCTGGCGATCCGGCCGACCC
ATGAAGGAGCGCTATCTAGGCTGGAACTTGCGTCGCTTTTGAGCCAGAAGTGGCGCTCGCGCGACGCAAGT
TCCATACTAGTTAGTGTTTACGGCGGCTACCGTGTAGCAACTTGTCGTAACGGGTCGTCTGGTTGGTAACG
CGCTCGCGTGGCTGGTTGTAACCTCTTGTCCTATCGCGTGCTACGCTACCGAGGGACGTGAATCGCTCCCA
AACAGGAGTCAGCG
>SEQ ID NO: 43 
CATTTCATCTGAAATCCGGTGTGGTGATCGACAGCAACAAGTGAGCGGACGCAACATTTCATCTGAAATCC
GGTGTGAACGGGAGTCCACTGACAAGCCTCGGGGCTTGACCCCGAGGCGGTTGACGTCGGATCCACTGATT
TAACGTGAGGACGCTATTACTAATATGCGAGGAAACCGGATACCCTATGGTTCACATTTACAATTGTTGTC
TAATAAAAATTTACGTGTGGGGAGGGGAGTCGGCCCTGTCAGCGAGCGTGGTGGGTTTCATCGAGTGTCGG
CTTCCTCCCCGACCTCAAGGATCGGGGCATCCGCCTCGTACTGCCTGGCCTCGCCGGGGACGTCCTCGTCG
TCTCGACGGACCGATCGGAGTCGACCACTGGGACCGAACACCCCCACACCGGATTTCAGATGAAATCGATC
GAACGAGGGGTGGGGGAGAGGGTGGTGGCCTCGGCGGTCGAATAAGACCAAACCGGCGCATAAACGAATTA
GATGGACTTAGAGAGATTCTCGATGGTGCTGTACCTCTTACTGCTACGTTACTTTCTGAACTCTATTATAT
ATCTTACTCTAGCAGTAATCTAGTAGTAGTAGTGTAGTGAGAGAATCCATCTTCAGGCTCCTGCAGGCACG
AAGTGATATACCGCTGCCTTTCTCCTGGTATCTTTATATTAGACGCTGGCTGCCTGGCGTGAGAACGCAAC
CCACGACGCGTTCGGCTCGCTCGCGATCGATCCGATTCGCTCACGAGCGGGTCAGTTCGCTCACAGGGTAC
GTCGCGAAACCGATCACCCGCCGTCCTCGGTTTGCGGGCCGACACCGGGTTTCAGATGAAATCGATCTTTC
AGACCCTGACGAACTCGATATCTATGGCCTGAGGTTCTTCTCCCGAATCGTTCCGCCCGGTTTGCAACTAC
CTACCTCTCTCGCTTCCGTCCGGTTTAGGACTGTTCGACCGCCCGGATCCGACCTCCTCCTCGCAGCGCTC
GTCTCCCACCCTCCTCCCGTTTCGGGAGAGTCGCACCCAGTGTGCCGACGGCCCATCTCCAGCGGTCGGGA
CCACTCCCCGGTTACCCACCACTCGTGGAGGGAAAGACCTAAGGAAAATTCATCTGAAAGACGGTGT
>SEQ ID NO: 44 
GTGTCGCCGATGGTGGAGGTTTGGGTGATCGACAGCAACAAGTGAGCGGACGCAACTAATGGTGTGTCGCC
GATGGTGGAGGTTTGGGCTTCCTCCGGCCTTGTGGCGAATAAGGGTGCTGGTGCGCCACCTGTCCGAATCC
TGGATGTCTGATGCCGTGCCGGCTGCATGCAATGCCGCGCGCCAGGTCTCCTGGTGCATCCGGTTGGTGTC
GTCGCCACACAGGTTAGCGGCAATCGAGAGGAATGTCACTAGTTCGCGCGAAAAAAATCACAAAATCACGC
 
CTGCCCTTCGCCCCCGGGCACACATCTGGGAATCCACCGTGCTCAGCCTTCAAGCCAGCCATACAGCACCA
GGGAATTCCCTTACTAATTTCTGTGCCGAATCCTTTTATCATGCCCCTCGCCAAGCGGGACAGTGCTGTGC
TCAGTCCGCTTGGTCATCCCCTGCGATGTGGGGTCGCCGATGGCGGATGTTAGGAATCCCTATAGGAATCT
GTATCCTCCGTTTTCGGCGACGGTTTTGTAGCACAATCATAGACCATGTGATAGAATGAGAGTCATGAAAA
GAAGCGCCAGAACCAAAGACAGTGAGACATGGAGGGTTGCCGTCGCAATTCCGCTTGTAGAGGAGCTGCCT
CTGCTTCGGGCCTGTAACCTCCACAATCGGCGACCCATGGATAACCTGTTCTAGGAGGTTGTGTCCTCCGT
TTTCGGCGACGAACCGTATCATTTCGTTTGCCTGTGCCGTGCCTGCGCCATGCCTGCGCGATGCGCTGACG
GGAATCATGGGGTATACAAAAGGGGCCGGTCCCGTGGAGGGAACCGGCCCCTTGAGTTCGTTCGGTGCTGC
CGAATCATTTGTGGTTTTCAACGTCGTCGGACGCTGAAAACCACGTCGACCAGCAAACTGTAGTCTCGCGG
CCAGCGGTCTGCGGTCTGCGGTCTGCGGCCACGGCCAGCGGCCTACCGCGACCGTGCCTTCGAC
>SEQ ID NO: 45 
GTTTCAAAATCTAATAGAGATTGCTGTGGATTGAAACGTGATCGACAGCAACAAGTGAGCGGACGCAACTA
ATGTTTCAAAATCTAATAGAGATTGCTGTGGATTGAAACCTTACGATGTTCCCTGGTTTCGAGGGATACGG
CGGTGTAGCTTCGGCAGCCGCAATCTGCTGGTGAGATCAACCAGCGATTTCTAATAGAGATTAGTTATTCA
TGAACCTTGAAAACTGAATATTCGGATTGCCCTCTAATTCACCGCAATCTCTATTCGCTTTTGGAAGAATT
CCGACACAGGGTAGATGTTAAGTTGAAGTGATTCCGAGATAGGCTACAGCCCCGATCCCGATTGACTTCTA
AGGACTCAACTGCTCAATCGACCGTCGGCCCTTATGCAGTCAGGGTTTCAGCGATTTTGGCAGGATCAAAG
TCTCTCATTCAGCCATCTTTGGGGTTCAGTGTCGGAAACTGAGTGTCAAAGCTATGGCTGATAAGGATTCT
ACAGCAAGCGGTAGGGGCAAAAAATCTTGCGACCTTACCATTAA
>SEQ ID NO: 46 
GTTTCAATCCCATTGCTAGGATTCATTAATAAGAAACGTGATCGACAGCAACAAGTGAGCGGACGCAACTA
ATGTTTCAATCCCATTGCTAGGATTCATTAATAAGAAACAGCTTGATCTACTAAACCATGTTTTTCCTTCA
AAAGCAGTGATCTTAATCACATGAACGTCTTATTTTTTTAGCAGTTAATAATCATTTTCTTTTAGCGTTTT
GATAGCTCGTGAGCTATACCTTATACCCAATCCTGTTTAGATAAACTGATTTACATTCTATCGGTTCCCTG
TTTCCTCTTATATTTTTGCTATTATTAAATCAAGAGATTTTGAACCTTGAAAACTGAATAAAAATAGATCC
CCTAATTTTGGCGAGAGCCACCGTACCCAAGTTTCAAGGGGGAACGTCGGTGAAAGACCGGAAAAGCTTGC
CTTTGAGTGCCATAGTGTGCTGTCAGCAAGAAGCCGCAAGGCACAGCCTGAGATTACAGGCTAATCCCTCC
TTTTTAGGGGATCTTCGCTAGGACTTAACTATCGCTAGAACTTAACTAAAACTGACGGCTCGAAGCGGGGT
CAAAATCCCTGGCCTCCCGTCAGATCGCCAGAACCTTGATAATTAAAGAGTTGTAGCTGTTTCACCTCTGG
GTTATTACTAACATTGATGTCCCCAATCACTCGCGATCGCGCCAAGCCCATCCCCATAAGGATTGTGAGGT
TAAATCTGACGGGACTCAAAAAAATGGAAGCATGGGGAAATCATTTTCTCCCTGCTTCTTGTAGGGAGATT
CATTAGGAATTTTCGTGTTCCCGTCTTTGTTGACAAGAAACAAAGATCATTTTTAATTGTCGGGGGTTGCT
TATTCT
>SEQ ID NO: 47 
GTCTCCACTACAAAGCGGCGGAGCACGCATGTCAACGTGATCGACAGCAACAAGTGAGCGGACGCAACTAA
TGTCTCCACTACAAAGCGGCGGAGCACGCATGTCAACTGCCTATCAACTATATCCTTAAAATTACGAGATT
ATTCCGGGGTGATTTCGAGCACCCCTTTCAATTCGTCTATTTCAGACCACGGATTTGCAGTTAACTCTCGT
TATCATACAAGAACTCGCTCCTTAAATTAAACGGTTCGAGCGGTACCGATGGTTTACGCAGGCTCTCCCTT
GAGAAAAATCTTTGTCACGTGACCACGAATATTAATACAACACCGTTGTGTAAACACGCAACCATCTAATG
GTTATTGAATAATTCGCATACACTAAA
>SEQ ID NO: 48 
GTCTCAGAACCCAAAGATCTGAGCTTCTCTTGTGAGGTGATCGACAGCAACAAGTGAGCGGACGCAACTAA
TGTCTCAGAACCCAAAGATCTGAGCTTCTCTTGTGAGATCGCTACGCTAGCAACTAGCTCACTTTACGTCA
AGAGCTACTAACACACTTTACGAACAGGTGTTAGGTTCAACGAATACCAGCTTGCCAGGCTTGAGCGATCT
TTACCGCACGAGGTATCTTGGCTTCTTCATCTCCGGGAACAGAGGTCGTATGCGGTTGTCAAAGAGCGCGC
GGACCGGCCCTTCGTCGGTCTAAGATCCCAAAGACCTGAGCTTCTATTGCAATACGCAGGAATCAC
>SEQ ID NO: 49 
GTCTCATTAACCAACCCAATGAGCAGGGTTTGTAACGTGATCGACAGCAACAAGTGAGCGGACGCAACTAA
TGTCTCATTAACCAACCCAATGAGCAGGGTTTGTAACAACATGTACATTATATGGGTGATAATAGCGACCG
ATGACGTAATTAAATATTACAGTCCTAACAGGTTGGTGAAAAACTATATTTGAGACGCTAATTTTGCTCAA
TCGAACCAGCATTTAGTAGGAAATCGGCTTCATCGTCACTAAATATAGAGTCATTCAAAGCCAAAAAAGAG
TTTTTCACCAACCTGCTAAGTGTAATATTACAGCGAATGGTGTCTTATTGGCGTTTTATGCCTAACACACA
CATTAGCATTAACCATAGTCGCTATATGTGCATCACTTTTCGGGATTCCGATCTATGTAATTGCCAATGCG
TCTTTCGTTGGTCGATGAATCTTATTGCCTTCCTACAGCGACTTTACTGTACTGAAAGGAGCACGGGGATT
TGTCTCATTAACCAGCCCAATGAGCAGGGTTTGAACTTTAAACATTCAGATTTCGCTAATTGCGGGGCTAC
 T
>SEQ ID NO: 50 
GTTAGGATGCCCGCAGGATGTCATATAGTTGGCAAGGTGATCGACAGCAACAAGTGAGCGGACGCAACTAA
TGTTAGGATGCCCGCAGGATGTCATATAGTTGGCAAGATACAATTAATTTGTGGGTGTACTAGACCTACTC
CTAGTGGTGAATCGGTGCTGACAACACCATCACGCGACTAGGTTTAATATTTTAATTTTACTGTATAAAGT
ACCAAAAAACTATTATTAATTGTGGTAAAATATTAAAGTCAGCCCACGAAGGAATACGGGAATATTCCGTG
TGGTTTCACTGACTTATCTTTCTCCTGAAATAGCAGGTTACTCAATCCATGTCACCAGAGAGTAAGCTGTG
TATGACTTTCATTGCTAGTTTCCGAGGGTCGCTGTTTTTTGGCATTTTTTCTTTTTGTTTTAATTGGTAAA
TCGTCTGTAACTTGGTCATGGCAAGTAACCGAAGTTCCCGGCTTGCCAACTCGGAGGATGTAATGTAGTTG
GGGGAGAATATTCATCTCCCGGTGCTATTTCCGATAAATCGGTCTTAGCTTCCTGATGACATTCCGTATTG
GAATCAGAGTCAGGCTT
>SEQ ID NO: 51 
GTTTCAATCCCATTGCTAGGATTCATTAATAAGAAACGTGATCGACAGCAACAAGTGAGCGGACGCAACTA
ATGTTTCAATCCCATTGCTAGGATTCATTAATAAGAAACAGCTTGATCTACTAAACCATGTTTTTCCTTCA
AAAGCAGTGATCTTAATCACATGAACGTCTTATTTTTTTAGCAGTTAATAATCATTTTCTTTTAGCGTTTT
GATAGCTCGTGAGGTATACCTTATACCCAATCCTGTTTAGATAAACTGATTTACATTCTATCGGTTCCCTG
TTTCCTCTTATTGCGATCGCGCCAAGCCCATCCCCATAAGGATTGTGAGCTTAAATCTGACGGGGCTCAAA
AAAATGAGTGATTGGGGACATCAATGTTAGTGATAACCCAGAGGTGAAACAGCTACAACTCTTTAATTATC
AAGGTTCTGGCGATCTGACGGGAGGCCAGGGATTTTGACCCCGCTTCGAGCCGTCAGTTTTAGTTAAGTTC
TAGCGATAGTTAAGTCCTAGCGAAGATCCCCTAAAAAGGAGGGATTAGCCTGTAATCTCAGGCTGTGCCTT
GCGGCTTCTTGCTGACAGCACACTATGGCACTCAAAGGCAAGCTTTTCCGGTCTTTCACCGACGTTCCCCC
TTGAAACTTGGGTACGGTGGCTCTCGCCAAAATTAGGGGATCTATTTTTATTCAGTTTTCAAGGTTCAAAA
TCTCTTGATTTAATAATAGCAAAAATGGAAGCATGGGGAAATCATTTTCTCCCTGCTTCTTGTAGGGAGAT
TCATTAGGAGTTTTCGCCTTCCCGTCTTTGTTGACAAGAAACAAAGATGATTTTTAATTGTCGGGGGTTAC
TTGTTCT
>SEQ ID NO: 52 
GTTTCAATCCCTAATAAGGCTTAAGCTTAATTGCTTCGTGATCGACAGCAACAAGTGAGCGGACGCAACTA
ATGTTTCAATCCCTAATAAGGCTTAAGCTTAATTGCTTCATTAAACCTAATTGAACTTATACTAGAGCTAG
GCACTCTTTCAATTTGGTATTTATCTGGTTTCTTTACATTCTTTTTTGCTTTCATGGTTTCGACCTCACAT
AACCACTAACACACTTTATATTAACTTAAAAATAAAGCACCCATCTTCCCGAAACCCTTGATGTAAAAGGA
TTTCGGGTAAGGATTGCGCCCCATCATTAAAAATTCAGTTTTCAAGGTTCTGCTCGGTTCAAAAAATTGTT
TCAGCGATCGCCGATCGCGTTGGTTGGTCTAGGTACGCGTACCGACGATCGTCGTGAATAAGTGCGGCGTT
GCTGAACGGGGGGATGATAGCCGAAAGATGCGAAACGGGAAAAAAGAGATCGAATCGATCGCGAAAAATGA
ATTTCAGGCGTTGCATAATAGAGATATGAATGGAGGAAATCAAAATGCAATGCCCTGAATGTAAATGGTTC
TTCCGAACTTACACTGTAGAAAATTCCTCAAGCTCCCTCTCTGCCAAGCAATGCTCTAAAGTTGATAAATA
TCAATCTAACAAAAACTCAAAGCCTTAGTATACAAGCTTTACAAGCACGAGTTGTTGAGAGTTTT
>SEQ ID NO: 53 
GGTGATTATCATCTTGAAATGAAAGGTATATCCAACGTGATCGACAGCAACAAGTGAGCGGACGCAGGTGA
TTATCATCTTGAAATGAAAGGTATATCCAACTAGTTATAATTGTAAAAAGCAAAAGTCCCACCCTGGTTCG
CTGTGCAAGTGGGAAGCGTGGTGGGAACTGTCACCGCAAAGGTATGTATATTTTTTGAAACTGCAAAACTT
TTTGCGAATAAACTTTTGTAAAGTACCCTGTTTTGAACGAAGATTACACATTGTTAGTGAAAATTTGGCTC
GTTTAACCATCAACTTCTTCAGAAGGTTTAGTGTTGCTGTCAGTTGATTTACTGTTGATTCTCCTGTAGAG
CCTTCAGACTGTTACACTTCCAAAGATACAGATCCTTGTGTCAAGCAGAGTGTAGTAGGCCATTTGTCGGT
TATTTTCAGTTGCAGTTTGGGCAGTTTTGCTGCAAAAACACTTTTTATCTAAAAAATCTGTTCATAAATAG
CTTATTCTCATTTATTTTTTTGTATTTTTGCAGAGCATTCGCAATAAATGCGTTTGTGAATAATAATTTCG
ATATGAAAATAGTTGAATTAAATATTCAAATGGGGTTGTTGGAGACCCTTATGCTGAGAATTTATGTCACT
CGGTAAATACCTTAATTAATTATATAACAGGCAATTGTTATTAATAGGGTGATTATCATCTTGAAATGAAA
GGTATATCCAACGTGATCGACAGCAACAAGTGAGCGGACGCAGGTGATTATCATTTTGAAATGAAAGGTAT
ATTTAACCGCACGTAAGACCGCATCATCGGTGGCTATCAACTTGAACTGAAAGGTATATCCAACTTGGGGG
TAACGCTGACAATGGTGCTGCGTTGGTGGTTATCATCCCAAATAAGAGTATTCTATGCAAATTCATAAAAG
TACAATTAATTTAATGTATGAAGAAAGCCATTTTGTTAATAATGATGTTGGTGTCGTTTTGTTGCAATATT
TCAGCCCAAACGCATCTAACTTTTATGGGCATTCCTATTACAGGTACAAGAAAAGCTATGGTGACAAAGCT
AAAGGCTAAAGGATTTACTGAGAAAATACAAGAAAATGATGTTGGTGTTGAATTGAAAGGAAAGTATCTTG
GAAAGGATGTCAGACTGTGTGTTTATAATAGCAATATAACTAAGACAGTATATCTCATAGATATATTTTTC
AAAAAAGGTTATTTTATGGACTTGTACGAACAATATCTTGACTTTCGAGAAAAGTTCAATGGTATATACGG
TAAGCCAACAGAAGTTCTTGACGAATCTTTTGAAGAAACATTAGAGTTTGGGAAACTCCCTATGTGTGCAA
 
CATATTATGAGAGTTCCAAAGGCTTGATTTCTTTGGAGATTTCAGAAGATAAGCATTGTGTGGAAATATCT
TTCCAAGATAAGCGTAATGCAGAAATTATGAGAAAAGAAAAACCTCAAAAATAAAAGT
>SEQ ID NO: 54 
TCGGTCACCCCCTGCGCGCCCGGTCCGCACGTGATCGACAGCAACAAGTGAGCGGACGCAACCGGTCACCC
CCTGCGCGCCCGGTCCGCACCAGACCGGCCGAGGCCAGGGCCTTGCTCGCGTGCTCTCCGCTGGCAAGCGA
GCCGGGCCGCTCCGGGGAGTTGCCGCCGCAGAGGACGCCGATTCGTAAGGTCTCGATGTCGGGCAGGAGCG
TCGTGGTCACCTGGGTCTCCTTCTCGGATGCGGTGGTCCGTGCGCCGGGCTTGGTCTCGTGCGAGCGGGGC
GGGGCGGGGTGCTGCGTGGCTAGGCCGCCGGTGAGGTCATGCACGTCGGCCGTCACGACTCCTCCCGCTTC
AGCTTGAAGAGGCGCTCCTCGGCCCTGGTCGTCCGAGTTTCGCGTCGCGTCTCGGCCGGGAGCGGCTTTTT
GACGTTCCGGAGTTCGGCCTGATTCCGGAGGCCATCAGGGGTCACGGGCTTCCCCTCGGACGCCTGTTTGG
CGTCCGCGCGCAGGGCGCGGATCGTGGGGCGGATCTTCTTCTTGCGTGCCATCGTGGTCTCTCTGTTCGGG
GTGCCCGCCCGCCCCTGGATGGCCGGGCGGTGAGGCCCGCCCCGGCTGCGGGAGCGCTGGGCAGCCGGGGC
GGGAGTCGGGGGTCAGTCGGCGAAGGCGTCAGTCCCGTGGAACGCCTCCGTGGCGAGCTTGACGAAGCGGC
GGTCGTGGCGGTCGCGGGCGTTCATCGCCCGGTAGCTTCCGGCCCGCTCCCAGAAGGCGCGGCCGTACTTG
GTCTGCATGAACTCGTGGAGGGACGCCTTGAGAGAGGACTCCGGGGTGTAGCCCACGCGCAGCATGAGGCT
CCAGGCGGTCATCCACCGGTTCGCCAGGACGTGCTGCACGGCCTCGTCCTCCGGGGTGTCCTCCCAGACGG
CGGGCACTGACTACGACCTCTTCACCAAGAGTGTCCGCGCCGGGCAGAGCAACCTTCTGCCGTAACCTCCG
CCCCCCAGGGGCGTGATGAGGGGGCCCCTGGCTACGCCGGCGGGCCTTCTCACCACGATGTGGGCCACTAG
CGGCAGCCGCCGCACCCACACCTCAGGAACAGCGATGTCGAGCGGTACCGCTCCACCTTTGACCGCTGACG
CTCCATCGCTCTATCTTCGCGAGAACCCGTGGACCTGATCGAGCGTCACACTCACGGTCCCTGAGAGCGAT
TAGGAGGCTCTCCTGATGGTGCGGCACGAACTTCCAGTCGAAGCGTGGAAAAAGTCGACGTACAGCGGCGA
CACTCACGGGCAGTGCATCGAGATCCAGGCGATGCCGGAGGGCAGCGTCGCGGTCGGCGACTCCAAGGACC
GCAGCAGGGGGGCCTTCGTCTTCCCGGCGGCGGCGTGGGCGAACTTCGTAACCGAAGCAAAAAACGCCCCG
CTCACCTGACTGTTCAACCCACGTACCCCCTGCCCCGAACTGCTGCGGGCGGGGGGTTTGTGCTTCTGGTG
TTCTGTGTCGCTGGCTCGGTGACTATCCGGCGTGAATGTTCAGTTTTGAATTGTTGTCGATGCCGGGCCGG
CCGGACTTCCAGGCCGCAGCCGTCACCTCATGACTTCTCGGGCCGGACCGTAGGACCAGGGTCGCCATGTG
GATGATCGTCGGTCACCCCTGCGCGCCCGGGCCGCACGTGATCGACAGCAACAAGTGAGCGGACGCAATCG
GTCACCCCCTGCGCGCCCGGTCCGCACGTGATCGACAGCAACAAGTGAGCGGACGCAACCGGTCACCCCCT
GCGCGCCCGGTCCGCACACTTCCTGACCTGCGGCGTTAGAACAGCTTTGCCTGTCCTTGACGCGGGTTGGG
GGGTGTCGGCGGATCGCTCCGCCGAGGTGGCACCGGCCCGGGAGGGCCGGTGTCTTCCCGCTTCGTCGAGC
CCTGCTTCTCCGCCCGGGGGCGGGGGAGTCTGACGGGTTCTCCACGGGCGTTTTGGGTCTCCCCCGCTGGG
TGGCCGTGGGCGCCAGGGTGAACTTGAACGCGCGCAGCACGACTGTCTTGGTGCTTGCCACCGAGACCCAC
CTTCCTCCGCCCGCACGGCACGTCCTCGTCCACGACACCCACGTGGTGCCGACGTGTTGGAGCATCTAGGG
CATGGCACTGACAACCGGGCCCGGGCGGATTGAGCGTGCAGGTGGGATGCCCGCTTCGCGTCGTCCCTGGC
GATGCAACGAGGCTGTTCAAGCGCTGGCAACGTCAATCCGGCCGTCCAGCTAACCCGTTGGAGCGGAACCT
GCCCAGGAGGGCGGAGCGCAGGGCGAAGCGGTGGCAGGGTCGCGACGTGAAGATGATCAAGCGCTTTCTGG
TGGCGGGTGCGGCCCTTGCGCTCCTCGCACCCGCGTCCGCGTCAGCTACTCCCTCCGCTGCCGCCGAGGGA
GTCAACTGGCGCATCAAGTGCCAGGCCAAGG
>SEQ ID NO: 55 
GTTGGATATACCATTCAAAACAATGTGATCGACAGCAACAAGTGAGCGGACGCAACTAATGGTAGCGTTGG
ATATACCATTCAAAACAATACTGACCTCCTTTTTTATTAGTGGGGACTGTAATGACAAAATTACAATTTTA
TTTGAAATGGCAAAGCTTTCTTGCAGATTATTACATACGTTAGTCGTTTCTTGTTACTAACGTATGCAATG
ATTGATTACTAACGTATGTGATAAGCAACCACATGCGTTAGCAATAATAGCCCTTATAAGTATATTACCTT
ATAACTAAAATCAGTAAAAAAAGACACGATTATCAACCCCCATGCGCACATCATCTCAGACGGCTTTATAT
TTTGATATACCATTCAAAACAATAATAAAACCAACATTCGGCCACATAGCCGTCTGTATTCTTCTGTTGGA
TATACCATAATAAAACCAACTCTGTGCCGGTTGTGCTGGGTGTAGGACTTGTTGGATATACCATTCAAAAC
AATGTTGGATATACCATTCAAAACAATGATAAAACCAACCGTTAGTTTCTAAGTTTATTAATTTCAATGTT
TTGACGTACTTCCCGAGTATAGTTTCCTATCTCAGGTTAAGGACTTCCAGCAGCCCCAATTAGAAAATTTC
TTTTTGTAATAATAGGCATACTCTTTTTAATTTTACAACCGCAAATATTGTAATTATAATTGTAAGTCTCA
AATCCAACTCAAATAATTTTCAGAATTTCACAAATTCTCAAAATCGCCCATAAAAAATCCCCCATGCAACC
GCATGAGGGATTCCACAAGTAAATTCTATTTCGAGAAATTTATTTTGCTACA
>SEQ ID NO: 56 
AGTTTTAATCCACGCACCCACAAGAGATACGACGTGATCGACAGCAACAAGTGAGCGGACGCAACTAATTT
TAATCCACGTACCTATTTGAGGTGCGACATTCGGATGACATGGCTGGTGCAGTATATGACTTCTGATTTTT
TTCCATTCTTGTTTTATGTTCCTCTGCTGTTGGTACTAATCGTAAATGTCTACAGTCTTTTTCATTTTACT
ATCTCCTCTATTTTGTAACTTTTTATGATTCATAACCCCGCCTACAAACTTGAAAACAACACAAAAAGAGC
TTCTTCAAGTCGATTGAAAATGTGCATAGCAAAATAAGCTATATACACTAATCGGCTCAAAGAAGCCCTTT
 
CACAATTGAAACAAGACGAAACTTGCTTCTACAACTCAACCACAACACAAAAAGGTGTAGGAATAAACGG
AATCAAATAAACTAACAATATACCTTTATATTACATAATAACATTATAGGTTTCAATAAGTATTAATTAAA
ATTAACGCATTAGCCAAAAGAATTCTTATTCCTCAAGCTTGTGTAGGACGAAAGTCCAAAGGTAAGTGAGA
GTTGAATTTTGGGTAATGCGTAAACTCATAATAAATTGCTATCAAGGGTCTTACCAGTTTTAATCCACGCA
CCCACAAGAGATACGACGTGATCGACAGCAACAAGTGAGCGGACGCAACTAATTTTAATCCACGTACCTAT
TTGAGGTGCGACCCCGATTTGTGTTCAAAATCAACCACAAATACGTTAATTCTATTCAAAAGTATAAAAAT
ATTTTTATTTAGTAAAGTAATTTTTATAAAAATTATAAATTCACCCCATAAAGTATAAGAATTCTAGGTGC
GAATGTACTAGGAATTTCATGTTTGCTATACATTCGTACCTAAAAAATTAAAGAGATTTCTAAATCAATTT
ATTCTTTTACACCAATATGTTCGACCCTATTTTCATAGCTATTACCTAACTGATTAACAAAAATGTATAGT
TTCATAATACCCTTTGTATATTGATTCATAATTTACAATGAAATTCACTATCTATTGTCTCTCTATCTACC
TATCAAGCTTAACTTTCGTATGATCATTTAATCTTTTTTACCAGATAACATTCTTGAAAGCATTGAATACG
AAATGCAATTTCTTTCGATAACTTACGAATACTTTTATAATTCTTATCTTTATACGTGTTCATCCTTTTTA
GAGTAATAGTATCAGTATGTCCAAGTTTTTTAGATCTTTTACACCAGAACCCTCTACTTCATTCCATAAGT
AACAGGTGCTTCTTCTGACTACCCATATTTTTCACTTTTATAATTAATTTTTAACTCTTCACCTATTACTA
CTCTGTGTTTCTTATATTTGTTTACTATCCTCTATACTATTAATTTCTTCCATCTTCTTATTCCACTATTT
TTATTACCGTTTTCCGACATCGTTATCTATCCTTTCTCTATTATTTATGCGTAAAAAGCAGGAGTTAATTG
CTAGATTCAATATTATTACCACGCTTTGTACAAAAATAGATTACTAAAAAGAAATAAAGATAAAATGCAGA
TTATGTCGAAATTAGTTAAAGAATATTGAAATTCAACATAGAATCTTATACTGAATTATCATTTTATTTAA
TAACGAAGTCGTTCGATACTTGGTTTAAAGCCTCACGGCCTGCCAAGCGAGTTACTGCAGCAATAGAGGCT
ATGCACACATACAAAAAAGGATAGATTCTTCCAAAAAGGAAGTATGTCAAGATCCAAAAAATTCATTCTTG
AACTACCCCCACTTTCACTTCGTTTAGAAGTGAGGGATTCCTAAGTAAAGAGTTCTATCGAACTCTAATTA
ATTAGGCTAACCCCGCAGTCCTTTGCTTGTGCTTGATTTGGATAGATACGAAACTTATAGGCTTTATTAAT
CATTATTTGATTCACCTCCATTTTGGTATATGACTATTATACACCAAACGTACATTCGATAGGTAGTGGAG
TAACACTTTCTGTATGTCGAACAATTAAGATGGCTTCCTGCCATCCCTTGTTCGAAGCCAATTCATCTCCC
ACCTTATTCACTGGGCTGTGACCCTTCACGCTCCTTGAGGAAGGAGTCTTCTTGGCTAAAATGATAAAATC
GTATATAGAATGTAGTAATTAAAAAGATCATAAAGTTATTCATTATCTCTCTTCGTCTTAATCTCTAATAT
ACGCATATAAATGAGACTTCCTTTGACAAATAAAGATAAAATTTATTTTATCTTTATCTTATCTTTATCTC
TTTATGTTAGTCTGTTTTCCTAGAAGAAATAATCCTAATATTAAAAAGAAACAGAGGAATAATGCATGAAG
AGTAAAAAAATGTTTGTATT
>SEQ ID NO: 57 
GACGCCTGGCTCGACACCCCCACCGAACACTGGCCGCTGACCCTGCCGCCGGCCACGACAGACCCGCCCCC
GAGGTCCTAGACCGCGAGACGAGAGGACGAGTTGTCGGGCCGCCGCCGCAGCGGTGATCGGTGAGCCCGAC
AGGGAGGCGTAAGCGGCCGCGCGGTCGGCGGTGATCCGCGCGGTGTCATACCGCAAGCAGCTTCGACAGCA
CCTGCGATCCAGGCTGTAGCACGCGGAGAGACGCGCGTAGTCCATCTCGGAACGGGTCCGGGCCGCCGCCC
GGGCGCGCTGGGAGACGGTGATCGACAGCAACAAGTGAGCGGACGCAACTACCCGTCGCCCGTGTGCGCTG
GGAGACGGTGATCGACAGCAACAAGTGAGCGGACGCAACTACCCGTCGCCCGTGTGCGCTGGGAGACGCCC
CTTCCCGAGCTGGTGTCTTCTCCCGACCGCTGTGAAGTTGTGGGGTGCACGGACGCCCGGACCTTACGTTC
CGGGGGTCCCCGTCTGACCTCGGCCGGGTGGCCGGGAGCGGGGTTCTCGTTTCGCCGAGAACTGCTCAGCT
TCGGCGCCCTCTCCGACGACATCGGCCCCTCCACATTCCGGCACCGTAAGGCTACCAACGAAACCGACCCC
GCCCGCACAGTGCCCGAAGCAGCCATGCGGCCGATGCCGGGCCGTCCCCGGGCCGCCGGGCAGGAGCACGC
GGGGGCCGAGGACACCGCGCGCCGGCCGGCTGCCGGGGCGCAGAGCCGGGGCGCATGCCCGCGCGAGCCGC
GCGGCGGGACCGCCACCCGGATCGCCGAAGCCGCCGGCCTCGGCACCGGTCCGAGCGGCGCAGGCTCCGGC
GGCCAGGTGCTTCGCTGGGGCGGCCGACGGGCGGCCGCCCCAGCGTGACGTCAGGTACGCGGCTTGAGCCT
GA
>SEQ ID NO: 58 
TCTACACTAGTAGAAATTCTGAATGAGTTTTAGACGTGATCGACAGCAACAAGTGAGCGGACTCTACACTA
GTAGAAATTCTGAATGAGTTTTAGACCCAGGTCCCGGACCGTAACTTTAATCCGAATGATTCCGATGTGGA
AGACAGCCTTTCGGCCTGTTTGTGTTTGGCGACTTAAACGAATCGAAAGGCCGCTTTTATTCTATCGCCAG
CAGACCGTATCGTGCAGGGCGTCAGCCCCTGCACCATATCAAAAATTCACTTGATAACTGTCACACTCGGG
TCTTAATCAATCTACACTAGTAGAAATTCTGAATGAGTTTTAGACGTGATCGACAGCAACAAGTGAGCGGA
CTCTACACTAGTAGAAATTCTGAATGAGTTTTAGACTTACCTAACATAAACTGTATGTAAGTAATGGTATA
TAACACCTCTTACCGTACCCTAGCCAGCATATGAAAAATTGATCAGAAAACAGGATTGCGATTACTTAGCA
CGCGCAGAATTTGGGTTTTAGACTGGACACGGTCCAACACGGTCCAACCATTTAGACCCGATAGTTCGTAC
AAGGCCCATTTTAATCGGTTTAACGCGTTAGATCAATGTTATATATCATAAATAAACGTTTTTTTCATCAT
ATATATACAGATTCGGACATCGTGCTGAAAAGAAGACGGAGGTTTGTCCATCCATGCTAAGTAATCCGCCT
GTGTGCATATATAATTTTGTATACCAAATGTTTTATTTATTTTTATAACATAATATTGTTATTAATGTAAT
TTTGCAATTATATATAAGAACGCACATGCAGCCAACATGAAGTCCGGCGGACGCAGGAAC
>SEQ ID NO: 59 
GACGCACACCGCACGCGGGTATGGGCTGAGAGGGTGATCGACAGCAACAAGTGAGCGGACGCAACTGACGC
ACACCGCACGCGGGTATGGGCTGAGAGGTTGGACAAGGCGCTGGACGTGACCGACCTGCACTTTGCGTTGG
ATCCCGTGCCTGCGCCCGATGCGCCCTGCGCCCGCGTGCTGGACACGACGTATTTCACGCTTGATCTGCTG
AAGGTGGATGGCGAAGCGGCGGTGCCCGCGGTGCAGGACTTCGGCATTCTGACGGCGCTGGACGCTGTGGA
GCTGGCATGGCAGGGCGGAGCTCGCCGTCTGGCGGCGGGCGAGACCGTGCTGGTACCGGCCAGTGCACCGG
AGATGACGGTGAAGGGGCAGGGAAGAGTGGCGCTGTCTATGCCGGCGGGGAAGTAAAAAATAAGTTCATCA
TAAGCGAAGGCAGGGTGTACATCCTGCCTTCGTATTTTATTGTTAGATTGAAGATTTTGCTATACGCTGGG
GCTGATTATGTGATATACTAACAAAAACAATAGCGATTGCACATGTGGAGGAGATAGCCTGGTTTCGCGTC
ATCGGCAAGGGTGACTCTACACCCTAAAATCGAGAGAGTGCGCAAAAAGCGCGGAATCATCGCTTGCGAAT
ACACTCGATAAGGTAGAAATTGTGCATATGGATTCCATGCACACAGCTCATTTTAGTAGCTTTTGCCGACG
CACACCGCACGCGGGTATGGGCTGAGAGGGTGATCGACAGCAACAAGTGAGCGGACGCAACTGACGCACAC
CGCACGCGGGTATGGGCTGAGAGGTCAGCGGATACTGCGCCGTGCCTGTGCCAATGGTGACGCACACCGCA
TGCGACAACACCTCTGCAAAAAAGTTTCCATGAACCCTTGACAACGAGCATTCTATCGTCTATAATATCAT
CTGTTTGAAGCAGAAGCGTGCCCGCTTCTCGCCCTGTGACTTGCGTTGCTGGGCTGATGGCAAAAAGGTTT
TCGTGTGAAAATCTGACTACGTCATGACACAGCGGGTGAGGCTTCTCCCCGCTGTTTTTTCATGGAAAGCG
GCGGTCAGACAATTTTGAGGAGGTGC
>SEQ ID NO: 60 
CCTTTATATCCCACTACGTTCATATAAAACTGTGATCGACAGCAACAAGTGAGCGGACGCAACTAATCCTT
TATATCCCACTACGTTCATATAAAACTTGTTCTTTTGTTGATAGTAGTTTAATTTTAACTGTTATTGTTTG
AGACATTGTTCTACACCTGTATCTCTAATCATTTTTGCTCTTTTTATACTTCTATACTCTTCTAAAGTTGC
TTCTCTTACAATAGACCAAGAACCAAGCCACATCATTTCTTTTATACACCCGTTAGGTATTTTAATTTGTG
ATGTATCACACTCATTCCTGCCACATAGAACTAATATGGCTCTTTCGTAGTCGGCTCTATATTCTTTATCA
AATTTATGGTCTTTTACAAACTGTTCAAAATCACAGTTACTACATTTCACTTGCATATCTTTTACCCCCTT
GTTTTTTGCTGTTCAACATACCGTTTAATCGTTTCTCTTGATACGTTTCCTGCGGTAGAAACGAAATAGGA
ACGTGTCCATAGGCTTGGCAAGTGTCGAAGATGAGGAAATTCCTCTCTCAGCTTTTTCGAAGTCACTCCTT
TGATTTTTGCCATGATATCGGAAGGACTAAATGTTGGTAGTGCATTTAGGAACATATGTGTATGGTCTTTA
TCACATTCTAAAGCAATAATGACAATTTCCAATTCTTCGCATATTTCCTTTACTAACTGTTTAAAACGTTC
CTCTACATCGGTACGAAGAAATATTTTCCTTCTGTATCGAGGGCAAAAAACGAAGTGATAATTGATTAAAG
ATACGGTTGTATGAGTTCTTCTATATTCGTTCATACTCATATCGTATCAGTTTAATCTACATACAGCAACG
AAAATACAAAAATATTTGGAACATTTGACGTGGCATAAAGCCACGCCTGTTCCAACCTCACTCTCATCCCA
CCCCTAAAGGAGTGGGCTTTCTCGCTCAGAAAATCTGTAAAGAAAGGTATCTATCATATCCAACATGTGAA
TTCGTATCACAGTCGCCTCAAAAATTGGATGAATGGATTTAATGGTGTGGCTACACGATACTTGGATAACT
ATTTATTTTAGTTCCGTTTCTTAGAGATAAATAAGGAAATTGGAGAAAAAATGACCCCAAAAGCAATGCTT
ATAGCATCATGTCAAAAGCCAAATTTTACGAAAGTAGGTTCTTTTCGGCACGTTAGTTTACATCTAACTTA
AATTTCTGACGACTACCTCCTTCTGAAATAACACCCTTTCGTTTACAATATTCACCACTTAGTCATACGAC
TATCTTAAACCGATGCAATCATACCATTTTTAGGATAATCCCACTACTGTCATCTATAAAGCAACAATTTT
TTAGAAAAAAGCGAAAGAAAAAGATCCTTTATATCCCACTACGTTCATATAAAACTGTGATCGACAGCAAC
AAGTGAGCGGACGCAACTAATCCTTTATATCCCACTACGTTCATATAAAACTCCCTAAAAACAAAACGGAC
GAAAACCTTCATCTATCAAGGATTTCATCGTTCAAAGAACTCAGCAAAAGTCGGCTTTTTGCAGTAAACCA
GGAATTCTATTTTTCACATAGGAGAAAAAACGCCTCAAATCCTTGCTATATAAGGCCTCATGTCCAGTATA
CCCAAAAGTAGGTTTACTGCAAATCATGAACGTATTCCGCCACGAACACCGAATATGAAGAATGATATCTA
AGTAACTT
>SEQ ID NO: 61 
ACTAACGTATGTAATGATTGATTACTAACGTATGTGATAAGCAACTACATGCGTTAGCAATAATAGCCCTT
ATAAGTATATTAAATTATAACCAAAATCAGTAAAAAAGACACGATTATCAACACCCATGCGTACATCATCG
CAGATGACTTTATAGTTGGATATACCATACAAAACAATAATAAAACCAACGTGATCGACAGCAACAAGTGA
GCGGACGCAACTAAATATACCATTCAAAACAATGATAAAACCAACGTGATCGACAGCAACAAGTGAGCGGA
CGCAACTAAATATACCATTCAAAACAATGATAAAACCAACATTTAGTTTCTAAGTTTATTAATTTCAATGT
TTTGACGTACTTCCCGAGTATAGTTTCCTATCTCAGGTTAAGGACTTCCAGCAGCCCCAATTAGAAAATTT
CTTTTGTGTAATAATAGGCATACTCTTTTTAATTTTACCACCGCAAATATTGTAATTATAATTGTAAGTCT
CAAATCCAACTCAAATAATTTCCTATAAAATCTATCGGCAAGTTTATATAATAGAATATCATGGCAAAGAT
ACATTTTTTGCCGATATCGGCAATATTTCAATCAAATAAAGTCAGGCTATTATCCACCTGT
>SEQ ID NO: 62 
GAAGCACTTCACAGAAGTGCATGGAGCAGAAGGTGATCGACAGCAACAAGTGAGCGGACGCAACTAGAAGC
ACTTCACAGAAGTGCATGGAGCAGAAGCGCACTCCGTCTCTGCCCTGTCAAGGCAGCTGTTTTGCAAGTTC
GATTATACCTTTGGGGTAAATGGTTGTCAATGGCGGGCCCGCGACAGAAAATAGGAAGCCTTTCTCTGCAC
 
ATTCAACAAACAACATTTACAGAATGCCCCAATTATGTTATACTAATGTTGCCTTGCAATAATGTGAACGT
GAAATGGAGGGATGTCTGTGGCAGATATCAAATCTCAAAGTGGTGGGAGGTCTGTCCCCACCATGGGGTGC
GAACCTTGTGTGCTCATCATTGCCGTGAGCGTTCGCACGTCCAAACGACCATATCATCGTTGCCCTGCGAC
CATTCAGCGGCAATCAAGACGCAGGCATGATATGTAACCATGCATTTCTGTGAACTGCTTCCAAAACGCAC
TGCTTCATTATAATCCTCCCTGTGCAGTTCTGCCGAAGCACTTCACAGAAGTGCATGGAGCAGAAGGTGAT
CGACAGCAACAAGTGAGCGGACGCAACTAGAAGCACTTCACAGAAGTGCATGGAGCAGAAGAAGTCGTTTA
TAGCGGTTTGTTTACAGGGAGTTAGAAGCACATTTCAATTTTTCAGTAAAACTCCTCCAGCTTTGCAGCTG
GGGGAGTTTTTTACTCCCCTCTTGACAATTATCTTCTACGTCTGTAGAATATAATCAAACTACAACCGTAG
AAGATATTCACAGAAGGGAGGATAACGCTATG
>SEQ ID NO: 63 
CTTCTGCTCCACGCACTCGTGCGGAATGCTTCGTGATCGACAGCAACAAGTGAGCGGACGCAACTAATCTT
CTGCTCCACGCACTCGTGCGGAATGCTTCGGGGTCCGTAAGCGAGACTGATACCATGAGAAAAACGAGCGC
CGTGGGGAGCCACGGCGCTCGTTCTTTTTTGTTGGGGGTTGAGAAAGGACCGCCGGTATCCCGGCGGTCCT
GCTTCGTTTACAGAATGGAGCGAGGATATGGCACCTCTCTCGTGCGGAATGCTTCCTTGCGGCTGCCAGGC
AGATGCGCATCGTCGTAGGTCTTCTGCTCCACGCACTCGTGCGGAATGCTTCGTGATCGACAGCAACAAGT
GAGCGGACGCAACTAATCTTCTGCTCCACGCACTCGTGCGGAATGCTTCGGCAAGATCCTACGATCTCATG
GCTGCGAGAGGACGACGCGGAAGCAGTCTGCACGAATGCGTGGTCCCATATCATACCCGCGCGCGGATCGC
CCACTCGAAATGGCGCGCAAGATAGCGATGATATGTCTGTTCGGAGGTGCGAACACCCGCGGCGACCGGCG
GGCGATCAAGGTTCGCACTCCTCGGTGGGGACAGACCTCCCACCGATGTTCTCATTTCCTTGTTTCGTTCT
GTTCGTTTCGTTCGATGTAGCTCCTCACCTCCCATGCTACATCATCCGACGCTTGGTCCTTTTCGGTCCCA
TCATACCACGCTGCGCCAGAGCTGTAAACACCGAGACCGTCCTAAGATGCAGAAAGGACCGCCGGGATGTC
GGCGGTCCTTCTCTTTATCTCGTCCT
>SEQ ID NO: 64 
CTTTCAATTCACGACCTCCCGAGGAGGTCGACGTGATCGACAGCAACAAGTGAGCGGACGCAACTACTTTC
AATTCACGACCTCCCGAGGAGGTCGACTTTTTTCGATTGTCAATTTCTTTTGGCAAATGACTATATTTCAT
TTTTAATCAGTGCAATTTTTTCCAATTTCTCGGATTCAGTGTATCGCGGAATTTTAATTTCTTATTATATT
TTTTCTCGTAATCCTTTTTGTTTAATCTGTATACACAATACCACAGAAGTAGCACGATTATTAGCCCTGAC
ATAGTATTTACCCCTTTTCAACTCACAACTTCTTAATAAAAATCACGCTGCTGCAAACCTTATTGCAGAAT
TTCTACTCACACGTCCCTTACGGGACGTGACATCTTCCGATTTAATAGAGACATTGTGGAATGTAATTTCT
ACTCACACGTCCCTCGCGGGACGTGACGTCGGATGATTTGAATATAGAAACAAAGAGTAATCCATTTCAAT
TCCAGACCTTCCGAGAAGGTCGACAACATGCCGCTCATATCCGTCACCTGACTGGTATCCTTTCAATTCAC
GACCTCCCGAGGAGGTCGACGTGATCGACAGCAACAAGTGAGCGGACGCAACTAATCTTTCAATTCACGAC
CTCCCGAGGAGGTCGACTGCAAAATTCCACAGTTTTCATCTCTAAAACCTTGAAATTTTTTAATACCTTTA
CTAATTTCTCTTACTCCTTCTTGCTTTAATACCACTCATTTTCCCTTTTCCTAACTAAAACCTCTCCATTT
TCTGGTGCGAATGCCGAAGACTTTTCTGTGTGCTTAGGATTCGCGCTCCACTTGAAACATGTTCTGACACG
G
>SEQ ID NO: 65 
GTCCGGTCCGTGCGCGTGGGGAGCGACCGTGATCGACAGCAACAAGTGAGCGGACGCAACTGTCCGGTCCG
TACGCGTGGGGAGCGACGAACGGATCCACAGCGGGCCCTTGATGACCCGACAGAAAGGCACCTTTCGTTGA
GAAACAGGCTCTCCCGCCCCCCTGCGGCTCAAGGCCCGAGAACCGGCCTTCGCTACTGGCTCGCCCGCCGC
CGCCGGGCCATGGCCGCACATGCACTGCGCGGCGCCTGCTACGGCATCGGCACCGGCGCAGTCGGCCTCGT
ATTCTGGTGGATCGAGCAGACCCTGTAAACGGGCTCCCGCACCCAAGGGCAGGGCGGGCGGCGCGGGTGAT
CTGGTGGTGAAGAAGCAGGTCAGCGGCCGAACGGGGACCGTTGTCAGTGGTGGCCTCTACAGTCGTCAACG
ACTCTTGGCGCGCGGGTGGTTGAAGGAGGTGGCTGATGGCGGAGACGGCGGTGCTGCGTGCGACCAGCAAA
CGGCAAAGCGGCCCTGAAACCGCAGGTGGCGAAGTGTCCGGTCCGTGCGCGTGGGGAGCGACCGTGATCGA
CAGCAACAAGTGAGCGGACGCAACTGTCCGGTCCGTACGCGTGGGGAGCGACGAGTTGCTGTTGTGCCTGC
CCGCATGCCGTCTTGGTCTAGGCGATCTCCCAGCGGTTGCTGTCGTGGGTCTGTTCGT
>SEQ ID NO: 66 
GGTCGGCATTTACACGAAAACCAATGCAGTTACAACAGTGATCGACAGCAACAAGTGAGCGGACGGGTCGG
CATTTACACGAAAACCAATGCAGTTACAACACGAACGGAGCGTTCGCGTCGGTTTTGATTCCGCTCCGGTT
TTCGCTCCGCGTGGAATCACGTTCCGTTCTCGGTTCCGTATTATTCGGAAATTCCGCCCTATCTGACAGGG
GCGGAATTTTTTTGCGCGCGTGCTTGCCGGCTGCGCGAATCATGTCGGTTAGGTTGAGCGAATCATGCCGG
TTATTGTGTAAGGTTTTTGCGGCAAACGTATTGTTTGCCGCAAAAGAGAAAGTCCCTAATGTCCTTAAAGT
CATTAAGGACTTTAATGTCGTTAATATTGTCGTGCGGCGAATATTTGTTCACCGCTTTTAAAACCTTTAAA
GACCCTAATGTCCTTAAAGACTTTAAAGTCCCTAATGCCGTTAAGGACTTTAATCCCCATTCTCCCTCCCC
GCCTAATTCTCCGCTTCCCTGTCGCGGCGGTCGAAGACATCGACGGTGAGGTCGGCATTTACACGAAAACC
 
AATGCAGTTACAACAGTGATCGACAGCAACAAGTGAGCGGACGGTTGGATATTCACTTGAAAATCAATGTG
GTTACAACATGAAAACTGTAACATGATGATATCCAATTTATATTATAAAAAATCAAGTGAACTTTTCGAGT
GCAGGGACATCCGTAAAATATTCATTATGCGGATTATGGTGTCCTCACCTCAAACCAAGGCGCTCCAACGC
GCCCATCCCGAAAGGGATTATATCGTTACATATATTTATCGTATAAAACAATAAGTATCATTAGTTCAAGT
GCAGGATTCCGAAAAATTCGGATTTTATTTTAAAGACCGACACAACTGTTGCAAAGATAGTATATTTAGTG
GGTTGTTGCGGTTAGTTTTCTTCTCTGTCGCGGCGGTCGAAGGCATCGACGATATGCTTGACAAGTTCGTG
GCGGACGATGTCGTGCTTGTCGAACTCGACGAAGCCGATGCCCTCGATGCCGCGCACCGTCTGCATCGCGC
GCACCAGTCCGCTGTCGCTGCGCCGCGGCAGGTCTACCTGCGTTACGTCGCCCGTAACGATGAACTTCGCG
TTGCGCCCCATACGCGTCAGGAACATCTTTATCTGCGACATCGTCGTGTTCTGCGCCTCGTCGAGAATCAC
GAACGCGTTGTCGAGCGTCCGTCCGCGCATGTAGG
>SEQ ID NO: 67 
TTAAATATAACCTATGTTAATCATTAACGTGATCGACAGCAACAAGTGAGCGGACGCAACTAATTTAAATA
TAACCTATGTTAATCATTAACGTACTCATTTTAAATCCCCCTCATTTTAATCTTACATTAAAAATTTTACC
TCTTTAACTGTTTTCTGTACACAAATTCGACAAATTTAGAATTAAACATTTATTATTAGACAATTATTTTT
AATAAAAAATGAGCCTAGAAGTAACTAAACTCCTAGGCTCATTTCGTGCAATATTCTTATTATATTTATAA
TTTATTTTAACATAATTCTATTATTTATTAAACTACATTAACCATTAAATTTACATTCATATTCAACATAA
TCTATGTTAATCATTAACAAAGGAGGTTGCTTATGTTTAATAACAATATTTCATTTAAATATAACCTATGT
TAATCATTAACGTGATCGACAGCAACAAGTGAGCGGACGCAACTAATTTAAATATAACCTATGTTAATCAT
TAACCTTAGTAACTCAGCCATTTCCTCTAATAATTATTAATTTTTCTAATTGGTTATATCATAGTACCCAC
CATGCTTATAGCATTTTTTATACTAGCTTTTTACCAGCAAATTTCAACTGGAGCGGTTGTAAGAGTGTTAA
CATGCTTCCTTTCAAGTAATTTCAATGTTTTTAAGCGTGTTACTTCTCTACTTAGGATGGTAAATCACCCT
TTATTAAAATCATTTACTCCCTCATTTCAAAGTTTTATTTTTAAAGTTTTATAAATAAAAAAAGAAGACCT
AGAGATTCCCCCTAGGTCTATTATGTGCAACATTTACTTATATTATTATAAAAATTATATTTTAAA
>SEQ ID NO: 68 
GACGAACCTGGGCATCACCACCGGCGACCTAGACGGTGATGGCCATCCGGAGCTGATCGGGGCGGGCTATA
CCGACTCCCGCGCGCAGGGATGAACCCTGTGATCGACAGCAACAAGTGAGCGGACGCAACCCGACTCCCGC
GCGCGCAGGGATGACCCAAAAGCACGCGGGCCGTAAAGATCAGCGCAAAGCCGACTCCCGCGCGCGCAGGG
ATGACCCGTGATCGACAGCAACAAGTGAGCGGACGCAACTCCGACTCCCGCGCGCGCAGGGATGACCCGCT
GCGCAAGGTGTGCACACCAGCGGTCGGCGCGCGGGGCTCTCCCTGCAAAGCACGGATTCCGGGGCCGATCG
CTCCAATGCCGCAACCGTCTACGTGCAGGCGGCGGCACCACCCGCGATCGTATCTCCTCCGGCGTCACTTC
GCCCCGTTGAATGTATGCGCATTGATTTCATGGTGCCAGGGCGCGACTTTATCGCGTTCCTCACGCCAGTA
GTAGAGGATGCGCTTGCCGATTCCTCCCTTTCCCTGCTTGAAAAACTCAAATCCCTTTTCTCGCACCAGAC
GTAGCGCCCAGTTGTAGATGAAGCGCGCCGCGCCGAAATGCTGATGCAGGAAGCGCCTTTTTCTCTCAGAA
GCAATTTCGAGCTCAAGACGGTAGGCGCGCAGCATACTTGCTCCGGTGCGTATATGCTTGTGACAGGAATA
TATCTTGTAATTGGCTACATGTCAACGACATATGTCCGGAGGCTCATCTTCAGAAAGTGTGTGTTGGAGTG
TATCATAGAAGACCCAGGACGCATTCGAGTACTCGGAGTAGAAGGCCATGGAGTTTCTCATCCTGGATTTG
ATGGACATGGAAGAAAGCCGGCGGTGGATTGCGCAGCACTTCCACCCCGAAGGCCTTCGCTGTCCTCGCTG
TCAGGCCTTGGTCACCGAGGCCCGTCTGTTTCGCCATACCAAGCGGAGTCAACTGCCAGTCTACCGGTGCA
AACGGTGTGGGCGAACCTACAACCTGTACACCGGCACGGTGTTCGAATTTGACCCCTCAGCAGGTGGTGTT
GCTGCTCCGGGGGATGGCCAAAGGGGAAAGCACCCAGGTGCTGGCGGCCGAACTGGGGTTGAGTTATGGCA
CGGTGTTGAGCTTACGCCATGCGCTTCAAGCCCGAGCGGCGGGGCTGCAACCGGAGAGGCCGCTGCCGGAT
GCGGAAGTGGAGGCCGACGAGATGTTCCAGAATGCGGGGGAAAAAGGCGCGCCGCATCGGCATCCCGACGA
CCCGCCCCGGCGGCGGGGAACCCG
>SEQ ID NO: 69 
ATTTAAATAACATCATAGTGCTATTAAACCGTGATCGACAGCAACAAGTGAGCGGACGCAACTAAATTTAA
ATAACATCATAGTGCTATTAAACCCTTTTAACTAGTTAGTAGTTTTTAGATAGTAGTTTTTAGTTATGATT
GAAAGATTAAATTAGTAACAAGTTACAAGTAATAAGTGACAAGTTTAGGTTGAAACTCCTTTATTAGAAAC
TCCTTTAGGAGTTTCTAATAATATATTTTCAAAAATTCAGCATAAGCTGAATTTTTCAGACTGTTGACAAA
GTGTCAGCAGTCTGATAATTATTATTTAATTAAATTAATATTTTAAATTCATATAATCCCTATATCTCTTA
TTATATCAAGGTTAAACTGATAAATTTTAGTTTAATACTACTTAAATAATGGGAGTTTTATTTCCTATAAC
TTATACTATTTTAAATAAAACTCTTATTATTTATTGCTACTACGTCTTAAGTTAATAGTCTCATTAATAAT
AAAATTATATCCATTATTAAACATTTAAATCAAAAAAATAGAAAACATTAAGTGATTCCTAAGAATCGTTT
AATGTTTTCTATTTTTACATTTATTCAATAATTATTATAATTCTCATCCTATATATAATATTTTTATAATT
TACAGTATCACAATTTCATTAATTTAAATAACATCATAGTGCTATTAAACCGTGATCGACAGCAACAAGTG
AGCGGACGCAACTAAATTAACATTCCATTATGGCGCTATTAAACCCCGTTAACTTTAAAGCCACTATATAT
CTAGTTTGATATAGTAGTTTCTGTCTACCTCTTATTTTATAATAAATTTTATCAATACTAATTACCAACTT
TAATTATAAAATAGCTACCCTGTACTTATATTACTTATTTTGTCTACCACCTGTGTTTTTACCCTATCTAT
 
GATAGACAATAAACTAATATAATTATTCAATTTTGGAACGCCTAACGGTCACACATTGTTAAGATTAACCC
TTACCGAGTGATTATGGTATATAGTTGGTCAAAACCTTATATATTTAATCCATCTCCTCAGCCTTCGGCAC
TTTAAGTTACCGTAACTGCTATATATTTATATATAACATCAATACAATTATATTACATTATTTTGAATTTG
TAAATAAAGATTAGTTTTATCAATGTATTAATTTAACCTTCCTTACTGTTATCATTTCTATATATCCTCAT
AAATTATTTTTTATTACAATTATACCATTTTTACATAATTATATCTATAATTAATAGTTTCTATACATTAT
TTTTTACTACATTATAATTTTATTGCTAAAACGTATTAATATAAATGTATATCTATAAAGAAAAAAGGCTA
TAAATTTATAGCCAAATGAATAAAAAATAAATTTAAGTAAATAATATAAATGTTAGTTTAAGAAAGCTTCT
TAGATATTTGTTTAACTGGATGCTTACCAGTAGATCTAAGAGCTTTTTTGCTTTTTTTATAATATGAATTG
TTTCTTTTATTTTTTTTCCTTTTTCACATTGTATTCAAATTTATATATGTAATAATGAATTACAATTGTCA
GTATTACTACAATAAGGAAGTATATGCTCCATTTCAAAAAAGCAAAAAAAGTTTATCTCCTAAATTCATCT
TTATCTCACCTTCTTTCTTAAGTTAATTTAGAGTTTTTGCTCATTTAATTTTCCCCCTGAAATTTATAGTG
TCAATTCTACAACGCCCTTCCCCAGCTACGCTGTGTTGTTTTTTGACACTGCCTAAAAATCACATGGAGAT
ATACTTGTCAAAAATTAAAGCATCTTTAAAGGAGTTGGTTTTATTCAGTTTACACAAAATTTTTTACAGGG
CCCTTCAATAAAAGAGATGGCCCTAAGCCATCTTTTTTCACATTTATTACAATTAAAACATTTAAAACATT
CAAAAATCCGATATTCTTGAAAATCTTCCATTCCACCTTAATGCACATGTACCCCTTTCACCATGCCTATT
CTTAATAATTACAGCATTAATACATTCATGATTTTCATCAGCATTTTTAGATTCATACTTTAACTTGCCAA
GCCTAAACAGCTTGTC
>SEQ ID NO: 70 
GATGCATTTTATATATATAAGTGGATTGAAATCGTGATCGACAGCAACAAGTGAGCGGACGCAACTGATGC
ATTCTACATATATAAGTGGATTGAAATACCAAACAGAAAAAATACAAGCCAAGAATATTAAAAGAATTATT
TGAATAAAAGGAGAAATGTAAAATGAAAACATTTTTATTTATAGCAGGTTTTTGCTTAGAAGTTGCATGTT
TTACCACTATTATAAATCTGTACGACTGGAAAGCTTTTGCATTATCAATAGGAGCTGCTCTTGGTGCATCT
ATAGTTGGTATAATGAGGAATAAATTGGATAGATAATTTAAAAGGAGAGAAAATGACAATGTTAATATATG
GTTGCAGGTTCAACGCCGCCAAGTGCGAATATAAAGTACACATAAATTACATGGCAATTCGCACTAAAGTA
GATAAAAGAAATTTGATAGTAGTATATATACTATGATTACTATCACTATATATACGAAACAAAATATTGCT
TTTATAACCATAACATTATGTAAATTATACAAAATATGATAGTCACATTTATGTATTTTTGCTGATGTAAT
TTACATATATGTGTGGATTGAAATCAAGGGGGTGCTAAAATGGAATATCTCTTTTTTAGAGGTAATTTATA
TATATGTGCGGATTGAAATCAGAAGTTCCACCAGTATTTTTTCTGGCTGCGAGGTAATTTGCATATATGTG
AGGATTGAAATAAGTTTTAGTACACTAAAGCGTTTAAAACAATAGAGATGTAATTTACATACATGCGTGTA
TTGAAATTACCTGTCTAGGGCGGTAGCCTATAAATTATAGATAGAACTTACATAGGTAAGATTATTATATC
TCAGACAAGTAAAATAACATGACTAATATTATAAATATTATAAATATTATATATAAAATATAGGTAGGAGG
TTCAACCCCACCTTGGTGCGATTAGACAGTGAACATATATTTCTTAATAGATTCGCACCAGAAGTAGATAA
AAGAAATCAGTTAGTACGATAGTTAATCATAGCAATATGTTAATTATTCGAAAACAGATAATGCTTTTATA
ACCACAAATATGTATAAAATGCACAATGATAATAGATAAGTCTTATATATATTTGCTGATGCATTTTATAT
ATATAAGTGGATTGAAATCGTGATCGACAGCAACAAGTGAGCGGACGCAACTGATGCATTCTACATATATA
AGTGGATTGAAATATTTCACAATATACACAAAAATAAAAGGAGTATAATAAAAATGACAATA
>SEQ ID NO: 71 
AAAAATCGTGAAAGATAATTAGAAAAGAAATTTTCTAATTGGGGCTGCTGGAAGTCCTTAACCTGAGATAG
GAAACTATACTCGGGAAGTACGTCAAAACATTGAAATTAATAAACTTAGAAACTAACGGTTGGTTTTATTA
TAGTTTTGAATGGTATATCCAACAGTGATCGACAGCAACAAGTGAGCGGACGCGTTGGTTTTATTATTGTT
TTGAATGGTATATCCAACAGTGATCGACAGCAACAAGTGAGCGGACGCGTTGGTTTTATTATTGTTTTGAA
TGGTATATCCAACAGTGATCGACAGCAACAAGTGAGCGGACGCAATTGGTTTTATTATTGCTTTTGAATAG
TATATCCAACTATAAAGTCGTCTGCGATGATGTGCGCATGGGTGTTGATAATCGTGTCTTTTTTTTACTGA
TTTTAGTTATAAGTTAATATACTTATAAGGGATATTATTGCTAACGCATGTAGCTGTTTATCACATACGTT
AGTAATCAATCATTGCATACGTTAGTAACAAGAAACGACTAACGTATGTAATAATCTGCAAGAAAACTTTG
CCATTTCAAATAAAATTGTAATTTTGTCATTACAGTCCCCACTAATAAAAAAGGAGGTCAGTAATGCTGTT
CATGGTGAAATGGCTGGAACGTGTAGCAAAATAAATTTCTCGAAATAGAATTTACTTGTGGAATCCCTCAT
GCGGTTGCATGGGGGATTTTTTATGGGCGATTTTGAGAATTTTTGAAATTCTGAAAATTATTTGAGTTGGA
TTTGAGACTTACAATTATAATTACAATATTTGCGGTTGTAAAATTAAAAAGAGTATGCCTATTATTACACG
TAAGATAGAATTGAAAATCGTAAAAGATGGATTGACAGATGAAGAATATGACCAACAATGGAAATATCTTT
ACCAGATAAACAACACC
>SEQ ID NO: 72 
GTTGCAATTCGTATGCGCAGGTAAGTTTCGAGGTGATCGACAGCAACAAGTGAGCGGACGCAACTAAGTTG
CAATTCGTATGCGCAGGTAAGTTTCGAGTACTGATTTTTAATCAAAGTGTAGACAGTGTAGACAAAGAATC
TGCGCCAAGATATATAAACCGGTTTTTAACATAAACCTGTTTTCAACAGCTACGAGCTTTTGCTTGTTTTT
GTACACTTTGAGTTTAAAAATAGTATAAAAGCTGATTCTATGTGCATAATAGCTATAGTAAGATACTGAAA
TTCAACTTTTAACTGTATAAAATGACAAAAAGCAAAATTTCTCGAAAATATTTTAAAAAAGCTATTGACAC
 
ATAGCCCCTTTAATGCTATAATGATTAAGTCGCACAGAGAGAATAACAAAAAAGCTGCTGAAGCTTTGCGG
CAATTGTAAATGGCGGCATGGCTCAGTTGGCTAGAGCAACCGGTTCATACCCGGTAGGTCGTAAGTTCAAG
TCTTACTGCCGCCACCATAATAAGGCCCGTTGGTCAAGAGGTTAAGACGACGCCCTTTCACGGCGTAATCA
TGAGTTCAATTCTCGTACGGGTCACCAAAATCCGAACACTTTCAGTGTTCGGATTTTTTTGTTTAATTATT
AAATTATAATTTGATTCAACATCGTTCATTAAAATATTATTGGTAAAAAAACACAAAAGTAAAATAGGTGT
TTGTTTATATTGTCAATTGAATCTATAAAAATAAAATGATAAAATAATTATATTATTTTTTTACGCAAAAT
CATTGTGGCAAAAAGCCAAAAGTTTTGATTGTCTACTTATAAGGCGAGGAAAATGATATTGGTGCGAATAT
AGAGTACACATAATTTATTAGAATATTCGCACTCCCAAAAGGGGCGGCTCGGCGTCCCAAAATCGAGAAAG
TATATGTAAATCTGAATTTATTCAGTAGTAGATACACTCGATAAGGTATAAACAATACACATTTTAATCCG
TGTATTGAACTTATTTAGCTATTTTGCAGTTGCAATTCGTATGCGCAGGTAAGTTTCGAGGTGATCGACAG
CAACAAGTGAGCGGACGCAACTAAGTTGCAATTCGTATGCGCAGGTAAGTTTCGAGTTTTAATAACAGATA
TTTAAACTCCTGTACAACCCAAAAGGTGTTCGGGAGTTTTTAATTATATTC
>SEQ ID NO: 73 
ATTTGATTAGAACCATATTGGAATGTAAATGTGATCGACAGCAACAAGTGAGCGGACGCAACTAATATTTG
ATTAGAACCATATTGGAATGTAAATATAAAGAAAATAATGGCTGTTGCATATTTTGCAGCAGCTTTTTTTC
GTCTAAGTTAAAATAATATCCCTCTTGCTCTCCAGACTTCGACGCCTCGTCCATTAAACTAACACATTTAA
ATGTTACGAGGCGTCTATAGATTGTCGAATTGAGAGGGGTATTTATTTTAAAAATAACCAAAGAATAATCT
CTGCAAAATATTGCATACAGCCATTATTTAATAAGTAGATAATTATTTTATATAGTAGAAAAAATTCTGTT
TAAAATATATAATGAATATAAGGAAATAAAAATCTGCGAAACATTTAATATTAAAAATTGTGTTTGATATA
TAAAATTATAATGTGTATATAAATTTGTGGGGAGTAAATTATAATTATCTACTTATTAAATAATGGCTGTA
TGCAATATTTTGCAGAGATTATTCTTTGGTTATTTTTAAAATAAATACCCCTCTCAATTCGACAATCTATA
GACGCCTCGTAACATTTAAATGTGTTAGTTTAATGGACGAGGCGTCGAAGTCTGGAGAGCAAGAGGGATAT
TATTTTAACTTAGACGAAAAAAAGCTGCTGCAATATTTATATAATTTTTAAATAATAATGGTTCTATAGGC
GATTTAGCGTCTAAAGGTTGAGGGATAAGACAAAATGGTTAAGGTTTCGACCAACTAACTACTTATCCACT
CGATAAACGGTAAAAACTCATACTAATATTCTTTATAGATAACGTGGTATGACATTCCAAGAATTATATAA
ATTGTCGACCTTAGATAATGTAAAATACCTAGGAGATAGACAAAATGAGATATTGCAATAGTTGGATCTGT
TTTTTATCAAGAAGGACTATTTTTTACTTTAGAAAATATATGTTTATTTGTAGGTAGACAAATAGTATATC
TGTATATGTTGCAATTGCTAGGTTTAATATATAACGTATTTGATTAGAACCATATTGGAATGTAAATGTGA
TCGACAGCAACAAGTGAGCGGACGCAACTAATATTTGATTAGAACCATATTGGAATGTAAATTATGTATTG
CATATTGTTGACCAAGGTCCTATTGAATCTGACTAGAACTATTTTATGACAATTATGCAAATTAATATTTT
TTAGCCAAGAAAACCAACATACGAAAGTTTTCTTGGCTATTATTTTAAATA
>SEQ ID NO: 74 
TTTGAGAATGATGTAAAAACGTATGGTACTCAGTGATCGACAGCAACAAGTGAGCGGACGCAACTATTTGA
GAATGATGTAAAAACGTATGGTACTCAGTTTGAAAAATAATAGTTGACGTTGGTTATTCAAAGGGGTATAT
TATAATTAGTTTTGTACCATACAAAATTACATCATAAGTTCAAATAACGATTTATCGTAAACGCCGCGTGT
GCGGCTCACAGTGTGTGAAAAAGTAACTGCTATTCGGAATCAGAGTGGCAGTTTCTTTTTACATAAATATA
AACAGAGATGAAGAATGTGGGAAAATAAAATTTGCTATTGGTAAAAGATATAAAGGAGGAAACTTTAAGTA
ACTGAATAAGTTGTCAGATGTGATAATATGTGTTTAAATATACTCATGGAGGTATACTATGAATACATCAA
ATATCACAAATTACAAGCCAAAAGATTTTGCTGAATTGCTAGGTGTTTCTGTCAAAACGTTACAGCGTTGG
GATAGAGAAGGAACTCTAAAAGCAAATCGTACTCCAACTGATAGGCGTTAGTACACTTACGACCAATATCT
TCAATTCAAAGGCATAAATACAGAAGATGATACGCGTCAAGTTGTCATTTATGCCAGAGTCTCTACAAGAA
ATCAAAAAGATGATTTACAAAAACAAGTCACATTTCTACGACAGTTTTGTAATGCTAAAGGTATTATCGTA
GACCAATGTATTGAAGATTATGGGAGCGGCCTTAATTACAATCGCAAGAAATGGAACGAATTATTAGATGA
AGTAATGGAACAGAAAATCAAAACCATTATCGTAACTCATAAGGATAGATTTATCCGATTTGGCTATGATT
GGTTTGCAAAATTCTGTATGAAGTTCAATACAACCATAGTGGTAGTAAACAATGAAGAACTATCGCCTCAA
GAAGAACTCGTGCAGGATATTGTTTCAATACTCCATGTATTCTCTTGTAGGTTATATGGACTTCGTAAATA
TAAAAAACAAATAGAAAGGGTCAAACGCAAACGTAAGTATGTACCGAAGGCTTATTCGGGAATTAACGACT
GTGGAGTGTACAAGAACCTGCAAGTAGATATGATTTCGGTCAATCAAAAGCATACACGATGAAGCAGTAAG
TAGAGTTCGTGAGAACCTATATTATCTCGATGTGCGTATATTTACACACATTTTGAGTGGCAGAATGTAAA
ACGTTTGAGTACCATGCCCGGTGCGGATGAGGCACTGCATTTTTGAACTTTGAGGTTTGAGAATGATGTAA
AAACGTATGGTACTCAGTGATCGACAGCAACAAGTGAGCGGACGCAACTATTTGAGAATGATGTAAAAACG
TATGGTACTCAAACATCTGTATGTCAGATCTGGCTTGTGGCTCAGTTTGAGAATGATGAAAAACGTATGCT
AAACTGAAGCGTACCAAAAAGTGTTATCCGCGTTTGAGAATGATGTATAATTTGATTTCTTAAAAAACTTC
TATGTCGCCATCCAATAGGTGGCGAATTTCTTATACTCAATATATCGTATATCGCATTTAGCGTTTTTAAT
TAATGTAATTAAATTGGAGAATGTGGTTTTGAGAATGATGTAATATTGTAAAAATATTTGTTGTGGGACAT
TATGAGAATGTCTGCGCAATATTGAGTTTATAGTCGATATTATTTGAGTTCAATAAAAGGGAGTTTTTATG
>SEQ ID NO: 75 sgRNA-pre scaffold (underlined is the reprogrammable region) 
GCCUUGCAAUAAUGUGAACGUGAAAUGGAGGGAUGUCUGUGGCAGAUAUCAAAUCUCAAAGUGGUGGGAGG
UCUGUCCCCACCAUGGGGUGCGAACCUUGUGUGCUCAUCAUUGCCGUGAGCGUUCGCACGUCCAAACGACC
AUAUCAUCGUUGCCCUGCGACCAUUCAGCGGCAAUCAAGACGCAGGCAUGAUAUGUAACCAUGCAUUGGAA
AGUGCAUGGAGCAGAAG
>SEQ ID NO: 76 sgRNA-opt scaffold(underlined is the reprogrammable region) 
GGUGGGAGGUCUGUCCCCACCAUGGGGUGCGAACCUUGUGUGCUCAUCAUUGCCGUGAGCGUUCGCACGUC
CAAACGACCAUAUCAUCGUUGCCCUGCGACCAUUCAGCGGCAAUCAAGACGCAGGCAUGAUAUGUAACCAU
GCAUUGGAAAGUGCAUGGAGCAGAAG
>SEQ ID NO: 77 882-TnpB-reRNA scaffold 
GGUGAACAUUCGCUACGCAACUGUGGUUGGCGGUAAUAGUCAAUCAGCCUAAGAACCUAUAUGGUCUGUCU
UAGGAGGGGUGAUGGCACACCCUCAUCUUGAAGGCUGUUCAAAACAGAAAUGGACUGCGAACGUUUAGUCA
UUCAAGAAUCCCAUCCGUUACACCAUAAGGUGUAGGACUUGCGGCUUUAGCCGUGGGAGUGUCAAAAAACA
CAGUAGGAGGGACUUACGUUAG
>SEQ ID NO: 78 966-TnpB-reRNA scaffold 
GGUUCUGCAUGGUCAGAAUCGGAAUGGGUCAAAGAGCAGCUACAAGCCCAUGACUUCAGUCGUGGGUAGUU
GACAAAACACAGUAGGAGGGACUUACGUUAG
>SEQ ID NO: 79 GFP-T1 GTGATCGACAGCAACAAGTGAGCG
>SEQ ID NO: 80 TraC-5M-7 
MSVSFSLNAKKIRLENYAMKMRLYPSPTQAEQMDKMFLALRLAYNMTFHEVFQQNPAVCGDPDEDGNVWPS
YKKMANKTWRKALIDQNPAIAEAPAAAITTNNGLFLSNGQKAWKTGMHNLPANKADRKDFRFYSLRKPRRS
FAVQIRPRCIIPSDTNQKVARIKLPKIDGAIKARGFNRKIWFGPDGKHTYEEALAAHELSNNLTVRVSKDT
CGDYFICITFSQGKVKGDKPTWEFYQEVRVSPIPEPIGLDVGIKDIAILNTGTKYENKQFKRDRAATLKKM
SRQLSRRWGPANSAFRDYNKNIRAENRALERAQQDPGSSGVGPEAPVLKSVAQPSRRYLTIQKNRAKLERK
IARRRDTYYHQVTREVAGKSSLLAVETLRVKNMLQNHRLAFALSDAAMSDFISKLKYKARRIQVPLVAIGT
FQPSSQTCSVCGSINPAVKNLSIRVWTCPNCGTRHNRDINAAKNILAIAQNMLEKKVPFADEALPDEKPPA
APVKKAARKPRDAVFPDHPDLVIRFSKELTQLNDPRYVIVNKATNQIVDNAQGAGYRSAAKAKNCYKAKLA
WSSKTNK
>SEQ ID NO: 81 TraC-B22 
MSVSFSLNAKKIRLENYAMKMRLYPSPTQAEQMDKMFLALRLAYNMTFHEVFQQNPAVCGDPDEDGNVWPS
YKKMANKTWRKALIDQNPAIAEAPAAAITTNNGLFLSNGQKAWKTGMHNLPANKADRKDFRFYSLRKPRRS
FAVQIRPRCIIPSDTNQKVARIKLPKIDGAIKARGFNRKIWFGPDGKHTYEEALAAHELSNNLTVRVSKDT
CGDYFICITFSQGKVKGDKPTWEFYQEVRVSPIPEPIGLDVGIKDIAILNTGTKYENKQFKRDRAATLKKI
SRQLSRRWGPANSAFRDYNKNIRAENRALERAQQDPGSSGVGPEAPVLKSVAQPSRRYLTIQKNRAKLERK
IARRRDTYYHQVTREVAGKSSLLAVETLRVKNMLQNHRLAFALSDAAMSDFISKLKYKARRIQVPLVSIGT
FQPSSQTCSVCGSINPAVKNLSIRVWTCPNCGTRHNRDINAAKNILAIAQNMLEKKVPFADEALPDEKPPA
APVKKAARKPRDAVFPDHPDLVIRFSKELTQLNDPRYVIVNKATNQIVDNAQGAGYRSAAKAKNCYKAKLA
WSSKTNK
>SEQ ID NO: 82 TraC-B24 
MSVSFSLNAKKIRLENYAMKMRLYPSPTQAEQMDKMFLALRLAYNMTFHEVFQQNPAVCGDPDEDGNVWPS
YKKMANKTWRKALIDQNPAIAEAPAAAITTNNGLFLSNGQKAWKTGMHNLPANKADRKDFRFYSLRKPRRS
FAVQIRPRCIIPSDTNQKVARIKLPKIDGAIKARGFNRKIWFGPDGKHTYEEALAAHELSNNLTVRVSKDT
CGDYFICITFSQGKVKGDKPTWEFYQEVRVSPIPEPIGLDVGIKDIAILNTGTKYENKQFKRDRAATLKKM
SRQLSRRWGPANSAFRDYNKNIRAENRALERAQQDPGSSGVGPEAPVLKSVAQPSRRYLTIQKNRAKLERK
IARRRDTYYHQVTREVAGKSSLLAVETLRVKNMLQNHRLAFALSDAAMSDFISKLKYKARRFQVPLVAIGT
FQPSSQTCSVCGSINPAVKNLSIRVWTCPNCGTRHNRDINAAKNILAIAQNMLEKKVPFADEALPDEKPPA
APVKKAARKPRDRVFPDHPDLVIRFSKELTQLNDPRYVIVNKATNQIVDNAQGAGYRSAAKAKNCYKAKLA
WSSKTNK
>SEQ ID NO: 83 TraC-B26 
MSVSFSLNAKKIRLENYAMKMRLYPSPTQAEQMDKMFLALRLAYNMTFHEVFQQNPAVCGDPDEDGNVWPS
YKKMANKTWRKALIDQNPAIAEAPAAAITTNNGLFLSNGQKAWKTGMHNLPANKADRKDFRFYSLRKPRRS
 
FAVQIRPRCIIPSDTNQKVARIKLPKIDGAIKARGFNRKIWFGPDGKHTYEEALAAHELSNNLTVRVSKDT
CGDYFICITFSQGKVKGDKPTWEFYQEVRVSGIPEPIGLDVGIKDIAILNTGTKYENKQFKRDRAATLKKM
SRQLSRRWGPANSAFRDYNKNIRAENRALERAQQDPGSSGVGPEAPVLKSVAQPSRRYLTIQKNRAKLERK
IARRRDTYYHQVTREVAGKSSLLAVETLRVKNMLQNHRLAFALSDAAMSDFISKLKYKARRIQVPLVAIGR
FQPSSQTCSVCGSINPAVKNLSIRVWTCPNCGTRHNRDINAAKNILAIAQNMLEKKVPFADEALPDEKPPA
APVKKAARKPRDAVFPDHPDLVIRFSKELTQLNDPRYVIVNKATNQIVDNAQGAGYRSAAKAKNCYKAKLA
WSSKTNK
>SEQ ID NO: 84 TraC-B32 
MSVSFSLNAKKIRLENYAMKMRLYPSPTQAEQMDKMFLALRLAYNMTFHEVFQQNPAVCGDPDEDGNVWPS
YKKMANKTWRKALIDQNPAIAEAPAAAITTNNGLFLSNGQKAWKTGMHNLPANKADRKDFRFYSLRKPRRS
FAVQIRPRCIIPSDTNQKVARIKLPKIDGAIKARGFNRKIWFGPDGKHTYEEALAAHELSNNLTVRVSKDT
CGDYFICITFSQGKVKGDKPTWEFYQEVRVSPIPEPIGLDVGIKDIAILNTGTKYENKQFKRDRAATLKKM
SRQLSRRWGPANSAFRDYNKNIRAENRALERAQQDPGSSGVGPEAPVLKSVAQPSRRYLTIQKNRAKLERK
IARRRDTYYHQVTREVAGKSSLLAVETLRVKNMLQNHRLAFALSDAAMSDFISKLKYKARRFQVPLVAIGT
FQPSSQTCSVCGSINPAVKNLSIRVWTCPNCGTRHNRDINAAKNILAIAQNMLEKKVPFADEALPDEKPPA
APVKKAARKPRDAVFPDHPDLVIRFSKELTQLNDPRYVIVNKATNQIVDNAQGAGYRSAAKAKNCYKAKLA
WSSKTNK
>SEQ ID NO: 85 TraC-B34 
MSVSFSLNAKKIRLENYAMKMRLYPSPTQAEQMDKMFLALRLAYNMTFHEVFQQNPAVCGDPDEDGNVWPS
YKKMANKTWRKALIDQNPAIAEAPAAAITTNNGLFLSNGQKAWKTGMHNLPANKADRKDFRFYSLRKPRRS
FAVQIRPRCIIPSDTNQKVARIKLPKIDGAIKARGFNRKIWFGPDGKHTYEEALAAHELSNNLTVRVSKDT
CGDYFICITFSQGKVKGDKPTWEFYQEVRVSPIPEPIGLDVGIKDIAILNTGTKYENKQFKRDRAATLKKM
SRQLSRRWGPANSAFRDYNKNIRAENRALERAQQDPGSSGVGPEAPVLKSVAQPSRRYLTIQKNRAKLERK
IARRRDTYYHQVTREVAGKSSLLAVETLRVKNMLQNHRLAFALSDAAMSDFISKLKYKARRIQVPLVAIGT
FQPSSQTCSVCGSINPAVKNLSIRVWTCPNCGTRHNRDINAAKNVLAIAQNMLEKKVPFADEALPDEKPPA
APVKKAARKPRDAVFPDHPDLVIRFSKELTQLNDPRYVIVNKATNQIVDNAQGAGYRSAAKAKNCYKAKLA
WSSKTNK
>SEQ ID NO: 86 TraC-B35 
MSVSFSLNAKKIRLENYAMKMRLYPSPTQAEQMDKMFLALRLAYNMTFHEVFQQNPAVCGDPDEDGNVWPS
YKKMANKTWRKALIDQNPAIAEAPAAAITTNNGLFLSNGQKAWKTGMHNLPANKADRKDFRFYSLRKPRRS
FAVQIRPRCIIPSDTNQKVARIKLPKIDGAIKARGFNRKIWFGPDGKHTYEEALAAHELSNNLTVRVSKDT
CGDYFICITFSQGKVKGDKPTWEFYQEVRVSPIPEPIGLDVGIKDIAILNTGTKYENKQFKRDRAATLKKM
SRQLSRRWGPANSAFRDYNKNIRAENRALERAQQDPGSSGVGPEAPVLKSVAQPSRRYLTIQKNRAKLERK
IARRRDTYYHQVTREVAGKSSLLAVETLRVKNMLQNHRLAFALSDAAMSDFISKLKYKARRIQVPLVSIGT
FQPSSQTCSVCGSINPAVKNLSIRVWTCPNCGTRHNRDINAAKNILAIAQNMLEKKVPFADEGLPDEKPPA
APVKKAARKPRDAVFPDHPDLVIRFSKELTQLNDPRYVIVNKATNQIVDNAQGAGYRSAAKAKNCYKAKLA
WSSKTNK
>SEQ ID NO: 87 TraC-B36 
MSVSFSLNAKKIRLENYAMKMRLYPSPTQAEQMDKMFLALRLAYNMTFHEVFQQNPAVCGDPDEDGNVWPS
YKKMANKTWRKALIDQNPAIAEAPAAAITTNNGLFLSNGQKAWKTGMHNLPANKADRKDFRFYSLRKPRRS
FAVQIRPRCIIPSDTNQKVARIKLPKIDGAIKARGFNRKIWFGPDGKHTYEEALAAHELSNNLTVRVSKDT
CGDYFICITFSQGKVKGDKPTWEFYQEVRVSPIPEPIGLDVGIKDIAILNTGTKYENKQFKRDRAATLKKM
SRQLSRRWGPANSAFRDYNKNIRAENRALERAQQDPGSSGVGPEAPVLKSVAQPSRRYLTIQKNRAKLERK
IARRRDTYYHQVTREVAGKSSLLAVETLRVKNMLQNHRLAFALSDAAMSDFISKLKYKARRIQVPLVAIGT
FQPSSQTCSVCGSINPAVKNLSIRVWTCPNCGTRHNRDINAAKNILAIAQNMLEKKVPFADEALPDEKPGA
APVKKAARKPRDRVFPDHPDLVIRFSKELTQLNDPRYVIVNKATNQIVDNAQGAGYRSAAKAKNCYKAKLA
WSSKTNK
>SEQ ID NO: 88 VEGFA-T17 AAACACAGTAGGAGGGACTTACGTTAG
>SEQ ID NO: 89 VEGFA-T3 CGAGCGCCGAGTCGCCACTGCGGCCCC
>SEQ ID NO: 90 APEX-T1 CCAGGCTCAAAGTGATTTAGGGGTGGT
>SEQ ID NO: 91 HEK-T1 CTAGACAGGGGCTAGTATGTGCAGCTC
>SEQ ID NO: 92 CCDC127-T1 CTGTTGTTGTTTGTACTTCCTGATGCA
>SEQ ID NO: 93 OsAAT-T1 AGCACCAGGGGCTTCCCTTCCTACTCC
>SEQ ID NO: 94 OsALS-T1 CTCTGGGGTACAAAACTTTTGGTGAAG
>SEQ ID NO: 95 OsEPSPS-T1 AGCACCAGGGGCTTCCCTTCCTACTCC
>SEQ ID NO: 96 OsPDS-T1 TTGTACCTCAAGAGTGGAAAGAAATAT
>SEQ ID NO: 97 OsPDS-T2 AGAAACAGTGAACAACCCACTAAACCA

## Claims

1. An engineered clustered regularly interspaced short palindromic repeat (CRISPR) system comprising:
a) a TraC (intermediates between Transposon and CRISPR-Cas12) effector protein or one or more nucleotide sequences encoding such effector protein; and
b) one or more guide RNAs, or a nucleotide sequence encoding the one or more guide RNAs,
wherein the guide RNA is selected from i) a guide RNA derived from a transposon right-element (reRNA) and/or ii) a guide RNA comprising tracrRNA and/or crRNA, such as a single guide RNA (sgRNA) comprising tracrRNA and crRNA;
the TraC effector protein can form a CRISPR complex with the guide RNA;
the TraC effector protein can target and bind to a target DNA sequence under the guidance of the guide RNA derived from transposon right element (reRNA), and can also target and bind to a target DNA sequence under the guidance of the guide RNA comprising tracrRNA and/or crRNA.

2. The engineered CRISPR system of claim 1, wherein the tracrRNA contains a non-targeting strand binding sequence (NTB) which is complementary to the non-targeting strand (NTS).

3. An engineered regularly interspaced short palindromic repeat sequence CRISPR vector system comprising one or more constructs, comprising:
a) a first regulatory element operably linked to a nucleotide sequence encoding a TraC (intermediates between Transposon and CRISPR-Cas12) effector protein; and
b) a second regulatory element operably linked to one or more nucleotide sequences encoding one or more guide RNAs selected from i) a guide RNA derived from a transposon right-element (reRNA) and/or ii) a guide RNA comprising tracrRNA and/or crRNA, such as a single guide RNA (sgRNA) comprising tracrRNA and crRNA;
the TraC effector protein can form a CRISPR complex with the guide RNA;
the TraC effector protein can target and bind to a target DNA sequence under the guidance of the guide RNA derived from a transposon right element, and can also target and bind to a target DNA sequence under the guidance of the guide RNA comprising tracrRNA and/or crRNA.

4. The engineered CRISPR vector system according to claim 3, wherein the tracrRNA contains a non-targeting strand binding sequence (NTB) which is complementary to the non-targeting strand (NTS).

5. The system according to claim 2 or 4, wherein the guide RNA is a guide RNA comprising tracrRNA and crRNA, wherein the tracrRNA contains a non-targeting strand binding sequence (NTB) complementary to the non-targeting strand (NTS), wherein the guide RNA hybridizes to the targeting strand (TS) of the target DNA sequence via the crRNA, and hybridizes to the non-targeting strand (NTS) via the NTB.

6. The system of claim 4, wherein when transcribed, the one or more guide RNAs hybridize to the target DNA, and the guide RNA forms a complex with the TraC effector protein that causes the cleavage at distal end of the target DNA sequence.

7. The system according to any one of claims 1-6, wherein the target DNA sequence is within a cell, preferably within a eukaryotic cell.

8. The system of any one of claims 1-7, wherein the effector protein comprises one or more nuclear localization sequences (NLS), cytoplasmic localization sequences, chloroplast localization sequences or mitochondrial localization sequences.

9. The system according to any one of claims 1-8, wherein the nucleic acid sequence encoding the effector protein is codon-optimized for expression in eukaryotic cells.

10. The system according to any one of claims 1-9, wherein components a) and b) or their nucleotide sequences are constructed on the same or different vectors.

11. A method for modifying a target DNA sequence, the method comprising delivering the system according to any one of claims 1-10 to the target DNA sequence or into a cell containing the target DNA sequence.

12. A method of modifying a target DNA sequence, the method comprising delivering a composition of a TraC effector protein and one or more nucleic acid components to the target DNA sequence, wherein the effector protein can target and bind to the target DNA sequence under the guidance of the guide RNA derived from a transposon right element, and can also target and bind to the target DNA sequence under the guidance of the guide RNA comprising tracrRNA and/or crRNA; the effector protein and the one or more nucleic acid components form a CRISPR complex, and upon targeted binding of said complex to the target DNA sequence that is 3' of a prospacer adjacent motif (PAM), the effector protein induces modification of the target DNA sequence.

13. The method of claim 12, wherein the target gene is within a cell, preferably within a eukaryotic cell.

14. The method of claim 13, wherein the cell is an animal cell or a human cell.

15. The method of claim 13, wherein the cell is a plant cell.

16. The method of claim 12, wherein the effector protein comprises one or more nuclear localization sequences (NLS), cytoplasmic localization sequences, chloroplast localization sequences or mitochondrial localization sequences.

17. The method of claim 12, wherein the effector protein and nucleic acid component, or a construct expressing the effector protein and nucleic acid component, are contained in a delivery system.

18. The method of claim 17, wherein the delivery system comprises virus, virus-like particle, virosome, liposome, vesicle, exosome, liposomal nanoparticle (LNP), N-acetylgalactosamine ( GalNAc) or engineered bacteria.

19. A TraC (intermediates between Transposon and CRISPR-Cas12) effector protein or functional variant thereof for genome editing in an organism or an organism cell, wherein the TraC effector protein or a functional variant thereof can form a CRISPR complex with a guide RNA;
the guide RNA is selected from i) a guide RNA derived from a transposon right element (reRNA) and/or ii) a guide RNA comprising tracrRNA and/or crRNA, such as a single guide RNA (sgRNA) comprising tracrRNA and crRNA;
the TraC effector protein or functional variant thereof can target and bind to the target DNA sequence under the guidance of the guide RNA derived from a transposon right element, and can also target and bind to the target DNA sequence under the guidance of the guide RNA comprising tracrRNA and/or crRNA.

20. A TraC (intermediates between Transposon and CRISPR-Cas12) effector protein or functional variant thereof for genome editing in an organism or a cell of an organism, the TraC effector protein or functional variant thereof
(i) comprises at least one, at least two or all three amino acid sequence motifs selected from the group consisting of "TSxxCxxCx", "GIDRG" and "CxxCGxxxxADxxAA", wherein x represents any amino acid, such as any naturally encoded amino acid;
(ii) comprises an amino acid sequence that are at least 30%, at least 35%, at least 40%, at least 45%, at least 50%, at least 55%, at least 60%, at least 65%, at least 70%, at least 75%, at least 80%, at least 85%, at least 90%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, at least 99.9%, even 100% identical to any one of SEQ ID NOs: 1-37, or comprises an amino acid sequence having one or more, for example, 1, 2, 3, 4, 5, 6, 7, 8, 9 or 10 amino acid substitutions, deletions or additions relative to any one of SEQ ID NOs: 1-37.

21. The TraC effector protein or functional variant thereof according to claim 20, wherein said effector protein functional variant is derived from SEQ ID NO: 25, and comprises one or more amino acid substitutions selected from K78R, D86R, S137R, V145R, 1147R, P148R, D150R, V228R, V254R, A510R, A278R, K315R, S334R, L343R, A369R, H392R, L394R, S408R, N456R, V500R, A510R, T573R, relative to the sequence of SEQ ID NO: 25.

22. The TraC effector protein or functional variant thereof according to claim 20 or 21, wherein the effector protein functional variant is derived from SEQ ID NO: 25, and comprises any set of mutations shown in Table 3 or 4 relative to the sequence of SEQ ID NO: 25.

23. The TraC effector protein or functional variant thereof according to claim 20, wherein the effector protein functional variant comprises an amino acid sequence selected from SEQ ID NOs: 80-87.

24. The TraC effector protein or functional variant thereof according to any one of Claims 20-23, which has at least guide RNA-mediated sequence-specific targeting ability.

25. The TraC effector protein or functional variant thereof according to any one of Claims 20-23, which has guide RNA-mediated sequence-specific targeting ability, and double-stranded nucleic acid cleavage activity.

26. The TraC effector protein or functional variant thereof according to any one of Claims 20-23, which has guide RNA-mediated sequence-specific targeting capability, and nickase activity.

27. The TraC effector protein or functional variant thereof according to any one of Claims 20-23, which has guide RNA-mediated sequence-specific targeting ability but does not have double-stranded nucleic acid cleavage activity and/or nickase activity.

28. The TraC effector protein or functional variant thereof according to any one of claims 24-24, wherein the guide RNA is selected from i) a guide RNA (reRNA) derived from a transposon right element and/or ii) a guide RNA comprising tracrRNA and/or crRNA, such as a single guide RNA (sgRNA) comprising tracrRNA and crRNA.

29. The TraC effector protein or functional variant thereof according to claim 28, the TraC effector protein or functional variant thereof can target and bind to the target DNA sequence under the guidance of the guide RNA derived from a transposon right element, and can also target and bind to the target DNA sequence under the guidance of the guide RNA comprising tracrRNA and/or crRNA.

30. The TraC effector protein or functional variant thereof according to claim 28, wherein the guide RNA is reRNA derived from the TnpB system, for example, the reRNA comprises a scaffold sequence shown in SEQ ID NO:77 or 78.

31. The TraC effector protein or functional variant thereof according to claim 28, wherein the guide RNA is a single guide RNA (sgRNA) of tracrRNA and crRNA, for example, the sgRNA comprises a scaffold sequence shown in SEQ ID NO:75 or 76.

32. The TraC effector protein or functional variant thereof according to any one of claims 19-31, further comprising at least one nuclear localization sequence (NLS), cytoplasmic localization sequence, chloroplast localization sequence or mitochondrial localization sequence.

33. A fusion protein comprising the TraC effector protein or functional variant thereof according to any one of claims 19-32, and at least one other functional protein.

34. The fusion protein of claim 33, wherein the other functional protein is a deaminase.

35. The fusion protein of claim 34, wherein said deaminase is a cytosine deaminase, for example, said cytosine deaminase is selected from the group consisting of APOBEC1 deaminase, activation-induced cytidine deaminase (AID), APOBEC3G, CDA1, human APOBEC3A deaminase, double-stranded DNA deaminase (Ddd), single-stranded DNA deaminase (Sdd), or functional variants thereof.

36. The fusion protein of claim 35, further comprising a uracil DNA glycosylase inhibitor (UGI).

37. The fusion protein of claim 34, wherein the deaminase is an adenine deaminase, e.g., a DNA-dependent adenine deaminase derived from Escherichia coli tRNA adenine deaminase TadA (ecTadA).

38. The fusion protein of any one of claims 34-37, wherein the fusion protein comprises a cytosine deaminase and an adenine deaminase.

39. The fusion protein of claim 33, wherein said other functional protein is selected from transcription activator protein, transcription repressor protein, DNA methylase, DNA demethylase, reverse transcriptase.

40. The fusion protein according to any one of claims 33-39, wherein different parts of the fusion protein can be independently linked through linkers or linked directly.

41. The fusion protein of any one of claims 33-40, further comprising at least one nuclear localization sequence (NLS), cytoplasmic localization sequence, chloroplast localization sequence or mitochondrial localization sequence.

42. Use of the TraC effector protein or functional variant thereof according to any one of claims 19-32 or the fusion protein according to any one of claims 33-41 for genome editing in cells, preferably eukaryotic cells, more preferably plant cells.

43. The use according to claim 42, wherein the genome editing includes base editing (Base Editor), prime editing (Prime Editor), or PrimeRoot editing (PrimRoot Editor).

44. A genome editing system for performing site-directed modification of a target nucleic acid sequence in a cell genome, comprising:
the TraC effector protein or functional variant thereof of any one of claims 19-32 or the fusion protein of any one of claims 33-41; and/or
an expression construct comprising the nucleotide sequence encoding the TraC effector protein or functional variant thereof according to any one of claims 19-32 or the fusion protein according to any one of claims 33-41.

45. The genome editing system of claim 44, further comprising at least one guide RNA (gRNA) and/or an expression construct comprising a nucleotide sequence encoding the at least one guide RNA.

46. The genome editing system of claim 45, said genome editing system comprises any one selected from the group consisting of:
i) the TraC effector protein or functional variant thereof of any one of Claims 19-32 or the fusion protein of any one of Claims 33-41, and the at least one guide RNA, optionally, the CRISPR nuclease or functional variant thereof or the fusion protein forms a complex with the at least one guide RNA;
ii) an expression construct comprising a nucleotide sequence encoding the TraC effector protein or functional variant thereof of any one of Claims 19-32 or a fusion protein of any one of Claims 33-41, and the at least one guide RNA;
iii) the TraC effector protein or functional variant thereof of any one of Claims 19-32 or the fusion protein of any one of Claims 33-41, and an expression construct comprising a nucleotide sequence encoding the at least one guide RNA;
iv) an expression construct comprising a nucleotide sequence encoding the TraC effector protein or functional variant thereof of any one of Claims 19-32 or a fusion protein of any one of Claims 33-41, and an expression construct comprising a nucleotide sequence encoding the at least one guide RNA;
v) an expression construct comprising a nucleotide sequence encoding the TraC effector protein or functional variant thereof of any one of Claims 19-32 or or the fusion protein of any one of claims 33-41, and a nucleotide sequence encoding the at least one guide RNA.

47. The genome editing system of any one of claims 45-46, wherein the guide RNA is selected from i) a guide RNA derived from a transposon right element (reRNA) and/or ii) a guide RNA comprising tracrRNA and/or crRNA, such as a single-guide RNA (sgRNA) comprising tracrRNA and crRNA.

48. The genome editing system of claim 47, wherein the guide RNA is reRNA derived from the TnpB system, for example, the reRNA comprises a scaffold sequence shown in SEQ ID NO:77 or 78.

49. The genome editing system according to any one of claims 45-46, wherein the guide RNA is a single guide RNA (sgRNA) comprising tracrRNA and crRNA, for example, the sgRNA comprises a scaffold sequence shown in SEQ ID NO:75 or 76.

50. The genome editing system of claim 47 or 49, wherein the guide RNA comprises tracrRNA and crRNA, such as a single guide RNA (sgRNA) comprising tracrRNA and crRNA, wherein the crRNA comprises a sequence identical to the target sequence immediately adjacent to the PAM, and the tracrRNA comprises a sequence (non-targeting strand binding sequence, NTB) complementary to a sequence distal to the PAM in the target sequence direction.

51. The genome editing system according to any one of claims 44-50, wherein said genome editing system further comprises a donor nucleic acid molecule, said donor nucleic acid molecule comprising a nucleotide sequence to be site-directed inserted into the genome, for example, the nucleotide sequence to be inserted into the genome comprises on its both sides sequences homologous to the sequences flacking the target sequence in the genome.

52. The genome editing system according to any one of claims 44-51, wherein the nucleotide sequence encoding the TraC effector protein or functional variant thereof or the fusion protein and/or the nucleotide sequence encoding the at least one guide RNA is operably linked to an expression control element such as a promoter.

53. The genome editing system of any one of claims 44-52, wherein the components of the genome editing system are contained in a delivery system selected from the group consisting of virus, virus-like particle, virosome, liposome , vesicle, exosome, liposomal nanoparticle (LNP), N-acetylgalactosamine (GalNAc) or engineered bacteria.

54. A method of producing a genetically modified cell comprising introducing the genome editing system of any one of claims 44-53 into said cell.

55. The method of claim 54, wherein said cell is from prokaryotes or eukaryotes, preferably from mammals such as humans, mice, rats, monkeys, dogs, pigs, sheep, cattle, cats; poultry such as chickens, ducks , goose; plants, including monocots and dicots, such as rice, maize, wheat, sorghum, barley, soybean, peanut, Arabidopsis.
